# EUROPEAN PATENT SPECIFICATION

(11) **EP 1 641 792 B1**
(45) Date of publication and mention of the grant of the patent: **25.08.2010**
(21) Application number: 04741932.0
(22) Date of filing: 30.06.2004
(51) Int. Cl.: C07D 471/04, A61K 31/4745, A61P 25/00, A61P 15/00, A61P 3/10

(54) **PYRROLODIHYDROISOQUINOLINES AS PDE10 INHIBITORS**
PYRROLODIHYDROISOCHINOLINE ALS PDE10- INHIBITOREN
PYRROLODIHYDROISOQUINOLINES COMME INHIBITEURS DE PDE10

(30) Priority: 30.06.2003 EP 03014424
(43) Date of publication of application: 05.04.2006
(73) Proprietor: Nycomed GmbH, 78467 Konstanz (DE)
(72) Inventor: VENNEMANN, Matthias, 78462 Konstanz (DE); BAER, Thomas, 78479 Reichenau (DE); BRAUNGER, Juergen, 88400 Biberach (DE); MAIER, Thomas, 78333 Stockach (DE); GRAEDLER, Ulrich, 69469 Weinheim (DE); Dr. GIMMNICH, Petra, 78467 Konstanz (DE); QUINTINI, Gianluca, 78464 Konstanz (DE); CIAPETTI, Paola, F-67120 Altorf (FR); CONTRERAS, Jean-Marie, F-67230 Benfeld (FR); WERMUTH, Camille Georges, F-67100 Strasbourg (FR)
(74) Representative: Wild, Robert
(86) International application number: PCT/EP2004/051307
(87) International publication number: WO 2005/003129

(56) References cited:
- WO-A-02/48144
- WO-A-03/014116
- WO-A-03/014117
- WO-A-03/051877
- US-A- 5 965 575
- DIAZ M. ET AL.: "Synthesis of Lamellarins I and K by [3+2] Cycloaddition of a Nitrone to an Alkyne" SYNLETT, vol. 7, 2001, pages 1164-1166, XP001155819
- MEYER H: "HETEROCYCLEN AUS NITROALKENEN, I.-//PYRROLE DURCH CYCLISIERENDE MICHAEL-ADDITIONVON ENAMINEN//HETEROCYCLES FROM NITROALKENES,I.- PYRROLES VIA MICHAEL ADDITION OF ENAMINES" LIEBIGS ANNALEN DER CHEMIE, VERLAG CHEMIE GMBH. WEINHEIM, DE, no. 9, 1981, pages 1534-1544, XP001068958 ISSN: 0170-2041
- HERSHENSON: "Synthesis of Ring-Fused Pyrroles.II.1,3-Dipolar Cycloaddition Reactions of Munchnone Derivatives Obtained from Tetrahydroisoquinoline-1-carboxylic Acids" J.ORG.CHEM., vol. 40, no. 6, 1975, XP002302597

## Description

### Field of application of the invention

The invention relates to novel pyrrolodihydroisoquinoline derivatives, which are used in the pharmaceutical industry for the production of pharmaceutical compositions.

### Prior Art

The International applications WO 02/48144, WO 03/014115, WO 03/014116, WO 03/014117 and WO 03/051877 disclose pyrrolodihydroisoquinoline derivatives with PDE10 inhibitory activity.

The European application EP 1250923 discloses the use of selective PDE10 inhibitors in general, and papaverine in particular, for the treatment of certain neurologic and psychiatric disorders.

Said European application is incorporated by reference into the specification of the present invention in its entirety for all purposes.

Additionally, the US application US 2003/0008806 likewise disclose the use of selective PDE10 inhibitors in general, and papaverine in particular, for the treatment of certain neurologic and psychiatric disorders; said US application is incorporated by reference into the specification of the present invention in its entirety for all purposes.

Yet additionally, the US application US 2003/0018047 also disclose the use of selective PDE10 inhibitors in general, and papaverine in particular, for the treatment of certain neurologic and psychiatric disorders; said US application is incorporated by reference into the specification of the present invention in its entirety for all purposes.

The US patent US 5965575 discloses pyrrolodihydroisoquinoline derivatives as 5HT_{1B} antagonists. The International application WO 03/000269 disclose the use of PDE10A inhibitors for the treatment of neurodegenerative diseases, especially Parkinson's disease.

### Description of the invention

It has now been found that the pyrroloisoquinoline derivatives, which are described in greater details below, differ from prior art compounds by unanticipated, sophisticated, originative and effect-constitutive structural features and have surprising and particularly advantageous properties.

The invention thus relates to compounds of formula I 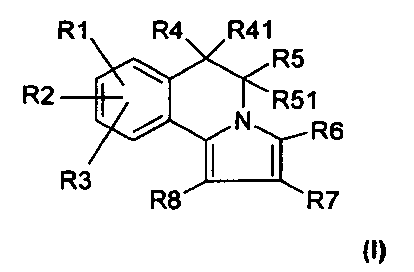 in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl, and
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl, and
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which
R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is either
   a monocyclic or fused bicyclic 5- to 10-membered heteroaryl radical comprising one to three heteroatoms, each of which is selected from a group consisting of nitrogen, oxygen and sulfur, or
   a fused bicyclic 9- or 10-membered, partially saturated heterocyclic ring radical containing a benzene ring and comprising one or two heteroatoms, each of which is selected from a group consisting of nitrogen, oxygen and sulfur,
   or
   N-oxy-pyridyl,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, completely or predominantly fluorine-substituted 1-4C-alkoxy, mono-or di-1-4C-alkylaminocarbonyl, carbamoyl, tetrazolyl, or -N(H)S(O)₂-N(R712)R713, in which
aryl is phenyl or R711-substituted phenyl, in which
R711 is halogen, 1-4C-alkyl, 1-4C-alkoxy, nitro or cyano,
R712 is 1-4C-alkyl,
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl, phenyloxy, phenyl-1-4C-alkyl, arylsulphonyl, 1-4C-alkylsulphonyl, or -S(O)₂-N(R712)R713,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(R82)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
   and
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl;
   and the salts, stereoisomers, hydrates and hydrates of the salts of these compounds.

Compounds according to the present invention more worthy to be mentioned are those compounds of formula I,
in which
R1 is hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 is 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
   and none of R1, R2 and R3 is bound to the 10-position of the pyrrolo[2.1-a]isoquinoline ring,
R4 is hydrogen or 1-4C-alkyl,
R41 is hydrogen or 1-4C-alkyl,
R5 is 1-4C-alkyl, cyano or 1-4C-alkoxycarbonyl, and
R51 is hydrogen or 1-4C-alkyl,
   or
R4 and R5 together form a 1-4C-alkylene bridge and R41 and R51 are both hydrogen,
R6 is 1-6C-alkyl, or 1-4C-alkyl substituted by R61, in which
R61 is 1-4C-alkoxycarbonyl or -N(R611)R612, in which
R611 is 1-4C-alkyl, and
R612 is 1-4C-alkyl, or
R611 and R612 together and with inclusion of the nitrogen atom to which they are bound form a radical Het1, in which
Het1 is pyrrolidin-1-yl, piperidin-1-yl, morpholin-1-yl, or N-(1-4C-alkyl)-piperazinyl,
R7 is Het2, R71- and/or R72- and/or R73-substituted phenyl, R74-substituted Het2, or naphthyl, in which
Het2 is either
   a monocyclic or fused bicyclic 5- to 10-membered heteroaryl radical comprising one to three heteroatoms, each of which is selected from a group consisting of nitrogen, oxygen and sulfur, or
   a fused bicyclic 9- or 10-membered, partially saturated heterocyclic ring radical containing a benzene ring and comprising one or two heteroatoms, each of which is selected from a group consisting of nitrogen, oxygen and sulfur,
   or
   N-oxy-pyridyl,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, 1-4C-alkoxycarbonyl, carboxyl, aryloxy, completely or predominantly fluorine-substituted 1-4C-alkoxy, mono- or di-1-4C-alkylaminocarbonyl, carbamoyl, tetrazolyl, or -N(H)S(O)₂-N(R712)R713, in which
aryl is phenyl or R711-substituted phenyl, in which
R711 is halogen or 1-4C-alkyl,
R712 is 1-4C-alkyl, and
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl,
R72 is halogen, 1-4C-alkyl or 1-4C-alkoxy,
R73 is 1-4C-alkyl or 1-4C-alkoxy,
R74 is 1-4C-alkyl, phenyl-1-4C-alkyl, arylsulphonyl, 1-4C-alkylsulphonyl, or -S(O)₂-N(R712)R713,
R8 is 1-4C-alkyl, cyano, or-C(O)-OR9, in which
R9 is hydrogen or 1-4C-alkyl;
   and the salts, stereoisomers, hydrates and hydrates of the salts of these compounds.

Compounds according to the present invention further more worthy to be mentioned are those compounds of formula I,
in which
R1 is bound to the 8-position of the pyrrolo[2.1-a]isoquinoline ring, and is 1-4C-alkoxy,
R2 is bound to the 7-position of the pyrrolo[2.1-a]isoquinoline ring, and is hydrogen, halogen or 1-4C-alkoxy,
R3 is bound to the 9-position of the pyrrolo[2.1-a]isoquinoline ring, and is 1-4C-alkoxy,
R4 is hydrogen,
R41 is hydrogen,
R5 is 1-4C-alkyl, cyano or 1-4C-alkoxycarbonyl, and
R51 is hydrogen or 1-4C-alkyl,
   or
R4 and R5 together form a 3-4C-alkylene bridge and R41 and R51 are both hydrogen,
R6 is 1-4C-alkyl, or 1-AC-alkyl substituted by R61, in which
R61 is 1-4C-alkoxycarbonyl or -N(R611)R612, in which
R611 and R612 together and with inclusion of the nitrogen atom to which they are bound form a radical Het1, in which
Het1 is morpholin-1-yl,
R7 is Het2, R71- and/or R72- and/or R73-substituted phenyl, R74-substituted Het2, or naphthyl, in which
Het2 is either
   a monocyclic or fused bicyclic 5- to 10-membered heteroaryl radical comprising one to three heteroatoms, each of which is selected from a group consisting of nitrogen, oxygen and sulfur, or
   a fused bicyclic 9- or 10-membered, partially saturated heterocyclic ring radical containing a benzene ring and comprising one or two heteroatoms, each of which is selected from a group consisting of nitrogen, oxygen and sulfur,
   or
   N-oxy-pyridyl,
R71 is hydroxyl, halogen, nitro, 1-4C-alkyl, 1-4C-alkoxy, amino, mono- or di-1-4C-alkylamino, 1-4C-alkylsulphonylamino, carboxyl, aryloxy, mono- or di-1-4C-alkylaminocarbonyl, carbamoyl, tetrazolyl, or -N(H)S(O)₂-N(R712)R713, in which
aryl is phenyl or R711-substituted phenyl, in which
R711 is halogen or 1-4C-alkyl,
R712 is 1-4C-alkyl, and
R713 is 1-4C-alkyl, or
R712 and R713 together and with inclusion of the nitrogen atom to which they are bound form a radical Het3, in which
Het3 is morpholin-4-yl,
R72 is halogen, 1-4C-alkyl or 1-4C-alkoxy,
R73 is 1-4C-alkyl or 1-4C-alkoxy,
R74 is 1-4C-alkyl, phenyl-1-4C-alkyl, arylsulphonyl, 1-4C-alkylsulphonyl, or -S(O)₂-N(R712)R713,
R8 is 1-4C-alkyl, cyano, or-C(O)-OR9, in which
R9 is hydrogen or 1-4C-alkyl;
   and the salts, stereoisomers, hydrates and hydrates of the salts of these compounds.

Compounds according to the present invention in particular worthy to be mentioned are those compounds of formula I,
in which
either, in a first independent embodiment,
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy,
R4 is hydrogen,
R41 is hydrogen,
R5 is 1-2C-alkyl or cyano, and
R51 is hydrogen,
   or
R4 and R5 together form a tetramethylene bridge and R41 and R51 are both hydrogen,
R6 is 1-2C-alkyl, or 1-2C-alkyl substituted by R61, in which
R61 is 1-2C-alkoxycarbonyl or -N(R611)R612, in which
R611 and R612 together and with inclusion of the nitrogen atom to which they are bound form a radical Het1, in which
Het1 is morpholin-1-yl,
R7 is naphthyl, 4-hydroxy-3,5-dimethylphenyl, 4-methoxy-3,5-dimethylphenyl, 4-carboxy-phenyl, 4-carbamoyl-phenyl, 2-methyl-4-hydroxy-phenyl, 4-amino-phenyl, 4-(2H-tetrazol-5-yl)-phenyl, 4-mopholino-sulphonylamino-phenyl, 4-methylsulphonylamino-phenyl, or 2-fluoro-3,4-dimethoxyphenyl,
   pyridyl, indolyl, quinolinyl, indolinyl,
   2-methyt-pyridin-4-yl, 3-methyl-pyridin-4-yl, or
   N-(R74)-Het2, in which
Het2 is pyrrolyl or indolyl,
R74 is arylsulphonyl, 1-2C-alkylsulphonyl, or -S(O)₂-N(R712)R713, in which
aryl is phenyl, or R711-substituted phenyl, in which
R711 is 1-2C-alkyl,
R712 is 1-2C-alkyl,
R713 is 1-2C-alkyl, or
R712 and R713 together and with inclusion of the nitrogen atom to which they are bound form a radical Het3, in which
Het3 is morpholin-4-yl, and
R8 is cyano;
   or, in a second independent embodiment,
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy,
R4 is hydrogen,
R41 is hydrogen,
R5 is 1-2C-alkyl or cyano,
R51 is hydrogen,
   or
R4 and R5 together form a tetramethylene bridge and R41 and R51 are both hydrogen,
R6 is 1-2C-alkyl, or 1-2C-alkyl substituted by R61, in which
R61 is 1-2C-alkoxycarbonyl or -N(R611)R612, in which
R611 and R612 together and with inclusion of the nitrogen atom to which they are bound form a radical Het1, in which
Het1 is morpholin-1-yl,
R7 is naphthyl, 4-hydroxy-3,5-dimethylphenyl, 4-methoxy-3,5-dimethylphenyl, 4-carboxy-phenyl, 4-carbamoyl-phenyl, 2-methyl-4-hydroxy-phenyl, 4-amino-phenyl, 4-(2H-tetrazol-5-yl)-phenyl, 4-mopholino-sulphonylamino-phenyl, 4-methylsulphonylamino-phenyl, or 2-fluoro-3,4-dimethoxyphenyl,
   pyridyl, indolyl, quinolinyl, indolinyl,
   2-methyl-pyridin-4-yl, 3-methyl-pyridin-4-yl, or
   N-(R74)-Het2, in which
Het2 is pyrrolyl or indolyl,
R74 is arylsulphonyl, 1-2C-alkylsulphonyl, or -S(O)₂-N(R712)R713, in which
aryl is phenyl, or R711-substituted phenyl, in which
R711 is 1-2C-alkyl,
R712 is 1-2C-alkyl, and
R713 is 1-2C-alkyl, or
R712 and R713 together and with inclusion of the nitrogen atom to which they are bound form a radical Het3, in which
Het3 is morpholin-4-yl, and
R8 is -C(O)-OR9, in which
R9 is 1-2C-alkyl;
   and the salts, stereoisomers, hydrates and hydrates of the salts of these compounds.

Compounds according to the present invention in further particular worthy to be mentioned are those compounds of formula I,
in which
either, in a first independent embodiment,
R1 is bound to the 8-position of the pyrrolo[2.1-a]isoquinoline ring, and is methoxy,
R2 is bound to the 7-position of the pyrrolo[2.1-a]isoquinoline ring, and is hydrogen or fluorine,
R3 is bound to the 9-position of the pyrrolo[2.1-a]isoquinoline ring, and is methoxy,
R4 is hydrogen,
R41 is hydrogen,
R5 is methyl or cyano,
R51 is hydrogen,
R6 is methyl or 2-methoxycarbonylethyl,
R7 is 4-hydroxy-3,5-dimethylphenyl, 4-methoxy-3,5-dimethylphenyl, 4-carboxy-phenyl, 2-methyl-4-hydroxy-phenyl, 4-amino-phenyl, 4-(2H-tetrazol-5-yl)-phenyl, 4-mopholino-sulphonylamino-phenyl, 4-methylsulphonylamino-phenyl,
   pyridyl, quinolinyl,
   2-methyl-pyridin-4-yl, 3-methyl-pyridin-4-yl,
   1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonyl-indol-3-yl, and
R8 is cyano;
   or, in a second independent embodiment,
R1 is bound to the 8-position of the pyrrolo[2.1-a]isoquinoline ring, and is methoxy,
R2 is bound to the 7-position of the pyrrolo[2.1-a]isoquinoline ring, and is hydrogen or fluorine,
R3 is bound to the 9-position of the pyrrolo[2.1-a]isoquinoline ring, and is methoxy,
R4 is hydrogen,
R41 is hydrogen,
R5 is methyl or cyano,
R51 is hydrogen,
R6 is methyl or 2-methoxycarbonylethyl,
R7 is 4-hydroxy-3,5-dimethylphenyl, 4-methoxy-3,5-dimethylphenyl, 4-carboxy-phenyl, 2-methyl-4-hydroxy-phenyl, 4-amino-phenyl, 4-(2H-tetrazol-5-yl)-phenyl, 4-mopholino-sulphonylamino-phenyl, 4-methylsulphonylamino-phenyl,
   pyridyl, quinolinyl,
   2-methyl-pyridin-4-yl, 3-methyl-pyridin-4-yl,
   1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonyl-indol-3-yl, and
R8 is -C(O)-OR9, in which
R9 is ethyl;
   and the salts, stereoisomers, hydrates and hydrates of the salts of these compounds.

An interesting embodiment of the compounds according to the present invention refers to those compounds of formula I,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is 1-2C-alkyl or cyano,
R51 is hydrogen,
   and
R8 is -C(O)-OR9, in which
R9 is 1-2C-alkyl.

A more interesting embodiment of the compounds according to the present invention refers to those compounds of formula I,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is hydrogen, chlorine or fluorine,
R3 is bound to the 9-position of the pyrroto[2.1-a]isoquinoline ring, and is 1-2C-alkoxy, such as e.g. methoxy,
   and
R4 is hydrogen,
R41 is hydrogen,
R5 is 1-2C-alkyl or cyano,
R51 is hydrogen,
   and
R8 is cyano.

Another more interesting embodiment of the compounds according to the present invention refers to those compounds of formula I,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is 1-2C-alkyl or cyano,
R51 is hydrogen,
   and
R8 is cyano.

Another more interesting embodiment of the compounds according to the present invention refers to those compounds of formula I,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is 1-2C-alkyl or cyano,
R51 is hydrogen,
   and
R8 is cyano.

A particular interesting embodiment of the compounds according to the present invention refers to those compounds of formula I,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is 1-2C-alkyl or cyano, in particular methyl,
R51 is hydrogen,
   and
R8 is cyano.

A variant (variant 1) of the present invention refers to those compounds of formula I, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 14C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy,cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(R82)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
   R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl;
   and to the salts, stereoisomers, hydrates and hydrates of the salts of these compounds.

The invention further relates in a first aspect (aspect a) of variant 1 to compounds of formula I, in which
in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl or -C(O)-N(R82)R83, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
   R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
   and to the salts, stereoisomers, hydrates and hydrates of the salts of these compounds.

The invention further relates in a second aspect (aspect b) of variant 1 to compounds of formula I, in which
R1 is halogen, nitro, amino, mono- or di-1-4C-alkylamino, 1-4C-alkyl, 1-4C-alkoxy-2-4C-alkoxy, 3-7C-cycloalkoxy, 3-7C-cycloalkylmethoxy, or completely or predominantly fluorine-substituted 1-4C-alkoxy,
   with the provisio that R1 is not trifluoromethoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or-CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono-or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(R82)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl, R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl,
   and to the salts, stereoisomers, hydrates and hydrates of the salts of these compounds.

The invention further relates in a third aspect (aspect c) of variant 1 to compounds of formula I, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is halogen or 1-4C-alkoxy,
R3 is 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-OR511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81 , phenylcarbonyl, -C(O)-N(R82)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl,
   and to the salts, stereoisomers, hydrates and hydrates of the salts of these compounds.

The invention further relates in a fourth aspect (aspect d) of variant 1 to compounds of formula I, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, or naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(R82)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl,
   and to the salts, stereoisomers, hydrates and hydrates of the salts of these compounds.

The invention further relates in a fifth aspect (aspect e) of variant 1 to compounds of formula I, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-AC-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-OR511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1AC-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl; nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is carboxyl,
   and to the salts, stereoisomers, hydrates and hydrates of the salts of these compounds.

1-4C-Alkyl represents a straight-chain or branched alkyl radical having 1 to 4 carbon atoms. Examples which may be mentioned are the butyl, isobutyl, sec-butyl, tert-butyl, propyl, isopropyl and preferably the ethyl and methyl radicals.

2-4C-Alkyl represents a straight-chain or branched alkyl radical having 2 to 4 carbon atoms. Examples which may be mentioned are the butyl, isobutyl, sec-butyl, tert-butyl, propyl, isopropyl and preferably the ethyl radical.

1-6C-Alkyl represents a straight-chain or branched alkyl radical having 1 to 6 carbon atoms. Examples which may be mentioned are the hexyl, isohexyl (4-methylpentyl), neohexyl (3,3-dimethylbutyl), pentyl, isopentyl (3-methylbutyl), neopentyl (2,2-dimethylpropyl), butyl, isobutyl, sec-butyl, tert-butyl, propyl, isopropyl, ethyl or methyl radicals.

1-4C-Alkoxy represents radicals which, in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy and preferably the ethoxy and methoxy radicals.

1-4C-Alkylthio represents radicals which, in addition to the sulfur atom, contain a straight-chain or branched alkyl radical having 1 to 4 carbon atoms. Examples which may be mentioned are the ethylthio and the methylthio radicals.

2-4C-Alkoxy represents radicals which, in addition to the oxygen atom, contain a straight-chain or branched alkyl radical having 2 to 4 carbon atoms. Examples which may be mentioned are the butoxy, isobutoxy, sec-butoxy, tert-butoxy, propoxy, isopropoxy and preferably the ethoxy radical.

3-7C-Cycloalkoxy represents cyclopropyloxy, cyclobutyloxy, cyclopentyloxy, cyclohexyloxy and cycloheptyloxy, of which cyclopropyloxy, cyclobutyloxy and cyclopentyloxy are preferred.

3-7C-Cycloalkyl represents cyclopropyl, cyclobutyl, cyclopentyl, cyclohexyl and cycloheptyl, of which 6yclopropyl, cyclobutyl and cyclopentyl are preferred.

3-7C-Cycloalkylmethoxy represents cyclopropylmethoxy, cyclobutylmethoxy, cyclopentylmethoxy, cyclohexylmethoxy and cycloheptylmethoxy, of which cyclopropylmethoxy, cyclobutylmethoxy and cyclopentylmethoxy are preferred.

3-7C-Cycloalkyl-1-4C-alkyl represents one of the abovementioned 1-4C-alkyl radicals, which is substituted by one of the abovementioned 3-7C-cycloalkyl radicals. Examples which may be mentioned are the cyclopropylmethyl, the cyclohexylethyl and the cyclohexylmethyl radicals.

As completely or predominantly fluorine-substituted 1-4C-alkoxy, for example, the 2,2,3,3,3-pentafluoropropoxy, the perfluoroethoxy, the 1,2,2-trifluoroethoxy, in particular the 1,1,2,2-tetrafluoroethoxy, the 2,2,2-trifluoroethoxy, the trifluoromethoxy and preferably the difluoromethoxy radicals may be mentioned. "Predominantly" in this connection means that more than half of the hydrogen atoms of the 1-4C-alkoxy radicals are replaced by fluorine atoms.

1-4C-Alkoxy-2-4C-alkoxy represents one of the abovementioned 2-4C-alkoxy radicals, which is substituted by one of the abovementioned 1-4C-alkoxy radicals. Examples which may be mentioned are the 2-methoxyethoxy, 2-ethoxyethoxy and the 2-isopropoxyethoxy radicals.

1-4C-Alkoxy-2-4C-alkyl represents one of the abovementioned 2-4C-alkyl radicals, which is substituted by one of the abovementioned 1-4C-alkoxy radicals. Examples which may be mentioned are the 2-methoxyethyl and the 2-isopropoxyethyl radicals.

1-4C-Alkoxy-1-4C-alkyl stands for one of the abovementioned 1-4C-alkyl radicals, which is substituted by one of the abovementioned 1-4C-alkoxy radicals. Examples which may be mentioned are the 2-methoxyethyl and 2-isopropoxyethyl radicals.

1-2C-Alkylenedioxy represents, for example, the methylenedioxy [-O-CH₂-O-] and the ethylenedioxy [-O-CH₂-CH₂-O-] radicals.

As completely or predominantly fluorine-substituted 1-2C-alkylenedioxy bridge, for example, the difluoromethylenedioxy [-O-CF₂-O-] radical may be mentioned. "Predominantly" in this connection means that more than half of the hydrogen atoms of the 1-4C-alkylenedioxy radical are replaced by fluorine atoms.

Phenyl-1-4C-alkyl stands for one of the abovementioned 1-4C-alkyl radicals, which is substituted by a phenyl radical. Examples which may be mentioned are the phenethyl and the benzyl radicals.

1-4C-Alkoxycarbonyl represents a radical which, in addition to the carbonyl group, contains one of the abovementioned 1-4C-alkoxy radicals. Examples which may be mentioned are the methoxycarbonyl and ethoxycarbonyl radicals.

1-4C-Alkylcarbonyl represents a radical which, in addition to the carbonyl group, contains one of the abovementioned 1-4C-alkyl radicals. An example which may be mentioned is the acetyl radical.

1-4C-Alkylene is a straight-chain alkylene radical such as, for example, the methylene (-CH₂-) or, particularly, the trimethylene (-CH₂-CH₂-CH₂-) or the tetramethylene (-CH₂-CH₂-CH₂-CH₂-) radical.

Halogen within the meaning of the invention is bromine and, preferably, chlorine and fluorine.

Hydroxy-2-4C-alkyl stands for one of the abovementioned 2-4C-alkyl radicals which is substituted by a hydroxyl group. Examples which may be mentioned are the 2-hydroxyethyl and 3-hydroxypropyl radicals.

Hydroxy-2-4C-alkoxy stands for one of the abovementioned 2-4C-alkoxy radicals which is substituted by a hydroxyl group. Examples which may be mentioned are the 2-hydroxyethoxy and 3-hydroxypropoxy radicals.

Amino-2-4C-alkyl stands for one of the abovementioned 2-4C-alkyl radicals which is substituted by an amino group. Examples which may be mentioned are the 2-aminoethyl and 3-aminopropyl radicals.

Amino-2-4C-alkoxy stands for one of the abovementioned 2-4C-alkoxy radicals which is substituted by an amino group. Examples which may be mentioned are the 2-aminoethoxy and 3-aminopropoxy radicals.

In addition to the nitrogen atom, mono- or di-1-4C-alkylamino radicals contain one or two of the abovementioned 1-4C-alkyl radicals. Di-1-4C-alkylamino is to be emphasized and here, in particular, dimethyl-, diethyl- and diisopropylamino.

Mono- or Di-1-4C-alkylamino-2-4C-alkyl stands for one of the abovementioned 2-4C-alkyl radicals which is substituted by one of the abovementioned mono- or di-1-4C-alkylamino radicals. Examples which may be mentioned are the 2-dimethylaminoethyl and 3-dimethylaminopropyl radicals.

Mono- or Di-1-4C-alkylamino -2-4C-alkoxy stands for one of the abovementioned 2-4C-alkoxy radicals which is substituted by one of the abovementioned mono- or di-1-4C-alkylamino radicals. Examples which may be mentioned are the 2-dimethylaminoethoxy and 3-dimethylaminopropoxy radicals.

1-4C-Alkylsulfonyl is a sulfonyl group to which one of the abovementioned 1-4C-alkyl radicals is bonded. An example is the methanesulfonyl radical (CH₃SO₂-).

1-4C-Alkylsulfonylamino is an amino group which is substituted by one of the abovementioned 1-4C-alkylsulfonyl radicals. An example is the methanesulfonylamino radical (CH₃SO₂NH-).

Aryl radicals referred to herein, including those forming part of other groups or radicals, include phenyl or R711-substituted phenyl radicals.

Aryloxy stands for phenoxy or R711-substituted phenoxy.

Aryl-1-4C-alkoxy stands for one of the abovementioned 1-4C-alkoxy radicals, which is substituted by one of the abovementioned aryl radicals. Examples which may be mentioned are the 2-arylethoxy (e.g. phenethoxy) and the arylmethoxy (e.g. benzyloxy) radicals.

Aryloxy-2-4C-alkoxy stands for one of the abovementioned 2-4C-alkoxy radicals, which is substituted by one of the abovementioned aryloxy radicals. An example which may be mentioned is the 2-aryloxyethoxy (e.g. 2-phenoxyethoxy) radical.

Aryloxy-1-4C-alkyl stands for one of the abovementioned 1-4C-alkyl radicals, which is substituted by one of the abovementioned aryloxy radicals. Examples which may be mentioned are the 2-aryloxyethyl (e.g. 2-phenoxyethyl) and the aryloxymethyl (e.g. phenoxymethyl) radicals.

Mono- or Di-1-4C-alkylaminocarbonyl radicals contain in addition to the carbonyl group one of the abovementioned mono- or di-1-4C-alkylamino radicals. Examples which may be mentioned are the N-methyl- the N,N-dimethyl-, the N-ethyl-, the N-propyl-, the N,N-diethyl- and the N-isopropylaminocarbonyl radical.

2-4C-Alkinyl is a straight chain or branched alkinyl radical having 2 to 4 carbon atoms. Examples are the 2-propinyl (propargyl) and the ethinyl radicals.

Het1 refers to a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom. Examples for Het1 include e.g. piperidin-1-yl, 4-methyl-piperidin-1-yl, 4-hydroxypiperidin-1-yl, morpholin-4-yl, pyrrolidin-1-yl, piperazin-1-yl, imidazolidin-1-yl, thiomorpholin-4-yl, homopiperidin-1-yl, homopiperazin-1-yl, 4-N-(1-4C-alkyl)-homopiperazin-1-yl or piperazinyl substituted on a ring nitrogen atom by R613 [4-N-(R613)-piperazin-1-yl] such as, for example, 4-N-(1-C-alkyl)-piperazin-1-yl, 4-N-(hydroxy-2-4C-alkyl)-piperazin-1-yl, 4-N-(dimethylamino-2-4C-alkyl)-piperazin-1-yl, 4-N-(3-6C-cycloalkyl)-piperazin-1-yl, 4-N-formyl-piperazin-1-yl, 4-N-(pyridin-4-yl)-piperazin-1-yl, 4-N-(pyrimidin-2-yl)-piperazin-1-yl or 4-N-(3-6C-cycloalkylmethyl)-piperazin-1-yl.

Het2 refers to a monocyclic or fused bicyclic 5 to 10-membered heteroaryl (heteroaromatic) radical comprising one to three heteroatoms, each of which is selected from a group consisting of nitrogen, oxygen and sulfur, and includes, for example, without being restricted to furanyl, thiophenyl, pyrrolyl, oxazolyl, isoxazolyl, thiazolyl, isothiazolyl, imidazolyl, pyrazolyl, triazolyl, thiadiazolyl, oxadiazolyl, pyridinyl, pyrimidinyl, pyrazinyl, pyridazinyl, benzo-fused analogues thereof, such as, for example, quinazolinyl, quinoxalinyl, cinnolinyl, quinolyl, isoquinolyl, indolyl, isoindolyl, indazolyl, benzothiophenyl, benzofuranyl, benzoxazolyl, benzothiazolyl or benzimidazolyl, or naphthyridinyl, phthalazinyl, imidazopyridinyl, purinyl, pteridinyl or imidazopyridazinyl. The monocyclic 5- to 6-membered radicals, such as, for example, furanyl, thiophenyl, pyrrolyl, pyrimidinyl and pyridinyl, and quinolinyl and indolyl are more worthy to be mentioned. In particular worthy to be mentioned are indolyl, quinolinyl and pyridinyl. In more particular worthy to be mentioned are quinolyl and pyridinyl, especially quinolin-4-yl and, particularly, pyridin-4-yl.

Alternatively Het2 refers to a fused bicyclic 9- or 10-membered, partially saturated heterocyclic ring radical containing a benzene ring and comprising one or two heteroatoms, each of which is selected from a group consisting of nitrogen, oxygen and sulfur, and includes, for example, without being restricted to indolinyl, isoindolinyl, 1,2,3,4-tetrahydroquinolinyl, 1,2,3,4-tetrahydroisoquinolinyl, 1,3-benzodioxolyl, 2,3-dihydro-1,4-benzodioxinyl or 2,3-dihydrobenzofuranyl.

N-(1-4C-alkyl)-piperazinyl stands for the piperazin-1-yl radical substituted by one of the abovementioned 1-4C-alkyl radicals on the 4-N ring nitrogen atom.

Naphthyl includes naphthalen-1-yl and naphthalen-2-yl.

The term Het2 includes all the possible isomeric forms thereof, in particular the positional isomers thereof. Thus, e.g. pyridinyl or pyridyl includes pyridin-2-yl, pyridin-3-yl and pyridin-4-yl.

Constituents which are substituted as described herein may be substituted, unless otherwise noted, at any possible position.

Thus, the substituents R1, R2 and/or R3 may be attached, unless otherwise noted, at any position of the benzo moiety of the pyrrolodihydroisoquinoline ring.

The substituents R71, R72 and/or R73 of the compounds according to this invention can be each attached in the ortho, meta or para position with respect to the binding position in which the phenyl ring is bonded to the pyrrolo moiety of the pyrrolodihydroisoquinoline ring, wherein in an embodiment of the present invention the attachement in the meta or, in particular, in para position is to be emphasized.

Suitable salts for compounds of the formula I - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds according to the invention on an industrial scale, are converted into pharmacologically tolerable salts by processes known to the person skilled in the art.

According to expert's knowledge the compounds of the invention as well as their salts may contain, e.g. when isolated in crystalline form, varying amounts of solvents. Included within the scope of the invention are therefore all solvates and in particular all hydrates of the compounds of formula I as well as all solvates and in particular all hydrates of the salts of the compounds of formula I.

Depending on substitution the compounds of formula I can be chiral compounds having, for example, chiral centers and/or chiral axes due to hindered rotation about single bonds. Chiral axes can be present in particular in those compounds according to the invention, in which R7 is a bicyclic ring, or a monocyclic ring substituted in the ortho position with respect to the binding position in which said monocyclic ring is bonded to the pyrrolo[2.1-a]isoquinoline ring system. The invention therefore includes all conceivable pure diastereomers and pure enantiomers and mixtures thereof in any mixing ratio including the racemates. The diastereomer mixtures can be separated into the individual isomers by chromatographic processes. The enantiomers can be separated in a known manner (e.g. by chromatographic processes on chiral phases or by resolution).

A special subaspect (subaspect 1) of aspects a, b, c, d and e refers to compounds of formula I according to aspects a, b, c, d and e, in which none of R1, R2 and R3 is bound to the 10-position of the pyrrolo[2.1-a]isoquinoline ring.

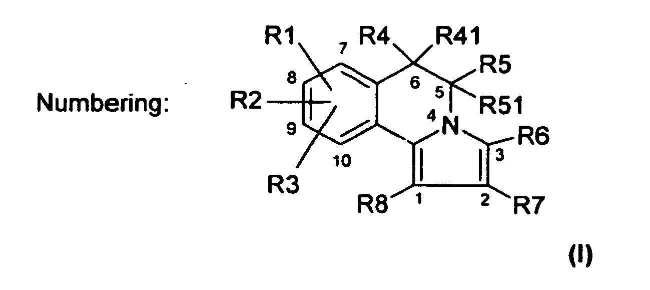

A further special subaspect (subaspect 2) of aspects a, b, c and d refers to compounds of formula I according to aspects a, b, c and d, in which
R8 is 1-4C-alkyl, phenyl, 2-4C-alkinyl, cyano, -CH₂-O-R81, phenylcarbonyl or -C(O)-N(R82)R83, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl.

A further special subaspect (subaspect 3) of aspects a, b, c, d and e refers to compounds of formula I according to aspects a, b, c, d and e, in which
R1 is halogen, nitro, amino, mono- or di-1-4C-alkylamino, 1-4C-alkyl, 1-4C-alkoxy-2-4C-alkoxy, 3-7C-cycloalkoxy, 3-7C-cycloalkylmethoxy, or completely or predominantly fluorine-substituted 1-4C-alkoxy,
   with the provisio that R1 is not trifluoromethoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen.

A further special subaspect (subaspect 4) of aspects a, c, d and e refers to compounds of formula I according to aspects a, c, d and e, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is halogen or 1-4C-alkoxy,
R3 is 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge.

A further special subaspect (subaspect 5) of aspects a, b, c, d and e refers to compounds of formula I according to aspects a, b, c, d and e, in which
R4 is fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-2-4-alkyl or 1-4C-alkylcarbonyl,
R41 is hydrogen or 1-4C-alkyl,
R5 is fluorine or 1-4C-alkyl,
R51 is hydrogen or 1-4C-alkyl,
   or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl,
R51 is hydrogen or 1-4C-alkyl,
   or
R4 and R5 together form a 1-4C-alkylene bridge and R41 and R51 are both hydrogen.

A further special subaspect (subaspect 6) of aspects a, b, d and e refers to compounds of formula I according to aspects a, b, d and e, in which
R1 is halogen, nitro, amino, 1-4C-alkyl, 1-4C-alkoxy-2-4C-alkoxy, or completely or predominantly fluorine-substituted 1-4C-alkoxy,
   with the provisio that R1 is not trifluoromethoxy,
R2 is hydrogen or 1-4C-alkoxy,
R3 is hydrogen or 1-4C-alkoxy, or
R1 and R2 bound to the benzo ring moiety in ortho-position to each other together form a completely or predominantly fluorine-substituted 1-2C-alkylenedioxy bridge and R3 is hydrogen.

A further special subaspect (subaspect 7) of said aspects a, c, d and e refers to compounds of formula I according to aspects a, c, d and e, in which
R1 is halogen, nitro, amino, 1-4C-alkyl, 1-4C-alkoxy, 1-4C-alkoxy-2-4C-alkoxy, or completely or predominantly fluorine-substituted 1-4C-alkoxy,
R2 is 1-4C-alkoxy,
R3 is 1-4C-alkoxy.

A further special subaspect (subaspect 8) of said aspects a, c, d and e refers to compounds of formula I according to aspects a, c, d and e, in which
R1 is halogen, nitro, amino, 1-4C-alkyl, 1-4C-alkoxy, 1-4C-alkoxy-24C-alkoxy, or completely or predominantly fluorine-substituted 1-4C-alkoxy,
R2 is halogen,
R3 is 14C-alkoxy.

A further special subaspect (subaspect 9) of said aspects a, d and e refers to compounds of formula I according to aspects a, d and e, in which
R1 is halogen, nitro, amino, 1-4C-alkyl, 1-4C-alkoxy, 1-4C-alkoxy-2-4C-alkoxy, or completely or predominantly fluorine-substituted 1-4C-alkoxy,
R2 is 1-4C-alkoxy,
R3 is hydrogen.

A further special subaspect (subaspect 10) of said aspects a, d and e refers to compounds of formula I according to aspects a, d and e, in which
R1 is 1-4C-alkoxy,
R2 is 1-4C-alkoxy,
R3 is hydrogen.

A further special subaspect (subaspect 11) of said aspects a, d and e refers to compounds of formula I according to aspects a, d and e, in which
R1 is halogen or 1-2C-alkoxy,
R2 is hydrogen or 1-2C-alkoxy,
R3 is 1-2C-alkoxy.

A further special subaspect (subaspect 12) of said aspects a, c, d and e refers to compounds of formula I according to aspects a, c, d and e, in which
R1 is 1-2C-alkoxy,
R2 is 1-2C-alkoxy,
R3 is 1-2C-alkoxy.
Compounds according to subaspect 12 more worthy to be mentioned are those, in which none of R1, R2 and R3 is bound to the 10-position of the pyrrolo[2.1-a]isoquinoline ring.

A further special subaspect (subaspect 13) of said aspects a, d and e refers to compounds of formula I according to aspects a, d and e, in which
R1 is 1-2C-alkoxy,
R2 is hydrogen,
R3 is 1-2C-alkoxy.
Compounds according to subaspect 13 more worthy to be mentioned are those, in which R1 is bound to the 8-position and R3 is bound to the 9-position of the pyrrolo[2.1-a]isoquinoline ring, or those, in which R1 is bound to the 9-position and R3 is bound to the 8-position of the pyrrolo[2.1-a]isoquinoline ring.

A further special subaspect (subaspect 14) of said aspects a, b, d and e refers to compounds of formula I according to aspects a, b, d and e, in which
R1 is halogen,
R2 is hydrogen,
R3 is 1-2C-alkoxy,
Compounds according to subaspect 14 more worthy to be mentioned are those, in which R1 is bound to the 8-position and R3 is bound to the 9-position of the pyrrolo[2.1-a]isoquinoline ring, or those, in which R1 is bound to the 9-position and R3 is bound to the 8-position of the pyrrolo[2.1-a]isoquinoline ring.

A further special subaspect (subaspect 15) of said aspects a, b, c, d and e refers to compounds of formula I according to aspects a, b, c, d and e, in which
R1 is halogen,
R2 is 1-2C-alkoxy,
R3 is 1-2C-alkoxy.
Compounds according to subaspect 15 more worthy to be mentioned are those, in which none of R1, R2 and R3 is bound to the 10-position of the pyrrolo[2.1-a]isoquinoline ring.

A further special subaspect (subaspect 16) of said aspects a, b, c, d and e refers to compounds of formula I according to aspects a, b, c, d and e, in which
R1 is halogen,
R2 is halogen,
R3 is 1-2C-alkoxy.
Compounds according to subaspect 16 more worthy to be mentioned are those, in which none of R1_{,} R2 and R3 is bound to the 10-position of the pyrrolo[2.1-a]isoquinoline ring.

A further special subaspect (subaspect 17) of said aspects a, b, c, d and e refers to compounds of formula I according to aspects a, b, c, d and e, in which
R1 is halogen, nitro, 14C-alkyl or 1-4C-alkoxy-2-4C-alkoxy.

A further special subaspect (subaspect 18) of aspects a, b, c, d and e refers to compounds of formula I according to aspects a, b, c, d and e, in which
R1 is chlorine or fluorine.
Compounds according to subaspect 18 more worthy to be mentioned are those, in which R1 is not bound to the 10-position of the pyrrolo[2.1-a]isoquinoline ring.

A further special subaspect (subaspect 19) of aspects a, b, c, d and e refers to compounds of formula I according to aspects a, b, c, d and e, in which
R4 is 1-4C-alkyl,
R41 is hydrogen or 1-4C-alkyl,
R5 is 1-4C-alkyl,
R51 is hydrogen or 1-4C-alkyl,
   or
R4 is hydrogen or 1-4C-alkyl,
R41 is hydrogen or 1-4C-alkyl,
R5 is 1-4C-alkyl or 1-4C-alkoxycarbonyl,
R51 is hydrogen or 1-4C-alkyl,
   or
R4 and R5 together form a 3-4C-alkylene bridge and R41 and R51 are both hydrogen.

A further special subaspect (subaspect 20) of aspects a, b, c, d and e refers to compounds of formula I according to aspects a, b, c, d and e, in which
R4 is hydrogen,
R41 is hydrogen,
R5 is 1-4C-alkyl or 1-4C-alkoxycarbonyl,
R51 is hydrogen,
   or
R4 is hydrogen,
R41 is hydrogen,
R5 is 1-4C-alkyl,
R51 is 1-4C-alkyl,
   or
R4 and R5 together form a 3-4C-alkylene bridge and R41 and R51 are both hydrogen.

A further special subaspect (subaspect 21) of aspects a, b, c, d and e refers to compounds of formula I according to aspects a, b, c, d and e, in which either
R4 is 1-4C-alkyl, or
R41 is 1-4C-alkyl, or
R5 is 1-4C-alkyl or 1-4C-alkoxycarbonyl, or
R51 is 1-4C-alkyl, or
R4 and R5 together form a 3-4C-alkylene bridge and R41 and R51 are both hydrogen.

A further special subaspect (subaspect 22) of aspects a, b, c, d and e refers to compounds of formula I according to aspects a, b, c, d and e, in which
R5 is 14C-alkyl.

A further special subaspect (subaspect 23) of aspects a, b, c, d and e refers to compounds of formula I according to aspects a, b, c, d and e, in which
R4 is hydrogen,
R41 is hydrogen,
R5 is 1-4C-alkyl,
R51 is hydrogen.

A further special subaspect (subaspect 24) of aspects a, b, c, d and e refers to compounds of formula I according to aspects a, b, c, d and e, in which
R4 is hydrogen,
R41 is hydrogen,
R5 is methyl or ethyl,
R51 is hydrogen.

A further special subaspect (subaspect 25) of aspects a, b, c, d and e refers to compounds of formula I according to aspects a, b, c, d and e, in which
R4 is hydrogen,
R41 is hydrogen,
R5 is methyl,
R51 is hydrogen.

A further special subaspect (subaspect 26) of aspects a, b, c, d and e refers to compounds of formula I according to aspects a, b, c, d and e, in which
R6 is 1-6C-alkyl, or 1-4C-alkyl substituted by R61, in which
R61 is 1-4C-alkoxycarbonyl.

A further special subaspect (subaspect 27) of aspects a, b, c, d and e refers to compounds of formula I according to aspects a, b, c, d and e, in which
R6 is methyl, ethyl or methoxycarbonylethyl.

A further special subaspect (subaspect 28) of aspects a, b, c, d and e refers to compounds of formula I according to aspects a, b, c, d and e, in which
R6 is methyl.

A further special subaspect (subaspect 29) of aspects a, b, c, d and e refers to compounds of formula I according to aspects a, b, c, d and e, in which
R6 is methoxycarbonylethyl.

A further special subaspect (subaspect 30) of aspects a, b, c, d and e refers to compounds of formula I according to aspects a, b, c, d and e, in which
R7 is Het2, R74- and/or R75-substituted Het2, or hydroxy-dimethyl-phenyl, in which
Het2 is pyridinyl or quinolinyl,
R74 is halogen, 1-4C-alkyl, trifluoromethyl, 1-4C-alkoxy, cyano, amino, mono- or di-1-4C-alkylamino, 1-4C-alkoxycarbonyl, carboxyl, nitro, phenyl or phenyloxy,
R75 is 1-4C-alkyl.

Compounds according to subaspect 30 more worthy to be mentioned are those, in which
R7 is Het2, R74- and/or R75-substituted Het2, or 4-hydroxy-3,5-dimethylphenyl, in which
Het2 is pyridin-4-yl or quinolin-4-yl,
R74 is halogen, 1-4C-alkyl, trifluoromethyl, 1-4C-alkoxy, cyano, amino, mono- or di-1-4C-alkylamino, 1-4C-alkoxycarbonyl, carboxyl, nitro, phenyl or phenyloxy,
R75 is 1-4C-alkyl.

A further special subaspect (subaspect 31) of aspects a, b, c, d and e refers to compounds of formula I according to aspects a, b, c, d and e, in which
R7 is pyridin-4-yl.

A further special subaspect (subaspect 32) of aspects a, b, c, d and e refers to compounds of formula I according to aspects a, b, c, d and e, in which
R7 is 2,6-dimethylpyridin-4-yl.

A further special subaspect (subaspect 33) of aspects a, b, c, d and e refers to compounds of formula I according to aspects a, b, c, d and e, in which
R7 is quinolin-4-yl.

A further special subaspect (subaspect 34) of aspects a, b, c and d refers to compounds of formula I according to aspects a, b, c and d, in which
R8 is 1-4C-alkyl, phenyl, 2-4C-alkinyl, cyano, -CH₂-O-R81, phenylcarbonyl or -C(O)-N(R82)RB3, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl.

A further special subaspect (subaspect 35) of aspects a, b, c and d refers to compounds of formula I according to aspects a, b, c and d, in which
R8 is phenyl, cyano, phenylcarbonyl or -C(O)-N(R82)R83, in which
R82 is hydrogen, 1-4C-alkyl, 3-7C-cycloalkyl or phenyl,
R83 is hydrogen or 1-4C-alkyl, or
R82 and R83 together and with inclusion of the nitrogen atom, to which they are bound, form a pyrrolidinyl ring.

A further special subaspect (subaspect 36) of aspects a, b, c and d refers to compounds of formula I according to aspects a, b, c and d, in which
R8 is cyano.

A further special subaspect (subaspect 37) of aspects b, c, d and e refers to compounds of formula I according to aspects b, c, d and e, in which
R8 is carboxyl.

A further special subaspect (subaspect 38) of aspects a, b, c and d refers to compounds of formula I according to aspects a, b, c and d, in which
R4 is hydrogen,
R41 is hydrogen,
R5 is 1-2C-alkyl,
R51 is hydrogen,
   and
R8 is cyano.

A further special subaspect (subaspect 39) of aspects b, c and d refers to compounds of formula I according to aspects b, c and d, in which
R4 is hydrogen,
R41 is hydrogen,
R5 is 1-2C-alkyl,
R51 is hydrogen,
   and
R8 is -C(O)-OR9, in which
R9 is 1-2C-alkyl.

A further special subaspect (subaspect 40) of aspects a, b, c, d and e refers to compounds of formula I according to aspects a, b, c, d and e, in which
R4 is hydrogen,
R41 is hydrogen,
R5 is 1-2C-alkyl,
R51 is hydrogen,
   and
R6 is methyl, ethyl or methoxycabonylethyl.

A further special subaspect (subaspect 41) of aspects a, b, c and d refers to compounds of formula I according to aspects a, b, c and d, in which
R4 is hydrogen,
R41 is hydrogen,
R5 is 1-2C-alkyl,
R51 is hydrogen,
R6 is methyl, ethyl or methoxycabonylethyl,
   and
R8 is cyano.

A further special subaspect (subaspect 42) of aspects a, c and d refers to compounds of formula I according to aspects a, c and d, in which
R1 is halogen or 1-2C-alkoxy,
R2 is hydrogen or 1-2C-alkoxy,
R3 is 1-2C-alkoxy,
R4 is hydrogen,
R41 is hydrogen,
R5 is 1-2C-alkyl,
R51 is hydrogen,
R6 is methyl, ethyl or methoxycarbonylethyl
   an
R8 is cyano.

Special subaspects more worthy to be mentioned are the subaspects 11, 12, 15, 24, 25, 27, 28, 29, 30, 31, 32, 33, 36, 38, 40, 41 and 42.

Special subaspects in particular worthy to be mentioned are the subaspects 25, 36, 38, 40, 41 and 42.

Special subaspects in more particular worthy to be mentioned are the subaspects 41 and, especially, 42.

Compounds according to aspect a more worthy to be mentioned are those of formula I, in which
R1 is halogen, nitro, amino, 1-4C-alkyl, 1-4C-alkoxy, 1-4C-alkoxy-2-4C-alkoxy, or completely or predominantly fluorine-substituted 1-4C-alkoxy,
R2 is hydrogen or 1-4C-alkoxy,
R3 is hydrogen or 1-4C-alkoxy, or
R1 and R2 bound to the benzo ring moiety in ortho-position to each other together form a completely or predominantly fluorine-substituted 1-2C-alkylenedioxy bridge and R3 is hydrogen,
R4 is hydrogen,
R41 is hydrogen,
R5 is 1-4C-alkyl,
R51 is hydrogen or 1-4C-alkyl,
   or
R4 is hydrogen,
R41 is hydrogen,
R5 is 1-4C-alkoxycarbonyl,
R51 is hydrogen,
   or
R4 and R5 together form a 3-4C-alkylene bridge and R41 and R51 are both hydrogen,
R6 is 1-6C-alkyl or 1-4C-alkyl substituted by R61, in which
R61 is 1-4C-alkoxycarbonyl,
R7 is phenyl, Het2, R71- and/or R72- and/or R73-substituted phenyl, R74- and/or R75-substituted Het2, or naphthyl, in which
Het2 is a heteroaryl radical selected from the group consisting of furanyl, thiophenyl, pyrrolyl, pyridinyl, quinolyl, indolyl, benzothiophenyl and benzofuranyl,
R71 is hydroxyl, halogen, nitro, trifluoromethyl, 1-4C-alkyl, 1-4C-alkoxy, amino, mono- or di-1-4C-alkylamino, 1-4C-alkylsulphonylamino, tolylsulphonylamino or aryloxy, in which
aryl is R711-substituted phenyl, in which
R711 is halogen,
R72 is 1-4C-alkyl, 1-4C-alkoxy or 1-4C-alkoxycarbonyl,
R73 is 1-4C-alkyl or 1-4C-alkoxy,
R74 is 1-4C-alkyl, trifluoromethyl, 1-4C-alkoxy, 1-4C-alkoxycarbonyl, nitro, phenyl or phenyloxy,
R75 is 1-4C-alkyl,
R8 is phenyl, cyano, phenylcarbonyl or -C(O)-N(R82)R83, in which
R82 is hydrogen, 1-4C-alkyl, 3-7C-cycloalkyl or phenyl,
R83 is hydrogen or 1-4C-alkyl, or
R82 and R83 together and with inclusion of the nitrogen atom, to which they are bound, form a heterocyclic ring radical selected from the group consisting of pyrrolidinyl and piperidinyl;
   and the salts, stereoisomers, hydrates and hydrates of the salts of these compounds.

Compounds according to aspect a in particular worthy to be mentioned are those of formula I, in which
R1 is chlorine, fluorine, nitro, amino, methyl, methoxy, methoxyethoxy or difluoromethoxy,
R2 is hydrogen or methoxy,
R3 is hydrogen or methoxy, or
R1 and R2 bound to the benzo ring moiety in ortho-position to each other together form a difluoromethylenedioxy bridge and R3 is hydrogen,
   and none of R1, R2 and R3 is bound to the 10-position of the pyrrolo[2.1.a]isoquinoline ring,
R4 is hydrogen,
R41 is hydrogen,
R5 is methyl,
R51 is methyl,
   or
R4 is hydrogen,
R41 is hydrogen,
R5 is methoxycarbonylethyl,
R51 is hydrogen,
   or
R4 and R5 together form a tetramethylene (-CH₂-CH₂-CH₂-CH₂-) bridge and R41 and R51 are both hydrogen,
R6 is methyl, ethyl or methoxycarbonylethyl,
R7 is phenyl, Het2, R71- and/or R72- and/or R73-substituted phenyl, or naphthyl, in which
Het2 is indolyl, pyridinyl or quinolyl,
R71 is hydroxyl, chlorine, methoxy, dimethylamino, or aryloxy, in which
aryl is R711-substituted phenyl, in which
R711 is chlorine,
R72 is methyl, tert-butyl or methoxy,
R73 is methyl, tert-butyl or methoxy,
R8 is phenyl, cyano, phenylcarbonyl or -C(O)-N(R82)R83, in which
R82 is hydrogen, methyl, ethyl, iso-propyl, iso-butyl, cyclohexyl, cyclopropyl or phenyl,
R83 is hydrogen or methyl, or
R82 and R83 together and with inclusion of the nitrogen atom, to which they are bound, form a pyrrolidinyl radical;
   and the salts, stereoisomers, hydrates and hydrates of the salts of these compounds.

Compounds according to aspect b more worthy to be mentioned are those of formula I, in which
R1 is halogen, nitro, amino, 1-4C-alkyl, 1-4C-alkoxy-2-4C-alkoxy, or completely or predominantly fluorine-substituted 1-4C-alkoxy,
   with the provisio that R1 is not trifluoromethoxy,
R2 is hydrogen or 1-4C-alkoxy,
R3 is hydrogen or 1-4C-alkoxy, or
R1 and R2 bound to the benzo ring moiety in ortho-position to each other together form a completely or predominantly fluorine-substituted 1-2C-alkylenedioxy bridge and R3 is hydrogen,
R4 is hydrogen,
R41 is hydrogen,
R5 is 1-4C-alkyl,
R51 is hydrogen or 1-4C-alkyl,
   or
R4 is hydrogen,
R41 is hydrogen,
R5 is 1-4C-alkoxycarbonyl,
R51 is hydrogen,
   or
R4 and R5 together form a 3-4C-alkylene bridge and R41 and R51 are both hydrogen,
R6 is 1-6C-alkyl, formyl, or 1-4C-alkyl substituted by R61, in which
R61 is 1-4C-alkoxycarbonyl,
R7 is phenyl, Het2, R71- and/or R72- and/or R73-substituted phenyl, R74- and/or R75-substituted Het2, or naphthyl, in which
Het2 is a heteroaryl radical selected from the group consisting of furanyl, thiophenyl, pyrrolyl, pyridinyl, quinolyl, indolyl, benzothiophenyl and benzofuranyl,
R71 is hydroxyl, halogen, nitro, trifluoromethyl, 1-4C-alkyl, 1-4C-alkoxy, amino, mono- or di-1-4C-alkylamino, 1-4C-alkylsulphonylamino, tolylsulphonylamino or aryloxy, in which
aryl is R711-substituted phenyl, in which
R711 is halogen,
R72 is 1-4C-alkyl, 1-4C-alkoxy or 1-4C-alkoxycarbonyl,
R73 is 1-4C-alkyl or 1-4C-alkoxy,
R74 is 1-4C-alkyl, trifluoromethyl, 1-4C-alkoxy, 1-4C-alkoxycarbonyl, nitro, phenyl or phenyloxy,
R75 is 1-4C-alkyl,
R8 is phenyl, cyano, phenylcarbonyl, -C(O)-N(R82)R83 or -C(O)-OR9, in which
R82 is hydrogen, 1-4C-alkyl, 3-7C-cycloalkyl or phenyl,
R83 is hydrogen or 1-4C-alkyl, or
R82 and R83 together and with inclusion of the nitrogen atom, to which they are bound, form a heterocyclic ring radical selected from the group consisting of pyrrolidinyl and piperidinyl,
R9 is 1-4C-alkyl;
   and the salts, stereoisomers, hydrates and hydrates of the salts of these compounds.

Compounds according to aspect b in particular worthy to be mentioned are those of formula I, in which
R1 is chlorine, fluorine, nitro, amino, methyl, methoxyethoxy or difluoromethoxy,
R2 is hydrogen or methoxy,
R3 is hydrogen or methoxy, or
R1 and R2 bound to the benzo ring moiety in ortho-position to each other together form a difluoromethylenedioxy bridge and R3 is hydrogen,
   and none of R1, R2 and R3 is bound to the 10-position of the pyrrolo[2.1-a]isoquinoline ring,
R4 is hydrogen,
R41 is hydrogen,
R5 is methyl,
R51 is hydrogen or methyl,
   or
R4 is hydrogen,
R41 is hydrogen,
R5 is methoxycarbonyl,
R51 is hydrogen,
   or
R4 and R5 together form a tetramethylene (-CH₂-CH₂-CH₂-CH₂-) bridge and R41 and R51 are both hydrogen,
R6 is methyl, ethyl or methoxycarbonylethyl,
R7 is phenyl, Het2, R71- and/or R72- and/or R73-substituted phenyl, or naphthyl, in which
Het2 is indolyl, pyridinyl or quinolyl,
R71 is hydroxyl, chlorine; methoxy, dimethylamino, or aryloxy, in which
aryl is R711-substituted phenyl, in which
R711 is chlorine,
R72 is methyl, tert-butyl or methoxy,
R73 is methyl, tert-butyl or methoxy,
R8 is phenyl, cyano, phenylcarbonyl, -C(O)-N(R82)R83 or -C(O)-OR9, in which
R82 is hydrogen, methyl, ethyl, iso-propyl, iso-butyl, cyclohexyl, cyclopropyl or phenyl,
R83 is hydrogen or methyl, or
R82 and R83 together and with inclusion of the nitrogen atom, to which they are bound, form a pyrrolidinyl radical,
R9 is methyl or ethyl;
   and the salts, stereoisomers, hydrates and hydrates of the salts of these compounds.

Compounds according to aspect c more worthy to be mentioned are those of formula I, in which
R1 is halogen, nitro, amino, 1-4C-alkyl, 1-4C-alkoxy, 1-4C-alkoxy-2-4C-alkoxy, or completely or predominantly fluorine-substituted 1-4C-alkoxy,
R2 is 1-4C-alkoxy,
R3 is 1-4C-alkoxy,
R4 is hydrogen,
R41 is hydrogen,
R5 is 1-4C-alkyl,
R51 is hydrogen or 1-4C-alkyl,
   or
R4 is hydrogen,
R41 is hydrogen,
R5 is 1-4C-alkoxycarbonyl,
R51 is hydrogen,
   or
R4 and R5 together form a 3-4C-alkylene bridge and R41 and R51 are both hydrogen,
R6 is 1-6C-alkyl or 1-4C-alkyl substituted by R61, in which
R61 is 1-4C-alkoxycarbonyl,
R7 is phenyl, Het2, R71- and/or R72- and/or R73-substituted phenyl, R74- and/or R75-substituted Het2, or naphthyl, in which
Het2 is a heteroaryl radical selected from the group consisting of furanyl, thiophenyl, pyrrolyl, pyridinyl, quinolyl, indolyl, benzothiophenyl and benzofuranyl,
R71 is hydroxyl, halogen, nitro, trifluoromethyl, 1-4C-alkyl, 1-4C-alkoxy, amino, mono- or di-1-4C-alkylamino, 1-4C-alkylsulphonylamino, tolylsulphonylamino or aryloxy, in which
aryl is R711-substituted phenyl, in which
R711 is halogen,
R72 is 1-4C-alkyl, 1-4C-alkoxy or 1-4C-alkoxycarbonyl,
R73 is 1-4C-alkyl or 1-4C-alkoxy,
R74 is 1-4C-alkyl, trifluoromethyl, 1-4C-alkoxy, 1-4C-alkoxycarbonyl, nitro, phenyl or phenyloxy,
R75 is 1-4C-alkyl,
R8 is phenyl, cyano, phenylcarbonyl, -C(O)-N(R82)R83 or -C(O)-OR9, in which
R82 is hydrogen, 1-4C-alkyl, 3-7C-cycloalkyl or phenyl,
R83 is hydrogen or 1-4C-alkyl, or
R82 and R83 together and with inclusion of the nitrogen atom, to which they are bound, form a heterocyclic ring radical selected from the group consisting of pyrrolidinyl and piperidinyl,
R9 is 1-4C-alkyl;
   and the salts, stereoisomers, hydrates and hydrates of the salts of these compounds.

Compounds according to aspect c in particular worthy to be mentioned are those of formula I, in which
R1 is chlorine, fluorine, nitro, amino, methyl, methoxy, methoxyethoxy or difluoromethoxy,
R2 is methoxy,
R3 is methoxy,
   and none of R1, R2 and R3 is bound to the 10-position of the pyrrolo[2.1-a]isoquinoline ring,
R4 is hydrogen,
R41 is hydrogen,
R5 is methyl,
R51 is hydrogen or methyl,
   or
R4 is hydrogen,
R41 is hydrogen,
R5 is methoxycarbonyl,
R51 is hydrogen,
   or
R4 and R5 together form a tetramethylene (-CH₂-CH₂-CH₂-CH₂-) bridge and R41 and R51 are both hydrogen,
R6 is methyl, ethyl or methoxycarbonylethyl,
R7 is phenyl, Het2, R71- and/or R72- and/or R73-substituted phenyl, or naphthyl, in which
Het2 is indolyl, pyridinyl or quinolyl,
R71 is hydroxyl, chlorine, methoxy, dimethylamino, or aryloxy, in which
aryl is R711-substituted phenyl, in which
R711 is chlorine,
R72 is methyl, tert-butyl or methoxy,
R73 is methyl, tert-butyl or methoxy,
R8 is phenyl, cyano, phenylcarbonyl, -C(O)-N(R82)R83 or-C(O)-OR9, in which
R82 is hydrogen, methyl, ethyl, iso-propyl, iso-butyl, cyclohexyl, cyclopropyl or phenyl,
R83 is hydrogen or methyl, or
R82 and R83 together and with inclusion of the nitrogen atom, to which they are bound, form a pyrrolidinyl radical,
R9 is methyl or ethyl;
   and the salts, stereoisomers, hydrates and hydrates of the salts of these compounds.

Compounds according to aspect d more worthy to be mentioned are those of formula I, in which
R1 is halogen, nitro, amino, 1-4C-alkyl, 1-4C-alkoxy, 1-4C-alkoxy-2-4C-alkoxy, or completely or predominantly fluorine-substituted 1-4C-alkoxy,
R2 is hydrogen or 1-4C-alkoxy,
R3 is hydrogen or 1-4C-alkoxy, or
R1 and R2 bound to the benzo ring moiety in ortho-position to each other together form a completely or predominantly fluorine-substituted 1-2C-alkylenedioxy bridge and R3 is hydrogen,
R4 is hydrogen,
R41 is hydrogen,
R5 is 1-4C-alkyl,
R51 is hydrogen or 1-4C-alkyl,
   or
R4 is hydrogen,
R41 is hydrogen,
R5 is 1-4C-alkoxycarbonyl,
R51 is hydrogen,
   or
R4 and R5 together form a 3-4C-alkylene bridge and R41 and R51 are both hydrogen,
R6 is 1-6C-alkyl or 1-4C-alkyl substituted by R61, in which
R61 is 1-4C-alkoxycarbonyl,
R7 is phenyl, Het2, R71- and/or R72- and/or R73-substituted phenyl, R74- and/or R75-substituted Het2, or naphthyl, in which
Het2 is a heteroaryl radical selected from the group consisting of furanyl, thiophenyl, pyrrolyl, pyridinyl, quinolyl, indolyl, benzothiophenyl and benzofuranyl,
R71 is hydroxyl, halogen, nitro, trifluoromethyl, 1-4C-alkyl, 1-4C-alkoxy, amino, mono- or di-1-4C-alkylamino, 1-4C-alkylsulphonylamino, tolylsulphonylamino or aryloxy, in which
aryl is R711-substituted phenyl, in which
R711 is halogen,
R72 is 1-4C-alkyl, 1-4C-alkoxy or 1-4C-alkoxycarbonyl,
R73 is 1-4C-alkyl or 1-4C-alkoxy,
R74 is 1-4C-alkyl, trifluoromethyl, 1-4C-alkoxy, 1-4C-alkoxycarbonyl, nitro, phenyl or phenyloxy,
R75 is 1-4C-alkyl,
R8 is phenyl, cyano, phenylcarbonyl, -C(O)-N(R82)R83 or -C(O)-OR9, in which
R82 is hydrogen, 1-4C-alkyl, 3-7C-cycloalkyl or phenyl,
R83 is hydrogen or 1-4C-alkyl, or
R82 and R83 together and with inclusion of the nitrogen atom, to which they are bound, form a heterocyclic ring radical selected from the group consisting of pyrrolidinyl and piperidinyl,
R9 is 1-4C-alkyl;
   and the salts, stereoisomers, hydrates and hydrates of the salts of these compounds.

Compounds according to aspect d in particular worthy to be mentioned are those of formula I, in which
R1 is chlorine, fluorine, nitro, amino, methyl, methoxy, methoxyethoxy or difluoromethoxy,
R2 is hydrogen or methoxy,
R3 is hydrogen or methoxy, or
R1 and R2 bound to the benzo ring moiety in ortho-position to each other together form a difluoromethylenedioxy bridge and R3 is hydrogen,
   and none of R1, R2 and R3 is bound to the 10-position of the pyrrolo[2.1-a]isoquinoline ring,
R4 is hydrogen,
R41 is hydrogen,
R5 is methyl,
R51 is hydrogen or methyl,
   or
R4 is hydrogen,
R41 is hydrogen,
R5 is methoxycarbonyl,
R51 is hydrogen,
   or
R4 and R5 together form a tetramethylene (-CH₂-CH₂-CH₂-CH₂-) bridge and R41 and R51 are both hydrogen,
R6 is methyl, ethyl or methoxycarbonylethyl,
R7 is phenyl, Het2, R71- and/or R72- and/or R73-substituted phenyl, or naphthyl, in which
Het2 is a heteroaryl radical selected from the group consisting of furanyl, thiophenyl, pyrrolyl, pyridinyl, quinolyl, indolyl, benzothiophenyl and benzofuranyl,
R71 is hydroxyl, chlorine, methoxy, dimethylamino, or aryloxy, in which
aryl is R711-substituted phenyl, in which
R711 is chlorine,
R72 is methyl, tert-butyl or methoxy,
R73 is methyl, tert-butyl or methoxy,
R8 is phenyl, cyano, phenylcarbonyl, -C(O)-N(R82)R83 or-C(O)-OR9, in which
R82 is hydrogen, methyl, ethyl, iso-propyl, iso-butyl, cyclohexyl, cyclopropyl or phenyl,
R83 is hydrogen or methyl, or
R82 and R83 together and with inclusion of the nitrogen atom, to which they are bound, form a pyrrolidinyl radical,
R9 is methyl or ethyl;
   and the salts, stereoisomers, hydrates and hydrates of the salts of these compounds.

A special interest in the compounds according to this invention refers to those compounds of formula I which are included -within the scope of this invention- by one or, when possible, by more of the following special embodiments:

A special embodiment (embodiment 1) of the compounds according to this invention refers to those compounds of formula I, in which
R8 is -C(O)-OR9, in which
R9 is 1-4C-alkyl.

Another special embodiment (embodiment 2) of the compounds according to this invention refers to those compounds of formula I, in which
R8 is cyano.

Another special embodiment (embodiment 3) of the compounds according to this invention refers to those compounds of formula I, in which
R4 is hydrogen, and
R41 is hydrogen.

Another special embodiment (embodiment 4) of the compounds according to this invention refers to those compounds of formula I, in which
R5 is 1-4C-alkyl, cyano or 1-4C-alkoxycarbonyl, and
R51 is hydrogen.

Another special embodiment (embodiment 5) of the compounds according to this invention refers to those compounds of formula I, in which
R4 is hydrogen,
R41 is hydrogen,
R5 is cyano; or, in particular, 1-4C-alkyl, such as e.g. 1-2C-alkyl, especially methyl; and
R51 is 1-4C-alkyl, such as e.g. 1-2C-alkyl, especially methyl; or, in particular hydrogen.

Another special embodiment (embodiment 6) of the compounds according to this invention refers to those compounds of formula I, in which
R5 is 1-4C-alkyl, such as e.g. 1-2C-alkyl, in particular methyl, and
R51 is hydrogen.

Another special embodiment (embodiment 7) of the compounds according to this invention refers to those compounds of formula I, in which
R4 is hydrogen,
R41 is hydrogen,
R5 is 1-4C-alkyl, such as e.g. 1-2C-alkyl, in particular methyl, and
R51 is hydrogen.

Another special embodiment (embodiment 8) of the compounds according to this invention refers to those compounds of formula I, in which
R4 is hydrogen,
R41 is hydrogen,
R5 is cyano, and
R51 is hydrogen.

Another special embodiment (embodiment 9) of the compounds according to this invention refers to those compounds of formula I, in which
none of R1, R2 and R3 is bound to the 10-position of the pyrrolo[2.1-a]isoquinoline ring.

Another special embodiment (embodiment 10) of the compounds according to this invention refers to those compounds of formula I, in which
none of R1 and R2 is bound to the 7- or 10-position of the pyrrolo[2.1-a]isoquinoline ring, and R3 is hydrogen.

Another special embodiment (embodiment 11) of the compounds according to this invention refers to those compounds of formula I, in which
none of R1 and R3 is bound to the 7- or 10-position of the pyrrolo[2.1-a]isoquinoline ring, and none of R1 and R3 is hydrogen, and
R2 is bound to the 7-position of the pyrrolo[2.1-a]isoquinoline ring.

Another special embodiment (embodiment 12) of the compounds according to this invention refers to those compounds of formula I, in which
R1 is 1-4C-alkoxy, 1-4C-alkoxy-2-4C-alkoxy, 3-7C-cycloalkoxy or 3-7C-cycloalkylmethoxy, in particular
R1 is 1-2C-alkoxy, 1-2C-alkoxy-3-4C-alkoxy, 3-5C-cycloalkoxy or 3-5C-cycloalkylmethoxy, in more particular
R1 is 1-2C-alkoxy, such as e.g. methoxy.

Another special embodiment (embodiment 13) of the compounds according to this invention refers to those compounds of formula I, in which
R2 is chlorine, or, in particular fluorine.

Another special embodiment (embodiment 14) of the compounds according to this invention refers to those compounds of formula I, in which
R3 is 1-4C-alkoxy, in particular 1-2C-alkoxy, such as e.g. methoxy.

Another special embodiment (embodiment 15) of the compounds according to this invention refers to those compounds of formula I, in which
R1 is 1-4C-alkoxy, 1-4C-alkoxy-2-4C-alkoxy, 3-7C-cycloalkoxy or 3-7C-cycloalkylmethoxy,
R2 is 1-4C-alkoxy,
R3 is hydrogen, and
   none of R1 and R2 is bound to the 7- or 10-position of the pyrrolo[2.1-a]isoquinoline ring.

Another special embodiment (embodiment 16) of the compounds according to this invention refers to those compounds of formula I, in which
R1 is bound to the 8-position of the pyrrolo[2.1-a]isoquinoline ring, and is 1-4C-alkoxy, such as e.g. 1-2C-alkoxy,
R2 is bound to the 9-position of the pyrrolo[2.1-a]isoquinoline ring, and is 1-4C-alkoxy, such as e.g. 1-2C-alkoxy, and
R3 is hydrogen.

Another special embodiment (embodiment 17) of the compounds according to this invention refers to those compounds of formula I, in which
R1 is 1-4C-alkoxy, 1-4C-alkoxy-2-4C-alkoxy, 3-7C-cycloalkoxy or 3-7C-cycloalkylmethoxy,
R2 is halogen, and
R3 is 1-4C-alkoxy.

Another special embodiment (embodiment 18) of the compounds according to this invention refers to those compounds of formula I, which are from formulae la or Ib shown below.

Another special embodiment (embodiment 19) of the compounds according to this invention refers to those compounds of formula I, in which
R1 is 1-2C-alkoxy, 1-2C-alkoxy-2-3C-alkoxy, 3-5C-cycloalkoxy or 3-5C-cycloalkylmethoxy,
R2 is halogen, such as e.g. chlorine or fluorine,
R3 is 1-2C-alkoxy,
   and none of R1, R2 and R3 is bound to the 10-position of the pyrrolo[2.1-a]isoquinoline ring.

Another special embodiment (embodiment 20) of the compounds according to this invention refers to those compounds of formula I, in which
R1 is 1-2C-alkoxy, 1-2C-alkoxy-2-3C-alkoxy, 3-5C-cycloalkoxy or 3-5C-cycloalkylmethoxy,
R2 is bound to the 7-position of the pyrrolo[2.1-a]isoquinoline ring, and is chlorine or fluorine,
R3 is 1-2C-alkoxy,
   and none of R1 and R3 is bound to the 10-position of the pyrrolo[2.1-a]isoquinoline ring.

Another special embodiment (embodiment 21) of the compounds according to this invention refers to those compounds of formula I, in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine, and
R3 is bound to the 9-position of the pyrrolo[2.1-a]isoquinoline ring, and is 1-2C-alkoxy, such as e.g. methoxy.

Another special embodiment (embodiment 22) of the compounds according to this invention refers to those compounds of formulae Ia or Ib as shown below, in which
as a first alternative,
R1 is hydrogen,
R2 is chlorine or fluorine,
R3 is methoxy or ethoxy,
   or, as a second alternative,
R1 is hydrogen,
R2 is methoxy or ethoxy,
R3 is methoxy or ethoxy,
   or, as a third alternative,
R1 is methoxy or ethoxy,
R2 is chlorine or fluorine,
R3 is methoxy or ethoxy,
   or, as a fourth alternative,
R1 is chlorine or fluorine,
R2 is methoxy or ethoxy,
R3 is methoxy or ethoxy,
   or, as a fifth alternative,
R1 is methoxy or ethoxy,
R2 is methoxy or ethoxy,
R3 is methoxy or ethoxy.

Another special embodiment (embodiment 23) of the compounds according to this invention refers to those compounds of formula I, in which
R7 is naphthyl (such as e.g. naphthalen-1-yl), or R71- and/or R72- and/or R73-substituted phenyl, such as, for example,
   4-hydroxy-3,5-dimethylphenyl, 4-methoxy-3,5-dimethylphenyl, 4-carboxy-phenyl, 4-carbamoylphenyl, 2-methyl-4-hydroxy-phenyl, 4-amino-phenyl, 4-(2H-tetrazol-5-yl)-phenyl, 4-mopholinosulphonylamino-phenyl, 4-methylsulphonylamino-phenyl, or 2-fluoro-3,4-dimethoxy-phenyl.

Another special embodiment (embodiment 24) of the compounds according to this invention refers to those compounds of formula I, in which
either
R7 is Het2, in which
Het2 is a monocyclic or fused bicyclic 5- to 10-membered heteroaryl radical comprising one to three heteroatoms each of which is selected from a group consisting of nitrogen, oxygen and sulfur, and which optionally contains a benzene ring,
   or
   a fused bicyclic 9- or 10-membered, partially saturated heterocyclic ring radical containing a benzene ring and comprising one or two heteroatoms, each of which is selected from a group consisting of nitrogen, oxygen and sulfur,
   or
   N-oxy-pyridyl;
   such as, for example,
   pyridyl, indolyl, quinolinyl, or indolinyl,
   e.g. pyridin-4-yl, indol-3-yl, indol-5-yl, quinolin-4-yl, N-oxy-pyridin-4-yl, or indolin-5-yl;
   or
R7 is R74-substituted Het2, in which
Het2 has one of the meanings as defined afore,
R74 is 1-4C-alkyl, arylsulphonyl, 14C-alkylsulphonyl, or -S(O)₂-N(R712)R713, in which
aryl is phenyl, or R711-substituted phenyl, in which
R711 is 1-4C-alkyl,
R712 is 1-4C-alkyl,
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl;
   such as, for example,
   1-tolylsulphonyl-pyrrol-3-yl, 1-tolylsulphonyl-indol-3-yl, 1-phenylsulphonyl-indol-3-yl, 1-methylsulphonyl-indol-3-yl, 2-methyl-pyridin-4-yl, 3-methyl-pyridin-4-yl, 1-dimethylaminosulphonylindol-3-yl, 1-morpholinosulphonyl-indol-3-yl.

Another special embodiment (embodiment 25) of the compounds according to this invention refers to those compounds of formula I, in which
R7 is 4-hydroxy-3,5-dimethylphenyl.

Another special embodiment (embodiment 26) of the compounds according to this invention refers to those compounds of formula I, in which
R7 is Het2, in which
Het2 is a fused bicyclic 9- or 10-membered heteroaryl radical comprising one to three heteroatoms, each of which is selected from a group consisting of nitrogen, oxygen and sulfur, which optionally contains a benzene ring,
   such as e.g. quinolyl or indolyl.

Another special embodiment (embodiment 27) of the compounds according to this invention refers to those compounds of formula I, in which
R6 is 1-4C-alkyl, such as e.g. methyl.

Another special embodiment (embodiment 28) of the compounds according to this invention refers to those compounds of formula I, in which
R6 is 1-4C-alkyl substituted by 1-4C-alkoxycarbonyl, such as e.g. 2-methoxycarbonyl-ethyl.

Another special embodiment (embodiment 29) of the compounds according to this invention refers to those compounds of formula I, in which
R1 is 1-2C-alkoxy, 1-2C-alkoxy-2-3C-alkoxy, 3-5C-cycloalkoxy, or 3-5C-cycloalkylmethoxy,
R2 is chlorine or fluorine,
R3 is 1-2C-alkoxy,
   and none of R1, R2 and R3 is bound to the 10-position of the pyrrolo[2.1-a]isoquinoline ring, and
R4 is hydrogen,
R41 is hydrogen,
R5 is 1-2C-alkyl or cyano,
R51 is hydrogen,
   and
R8 is cyano;
   in particular
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy,
   and
R4 is hydrogen,
R41 is hydrogen,
R5 is 1-2C-alkyl or cyano,
R51 is hydrogen,
   and
R8 is cyano;
   in more particular
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is methyl,
R51 is hydrogen,
   and
R8 is cyano.

Another special embodiment (embodiment 30) of the compounds according to this invention refers to those compounds of formula I, in which
R1 is 1-2C-alkoxy, 1-2C-alkoxy-2-3C-alkoxy, 3-5C-cycloalkoxy, or 3-5C-cycloalkylmethoxy,
R2 is hydrogen,
R3 is 1-2C-alkoxy,
   and none of R1 and R2 is bound to the 7- or 10-position of the pyrrolo[2.1-a]isoquinoline ring, and
R4 is hydrogen,
R41 is hydrogen,
R5 is 1-2C-alkyl or cyano,
R51 is hydrogen,
   and
R8 is cyano;
   in particular
R1 is bound to the 8-position of the pyrrolo[2.1-a]isoquinoline ring, and is 1-2C-alkoxy,
R2 is hydrogen,
R3 is bound to the 9-position of the pyrrolo[2.1-a]isoquinoline ring, and is 1-2C-alkoxy,
   and
R4 is hydrogen,
R41 is hydrogen,
R5 is 1-2C-alkyl or cyano,
R51 is hydrogen,
   and
R8 is cyano;
   in more particular
R1 is bound to the 8-position of the pyrrolo[2.1-a]isoquinoline ring, and is 1-2C-alkoxy, such as e.g. methoxy,
R2 is hydrogen,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is methyl,
R51 is hydrogen,
   and
R8 is cyano.

Another special embodiment (embodiment 31) of the compounds according to this invention refers to those compounds of formula I, in which
R1 is 1-2C-alkoxy, 1-2C-alkoxy-2-3C-alkoxy, 3-5C-cycloalkoxy, or 3-5C-cycloalkylmethoxy,
R2 is chlorine or fluorine,
R3 is 1-2C-alkoxy,
   and none of R1, R2 and R3 is bound to the 10-position of the pyrrolo[2.1-a]isoquinoline ring, and
R4 is hydrogen,
R41 is hydrogen,
R5 is 1-2C-alkyl or cyano,
R51 is hydrogen,
   and
R6 is 1-2C-alkyl or 2-methoxycarbonylethyl,
   and
R8 is cyano;
   in particular
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy,
   and
R4 is hydrogen,
R41 is hydrogen,
R5 is 1-2C-alkyl or cyano,
R51 is hydrogen,
   and
R6 is 1-2C-alkyl or 2-methoxycarbonylethyl,
   and
R8 is cyano;
   in more particular
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is fluorine,
R3 is bound to the 9-position of the pyrrolo[2.1-a]isoquinoline ring, and is 1-2C-alkoxy, such as e.g. methoxy,
   and
R4 is hydrogen,
R41 is hydrogen,
R5 is methyl,
R51 is hydrogen,
   and
R6 is methyl,
   and
R8 is cyano.

Another special embodiment (embodiment 32) of the compounds according to this invention refers to those compounds of formula la as shown below, in which
R2 is methoxy,
R3 is methoxy,
R4 is hydrogen,
R41 is hydrogen,
R51 is hydrogen,
   and in which the following combinations 1.) to 75.) of the substituent meanings for R1, R5, R6 and R8 shown in Table X apply:

**Table X:**

| | R1 | R5 | R6 | R8 |
|---|---|---|---|---|
| 1.) | hydrogen | methyl | methyl | cyano |
| 2.) | hydrogen | methyl | methyl | ethoxycarbonyl |
| 3.) | hydrogen | methyl | 2-methoxycarbonylethyl | cyano |
| 4.) | hydrogen | methyl | 2-methoxycarbonylethyl | ethoxycarbonyl |
| 5.) | fluorine | methyl | methyl | cyano |
| 6.) | fluorine | methyl | methyl | ethoxycarbonyl |
| 7.) | fluorine | methyl | 2-methoxycarbonylethyl | cyano |
| 8.) | fluorine | methyl | 2-methoxycarbonylethyl | ethoxycarbonyl |
| 9.) | hydrogen | cyano | methyl | cyano |
| 10.) | hydrogen | cyano | methyl | ethoxycarbonyl |
| 11.) | hydrogen | cyano | 2-methoxycarbonylethyl | cyano |
| 12.) | hydrogen | cyano | 2-methoxycarbonylethyl | ethoxycarbonyl |
| 13.) | fluorine | cyano | methyl | cyano |
| 14.) | fluorine | cyano | methyl | ethoxycarbonyl |
| 15.) | fluorine | cyano | 2-methoxycarbonylethyl | cyano |
| 16.) | fluorine | cyano | 2-methoxycarbonylethyl | ethoxycarbonyl |
| 17.) | chlorine | methyl | methyl | cyano |
| 18.) | chlorine | methyl | methyl | ethoxycarbonyl |
| 19.) | chlorine | methyl | 2-methoxycarbonylethyl | cyano |
| 20.) | chlorine | methyl | 2-methoxycarbonylethyl | ethoxycarbonyl |
| 21.) | chlorine | cyano | methyl | cyano |
| 22.) | chlorine | cyano | methyl | ethoxycarbonyl |
| 23.) | chlorine | cyano | 2-methoxycarbonylethyl | cyano |
| 24.) | chlorine | cyano | 2-methoxycarbonylethyl | ethoxycarbonyl |
| 25.) | hydrogen | methyl | methyl | methoxycarbonyl |
| 26.) | hydrogen | methyl | 2-methoxycarbonylethyl | methoxycarbonyl |
| 27.) | fluorine | methyl | methyl | methoxycarbonyl |
| 28.) | fluorine | methyl | 2-methoxycarbonylethyl | methoxycarbonyl |
| 29.) | hydrogen | cyano | methyl | methoxycarbonyl |
| 30.) | hydrogen | cyano | 2-methoxycarbonylethyl | methoxycarbonyl |
| 31.) | fluorine | cyano | methyl | methoxycarbonyl |
| 32.) | fluorine | cyano | 2-methoxycarbonylethyl | methoxycarbonyl |
| 33.) | chlorine | methyl | methyl | methoxycarbonyl |
| 34.) | chlorine | methyl | 2-methoxycarbonylethyl | methoxycarbonyl |
| 35.) | chlorine | cyano | methyl | methoxycarbonyl |
| 36.) | chlorine | cyano | 2-methoxycarbonylethyl | methoxycarbonyl |
| 37.) | hydrogen | methyl | ethyl | cyano |
| 38.) | hydrogen | methyl | ethyl | ethoxycarbonyl |
| 39.) | fluorine | methyl | ethyl | cyano |
| 40.) | fluorine | methyl | ethyl | ethoxycarbonyl |
| 41.) | hydrogen | cyano | ethyl | cyano |
| 42.) | hydrogen | cyano | ethyl | ethoxycarbonyl |
| 43.) | fluorine | cyano | ethyl | cyano |
| 44.) | fluorine | cyano | ethyl | ethoxycarbonyl |
| 45.) | chlorine | methyl | ethyl | cyano |
| 46.) | chlorine | methyl | ethyl | ethoxycarbonyl |
| 47.) | chlorine | cyano | ethyl | cyano |
| 48.) | chlorine | cyano | ethyl | ethoxycarbonyl |
| 49.) | hydrogen | methyl | ethyl | methoxycarbonyl |
| 50.) | fluorine | methyl | ethyl | methoxycarbonyl |
| 51.) | hydrogen | cyano | ethyl | methoxycarbonyl |
| 52.) | fluorine | cyano | ethyl | methoxycarbonyl |
| 53.) | chlorine | methyl | ethyl | methoxycarbonyl |
| 54.) | chlorine | cyano | ethyl | methoxycarbonyl |

wherein those combinations, in which R8 is cyano, are more worthy to be mentioned, and
wherein the combinations 1 to 5, 10, 18 and 25 are to be emphasized, and
wherein the combinations 1, 3 and 5 are more to be emphasized, and
wherein the combination 5 is in particular to be emphasized.

Another special embodiment (embodiment 33) of the compounds according to this invention refers to those compounds of formula I, in which
Het2 is a fused bicyclic 9- or 10-membered, partially saturated heterocyclic ring radical containing a benzene ring and comprising one or two heteroatoms, each of which is selected from a group consisting of nitrogen, oxygen and sulfur,
   or
   N-oxy-pyridyl.

Another special embodiment (embodiment 34) of the compounds according to this invention refers to those compounds of formula I, in which
R71 is mono- or di-1-4C-alkylaminocarbonyl, carbamoyl, tetrazolyl, or -N(H)S(O)₂-N(R712)R713, in which
R712 is 1-4C-alkyl,
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl.

Another special embodiment (embodiment 34) of the compounds according to this invention refers to those compounds of formula I, in which
R74 is phenyl-1-4C-alkyl, arylsulphonyl, 1-4C-alkylsulphonyl, or -S(O)₂-N(R712)R713, in which
R712 is 1-4C-alkyl,
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl.

A notable embodiment (embodiment a) of variant 1 the present invention includes compounds of formula I, in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen,
R41 is hydrogen,
R5 is 1-4C-alkyl,
R51 is hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which
R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is cyano,
   and the salts, stereoisomers, hydrates and hydrates of the salts of these compounds.

A further notable embodiment (embodiment b) of variant 1 of the present invention includes compounds of formula **I,** in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy,
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen,
R41 is hydrogen,
R5 is 1-2C-alkyl,
R51 is hydrogen, or
R4 and R5 together form a tetramethylene (-CH₂-CH₂-CH₂-CH₂-) bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or-N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which
R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-14C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(RS2)R83 or -C(O)-OR9, in which
R81 is hydrogen, 1-4C-alkyl, 1-4C-alkoxy-2-4-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl,
R83 is hydrogen or 1-4C-alkyl, or
R82 and R83 together and with inclusion of the nitrogen atom, to which they are bound, form a heterocyclic ring radical selected from the group consisting of pyrrolidinyl, piperidinyl, morpholinyl or N-(1-4C-alkyl)-piperazinyl,
R9 is hydrogen or 1-4C-alkyl,
   and the salts, stereoisomers, hydrates and hydrates of the salts of these compounds.

A special subclass of embodiment b includes compounds of formulae Ia or Ib 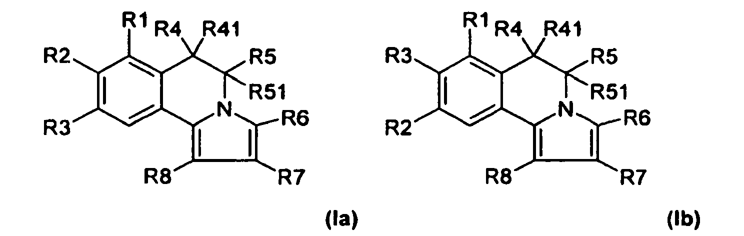 in which,
as a first alternative,
R1 is hydrogen,
R2 is chlorine or fluorine,
R3 is methoxy or ethoxy,
   or, as a second alternative,
R1 is hydrogen,
R2 is methoxy or ethoxy,
R3 is methoxy or ethoxy,
   or, as a third alternative,
R1 is methoxy or ethoxy,
R2 is chlorine or fluorine,
R3 is methoxy or ethoxy,
   or, as a fourth alternative,
R1 is chlorine or fluorine,
R2 is methoxy or ethoxy,
R3 is methoxy or ethoxy,
   or, as a fifth alternative,
R1 is methoxy or ethoxy,
R2 is methoxy or ethoxy,
R3 is methoxy or ethoxy,
R4 is hydrogen,
R41 is hydrogen,
R5 is ethyl or, in particular, methyl,
R51 is hydrogen,
R6 is methyl, ethyl or methoxycarbonylethyl,
R7 is Het2, R74- and/or R75-substituted Het2, or hydroxy-dimethyl-phenyl, in which
Het2 is pyridinyl or quinolinyl,
R74 is halogen, 1-4C-alkyl, trifluoromethyl, 1-4C-alkoxy, cyano, amino, mono- or di-1-4C-alkylamino, . 1-4C-alkoxycarbonyl, carboxyl, nitro, phenyl or phenyloxy,
R75 is 1-4C-alkyl.
R8 is -C(O)-OR9, in which
R9 is 1-4C-alkyl,
   and the salts, stereoisomers, hydrates and hydrates of the salts of these compounds.

Compounds according to embodiments a or b more worthy to be mentioned are compounds of formula I, in which
R1 is halogen or 1-4C-alkoxy,
R2 is hydrogen, halogen or 1-4C-alkoxy,
R3 is 1-4C-alkoxy,
R4 is hydrogen,
R41 is hydrogen,
R5 is 1-2C-alkyl,
R51 is hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which
R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, in which
Het2 is a monocyclic or fused bicyclic 5 to 10-membered heteroaryl radical comprising one to three heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, or completely or predominantly fluorine-substituted 1-4C-alkoxy, in which
aryl is phenyl or R711-substituted phenyl, in which
R711 is halogen, 1-4C-alkyl, 1-4C-alkoxy, nitro or cyano,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl or phenyloxy,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is cyano,
   and the salts, stereoisomers, hydrates and hydrates of the salts of these compounds.

Compounds according to embodiments a or b in particular worthy to be mentioned are compounds of formulae Ia or Ib,
in which,
as a first alternative,
R1 is hydrogen,
R2 is chlorine or fluorine,
R3 is methoxy or ethoxy,
   or, as a second alternative,
R1 is hydrogen,
R2 is methoxy or ethoxy,
R3 is methoxy or ethoxy,
   or, as a third alternative,
R1 is methoxy or ethoxy,
R2 is chlorine or fluorine,
R3 is methoxy or ethoxy,
   or, as a fourth alternative,
R1 is chlorine or fluorine,
R2 is methoxy or ethoxy,
R3 is methoxy or ethoxy,
   or, as a fifth alternative,
R1 is methoxy or ethoxy,
R2 is methoxy or ethoxy,
R3 is methoxy or ethoxy,
R4 is hydrogen,
R41 is hydrogen,
R5 is ethyl or, in particular, methyl,
R51 is hydrogen,
R6 is methyl, ethyl or methoxycarbonylethyl,
R7 is Het2, R74- and/or R75-substituted Het2, or hydroxy-dimethyl-phenyl, in which
Het2 is pyridinyl or quinolinyl,
R74 is halogen, 1-4C-alkyl, trifluoromethyl, 1-4C-alkoxy, cyano, amino, mono- or di-1-4C-alkylamino, 1-4C-alkoxycarbonyl, carboxyl, nitro, phenyl or phenyloxy,
R75 is 1-4C-alkyl.
R8 is cyano,
   and the salts, stereoisomers, hydrates and hydrates of the salts of these compounds.

As exemplary compounds according to variant 1 of this invention may be mentioned any compound selected from the group consisting of:
1. 2-(4-Hydroxy-3,5-dimethyl-phenyl)-8,9-dimethoxy-3,5,5-trimethyl-5,6-dihydro-pyrrolo[2,1-α]isoquinoline-1-carboxylic acid ethyl ester
2. 8,9-Dimethoxy-3,5,5-trimethyl-2-(3,4,5-trimethoxy-phenyl)-5,6-dihydro-pyrrolo[2,1-α]isoquinoline-1-carboxylic acid ethyl ester
3. 2-[3-(4-Chloro-phenoxy)-phenyl]-8,9-dimethoxy-3,5,5-trimethyl-5,6-dihydro-pyrrolo[2,1-a]isoquinoline-1-carboxylic acid ethyl ester
4. 2-(3-Dimethylamino-phenyl)-8,9-dimethoxy-3,5,5-trimethyl-5,6-dihydro-pyrrolo[2,1-a]isoquinoline-1-carboxylic acid ethyl ester
5. (5RS)- (4-Hydroxy-3,5-dimethyl-phenyl)-8,9-dimethoxy-3,5-dimethyl-5,6-dihydro-pyrrolo[2,1-a]isoquinoline-1-carboxylic acid ethyl ester
6. (5RS)-5-Ethyl-2-(4-hydroxy-3,5-dimethyl-phenyl)-8,9-dimethoxy-3-methyl-5,6-dihydropyrrolo[2,1-a]isoquinoline-1-carboxylic acid ethyl ester
7. (SRS)-2-Chloro-5-ethyl-8,9-dimethoxy-3-methyl-5,6-dihydro-pyrrolo[2,1-a]isoquinoline-1-carboxylic acid ethyl ester
8. (4aRS,8aRS)-cis-2-(4-hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
9. (5RS)-3-Ethyl-2-(4-hydroxy-3,5-dimethyl-phenyl)-8,9-dimethoxy-5-methyl-5,6-dihydropyrrolo[2,1-a]isoquinoline-1-carboxylic acid ethyl ester
10. (5RS)-8,9-Dimethoxy-3,5-dimethyl-2-(3,4,5-trimethoxy-phenyl)-5,6-dihydro-pyrrolo[2,1-a]isoquinoline-1-carboxylic acid ethyl ester
11. (5RS)-8,9-Dimethoxy-3,5-dimethyl-2-naphthalen-1-yl-5,6-dihydro-pyrrolo[2,1-a]isoquinoline-1-carboxylic acid ethyl ester
12. (4aRS,8aRS)-cis-10,11-Dimethoxy-3-methyl-2-naphthalen-1-yl-4a,5,6,7,8,8a-hexahydropyrrolo[2,1-f]phenanthridine-1-carboxylic acid ethyl ester
13. (4aRS,8aRS)-cis-10,11-Dimethoxy-3-methyl-2-quinolin-4-yl-4a,5,6,7,8,8a-hexahydropyrrolo[2,1-f]phenanthridine-1-carboxylic acid ethyl ester
14. (4aR,8aR)-10,11-Dimethoxy-3-methyl-2-quinolin-4-yl-4a,5,6,7,8,8a-hexahydro-pyrrolo[2,1-f]phenanthridine-1-carboxylic acid ethyl ester
15. (4aR,8aR)-10,11-Dimethoxy-3-methyl-2-naphthaten-1-yl-4a,5,6,7,8,8a-hexahydro-pyrrolo[2,1 - f]phenanthridine-1-carboxylic acid ethyl ester
16. (4aR,8aR)-2-(4-Hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
17. (5RS)-5-Ethyl-8,9-dimethoxy-3-methyl-2-naphthalen-1-yl-5,6-dihydro-pyrrolo[2,1-a]isoquinoline-carboxylic acid ethyl ester
18. (5RS)-2-(4-Hydroxy-3,5-dimethyl-phenyl)-7,8,9-trimethoxy-3,5-dimethyl-5,6-dihydro-pyrrolo[2,1-a]isoquinoline-1-carboxylic acid ethyl ester
19. 2-(4-Hydroxy-3,5-dimethyl-phenyl)-8,9-dimethoxy-5,6-dihydro-pyrrolo[2,1-a]isoquinoline-1,5-dicarboxylic acid 1-ethyl 5-methyl ester
20. (5RS)-8,9-Dimethoxy-3-(2-methoxycarbonyl-ethyl)-5-methyl-2-naphthalen-1-yl-5,6-dihydropyrrolo[2,1-a]isoquinoline-1-carboxylic acid ethyl ester
21. 8,9-Dimethoxy-3,5-dimethyl-2-pyridin-4-yl-5,6-dihydro-pyrrolo[2,1-a]isoquinoline-1-carbonitrile
22. 3-[1-Cyano-2-(4-hydroxy-3,5-dimethyl)-8,9-dimethoxy-5-methyl-5,6-dihydro-pyrrolo[2,1-a]isoquinolin3-yl]-propionic acid methyl ester
23. 2-(4-Hydroxy-3,5-dimethyl-phenyl)-8,9-dimethoxy-3,5-dimethyl-5,6-dihydro-pyrrolo[2,1-a]isoquinoline-1-carbonitrile
or a salt, stereoisomer, hydrate or hydrate of a salt thereof.

As further exemplary compounds according to this invention may be mentioned any compound selected from the group consisting of those compounds individualized and listed as Examples 29 to 69 in the following examples, or a salt, stereoisomer, hydrate or hydrate of a salt thereof.

As exemplary compounds according to this invention the following compounds of formula Ic, and the salts, stereoisomers, hydrates or hydrates of the salts thereof, 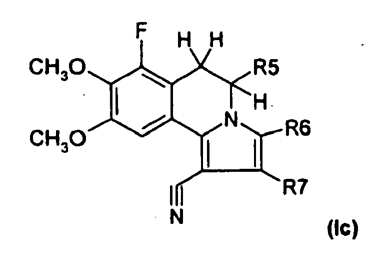 are notably to be mentioned by means of the substituent meanings for R5, R6 and R7 in the following Tables A1, A2, A3 and A4:

**Table A1:**

| Example No. | R5 | R6 | R7 |
|---|---|---|---|
| 70. | -CH₃ | -CH₃ | 4-methoxy-3,5-dimethylphenyl |
| 71. | -CH₃ | -CH₃ | 4-carboxy-phenyl |
| 72. | -CH₃ | -CH₃ | 2-methyl-4-hydroxy-phenyl |
| 73. | -CH₃ | -CH₃ | 4-amino-phenyl |
| 74. | -CH₃ | -CH₃ | 4-(2H-tetrazol-5-yl)-phenyl |
| 75. | -CH₃ | -CH₃ | 4-morpholino-sulphonylamino-phenyl |
| 76. | -CH₃ | -CH₃ | 4-methylsulphonylamino-phenyl |
| 77. | -CH₃ | -CH₃ | pyridin-4-yl |
| 78. | -CH₃ | -CH₃ | quinolin-4-yl |
| 79. | -CH₃ | -CH₃ | 2-methyl-pyridin-4-yl |
| 80. | -CH₃ | -CH₃ | 3-methyl-pyridin-4-yl |
| 81. | -CH₃ | -CH₃ | 1-tolylsulphonyl-pyrrol-3-yl |
| 82. | -CH₃ | -CH₃ | 1-phenylsulphonyl-indol-3-yl |
| 83. | -CH₃ | -CH₃ | 1-methylsulphonyl-indol-3-yl |
| 84. | -CH₃ | -CH₃ | 1-dimethylaminosulphonyl-indol-3-yl |
| 85. | -CH₃ | -CH₃ | 1-morpholinosulphonyl-indol-3-yl |

**Table A2:**

| Example No. | R5 | R6 | R7 |
|---|---|---|---|
| 86. | -CN | -CH₃ | 4-hydroxy-3,5-dimethylphenyl |
| 87. | -CN | -CH₃ | 4-methoxy-3,5-dimethylphenyl |
| 88. | -CN | -CH₃ | 4-carboxy-phenyl |
| 89. | -CN | -CH₃ | 2-methyl-4-hydroxy-phenyl |
| 90. | -CN | -CH₃ | 4-amino-phenyl |
| 91. | -CN | -CH₃ | 4-(2H-tetrazol-5-yl)-phenyl |
| 92. | -CN | -CH₃ | 4-morpholino-sulphonylamino-phenyl |
| 93. | -CN | -CH₃ | 4-methylsulphonylamino-phenyl |
| 94. | -CN | -CH₃ | pyridin-4-yl |
| 95. | -CN | -CH₃ | quinolin-4-yl |
| 96. | -CN | -CH₃ | 2-methyl-pyridin-4-yl |
| 97. | -CN | -CH₃ | 3-methyl-pyridin-4-yl |
| 98. | -CN | -CH₃ | 1-tolylsulphonyl-pyrrol-3-yl |
| 99. | -CN | -CH₃ | 1-tolylsulphonyl-indol-3-yl |
| 100. | -CN | -CH₃ | 1-phenylsulphonyl-indol-3-yl |
| 101. | -CN | -CH₃ | 1-methylsulphonyl-indol-3-yl |
| 102. | -CN | -CH₃ | 1-dimethylaminosulphonyl-indol-3-yl |
| 103. | -CN | -CH₃ | 1-morpholinosulphonyl-indol-3-yl |

**Table A3:**

| Example No. | R5 | R6 | R7 |
|---|---|---|---|
| 104. | -CH₃ | -(CH₂CH₂)C(O)OCH₃ | 4-hydroxy-3,5-dimethylphenyl |
| 105. | -CH₃ | -(CH₂CH₂)C(O)OCH₃ | 4-methoxy-3,5-dimethylphenyl |
| 106. | -CH₃ | -(CH₂CH₂)C(O)OCH₃ | 4-carboxy-phenyl |
| 107. | -CH₃ | -(CH₂CH₂)C(O)OCH₃ | 2-methyl-4-hydroxy-phenyl |
| 108. | -CH₃ | -(CH₂CH₂)C(O)OCH₃ | 4-amino-phenyl |
| 109. | -CH₃ | -(CH₂CH₂)C(O)OCH₃ | 4-(2H-tetrazol-5-yl)-phenyl |
| 110. | -CH₃ | -(CH₂CH₂)C(O)OCH₃ | 4-morpholino-sulphonylamino-phenyl |
| 111. | -CH₃ | -(CH₂CH₂)C(O)OCH₃ | 4-methylsulphonylamino-phenyl |
| 112. | -CH₃ | -(CH₂CH₂)C(O)OCH₃ | pyridin-4-yl |
| 113. | -CH₃ | -(CH₂CH₂)C(O)OCH₃ | quinolin-4-yl |
| 114. | -CH₃ | -(CH₂CH₂)C(O)OCH₃ | 2-methyl-pyridin-4-yl |
| 115. | -CH₃ | -(CH₂CH₂)C(O)OCH₃ | 3-methyl-pyridin-4-yl |
| 116. | -CH₃ | -(CH₂CH₂)C(O)OCH₃ | 1-tolylsulphonyl-pyrrol-3-yl |
| 117. | -CH₃ | -(CH₂CH₂)C(O)OCH₃ | 1-tolylsulphonyl-indol-3-y1 |
| 118. | -CH₃ | -(CH₂CH₂)C(O)OCH₃ | 1-phenylsulphonyl-indol-3-yl |
| 119. | -CH₃ | -(CH₂CH₂)C(O)OCH₃ | 1-methylsulphonyl-indol-3-yl |
| 120. | -CH₃ | -(CH₂CH₂)C(O)OCH₃ | 1-dimethylaminosulphonyl-indol-3-yl |
| 121. | -CH₃ | -(CH₂CH₂)C(O)OCH₃ | 1-morpholinosulphonyl-indol-3-yl |

**Table A4:**

| Example No. | R5 | R6 | R7 |
|---|---|---|---|
| 122. | -CN | -CH₃ | 4-hydroxy-3,5-dimethylphenyl |
| 123. | -CN | -CH₃ | 4-methoxy-3,5-dimethylphenyl |
| 124. | -CN | -CH₃ | 4-carboxy-phenyl |
| 125. | -CN | -CH₃ | 2-methyl-4-hydroxy-phenyl |
| 126. | -CN | -CH₃ | 4-amino-phenyl |
| 127. | -CN | -CH₃ | 4-(2H-tetrazol-5-yl)-phenyl |
| 128. | -CN | -CH₃ | 4-morpholino-sulphonylamino-phenyl |
| 129. | -CN | -CH₃ | 4-methylsulphonylamino-phenyl |
| 130. | -CN | -CH₃ | pyridin-4-yl |
| 131. | -CN | -CH₃ | quinolin-4-yl |
| 132. | -CN | -CH₃ | 2-methyl-pyridin-4-yl |
| 133. | -CN | -CH₃ | 3-methyl-pyridin-4-yl |
| 134. | -CN | -CH₃ | 1-tolylsulphonyl-pyrrol-3-yl |
| 135. | -CN | -CH₃ | 1-tolylsulphonyl-indol-3-yl |
| 136. | -CN | -CH₃ | 1-phenylsulphonyl-indol-3-yl |
| 137. | -CN | -CH₃ | 1-methylsulphonyl-indol-3-yl |
| 138. | -CN | -CH₃ | 1-dimethylaminosulphonyl-indol-3-yl |
| 139. | -CN | -CH₃ | 1-morpholinosulphonyl-indol-3-yl |

as well as compound 30.

The following compounds of formula Id or Ie, and the salts, stereoisomers, hydrates or hydrates of the salts thereof, 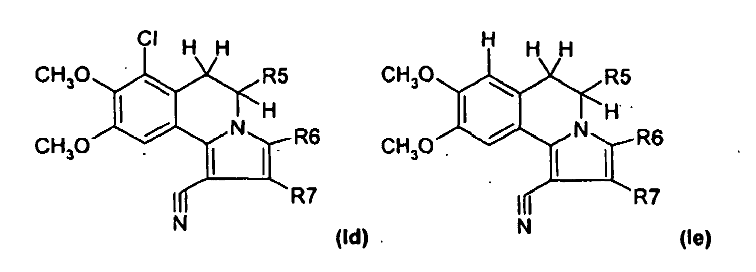 may also be mentioned as exemplary compounds according to this invention by means of the substituent meanings for R5, R6 and R7 in the following Tables B1, B2, B3 and B4:

**Table B1:**

| R5 | R6 | R7 |
|---|---|---|
| -CH₃ | -CH₃ | 4-hydroxy-3,5-dimethylphenyl |
| -CH₃ | -CH₃ | 4-methoxy-3,5-dimethylphenyl |
| -CH₃ | -CH₃ | 4-carboxy-phenyl |
| -CH₃ | -CH₃ | 2-methyl-4-hydroxy-phenyl |
| -CH₃ | -CH₃ | 4-amino-phenyl |
| -CH₃ | -CH₃ | 4-(2H-tetrazol-5-yl)-phenyl |
| -CH₃ | -CH₃ | 4-morpholino-sulphonylamino-phenyl |
| -CH₃ | -CH₃ | 4-methylsulphonylamino-phenyl |
| -CH₃ | -CH₃ | pyridin-4-yl |
| -CH₃ | -CH₃ | quinolin-4-yl |
| -CH₃ | -CH₃ | 2-methyl-pyridin-4-yl |
| -CH₃ | -CH₃ | 3-methyl-pyridin-4-yl |
| -CH₃ | -CH₃ | 1-tolylsulphonyl-pyrrol-3-yl |
| -CH₃ | -CH₃ | 1-phenylsulphonyl-indol-3-yl |
| -CH₃ | -CH₃ | 1-methylsulphonyl-indol-3-yl |
| -CH₃ | -CH₃ | 1-dimethylaminosulphonyl-indol-3-yl |
| -CH₃ | -CH₃ | 1-morpholinosulphonyl-indol-3-yl |

**Table B2:**

| R5 | R6 | R7 |
|---|---|---|
| -CN | -CH₃ | 4-hydroxy-3,5-dimethylphenyl |
| -CN | -CH₃ | 4-methoxy-3,5-dimethylphenyl |
| -CN | -CH₃ | 4-carboxy-phenyl |
| -CN | -CH₃ | 2-methyl-4-hydroxy-phenyl |
| -CN | -CH₃ | 4-amino-phenyl |
| -CN | -CH₃ | 4-(2H-tetrazol-5-yl)-phenyl |
| -CN | -CH₃ | 4-morpholino-sulphonylamino-phenyl |
| -CN | -CH₃ | 4-methylsulphonylamino-phenyl |
| -CN | -CH₃ | pyridin-4-yl |
| -CN | -CH₃ | quinolin-4-yl |
| -CN | -CH₃ | 2-methyl-pyridin-4-yl |
| -CN | -CH₃ | 3-methyl-pyridin-4-yl |
| -CN | -CH₃ | 1-tolylsulphonyl-pyrrol-3-yl |
| -CN | -CH₃ | 1-totylsulphonyl-indol-3-yl |
| -CN | -CH₃ | 1-phenylsulphonyl-indol-3-yl |
| -CN | -CH₃ | 1-methylsulphonyl-indol-3-yl |
| -CN | -CH₃ | 1-dimethylaminosulphonyl-indol-3-yl |
| -CN | -CH₃ | 1-morpholinosulphonyl-indol-3-yl |

**Table B3:**

| R5 | R6 | R7 |
|---|---|---|
| -CH₃ | -(CH₂CH₂)C(O)OCH₃ | 4-hydroxy-3,5-dimethylphenyl |
| -CH₃ | -(CH₂CH₂)C(O)OCH₃ | 4-methoxy-3,5-dimethylphenyl |
| -CH₃ | -(CH₂CH₂)C(O)OCH₃ | 4-carboxy-phenyl |
| -CH₃ | -(CH₂CH₂)C(O)OCH₃ | 2-methyl-4-hydroxy-phenyl |
| -CH₃ | -(CH₂CH₂)C(O)OCH₃ | 4-amino-phenyl |
| -CH₃ | -(CH₂CH₂)C(O)OCH₃ | 4-(2H-tetrazol-5-yl)-phenyl |
| -CH₃ | -(CH₂CH₂)C(O)OCH₃ | 4-morpholino-sulphonylamino-phenyl |
| -CH₃ | -(CH₂CH₂)C(O)OCH₃ | 4-methylsulphonylamino-phenyl |
| -CH₃ | -(CH₂CH₂)C(O)OCH₃ | pyridin-4-yl |
| -CH₃ | -(CH₂CH₂)C(O)OCH₃ | quinolin-4-yl |
| -CH₃ | -(CH₂CH₂)C(O)OCH₃ | 2-methyl-pyridin-4-yl |
| -CH₃ | -(CH₂CH₂)C(O)OCH₃ | 3-methyl-pyridin-4-yl |
| -CH₃ | -(CH₂CH₂)C(O)OCH₃ | 1-tolylsulphonyl-pyrrol-3-yl |
| -CH₃ | -(CH₂CH₂)C(O)OCH₃ | 1-tolylsulphonyl-indol-3-yl |
| -CH₃ | -(CH₂CH₂)C(O)OCH₃ | 1-phenylsulphonyl-indol-3-yl |
| -CH₃ | -(CH₂CH₂)C(O)OCH₃ | 1-methylsulphonyl-indol-3-yl |
| -CH₃ | -(CH₂CH₂)C(O)OCH₃ | 1-dimethylaminosulphonyl-indol-3-yl |
| -CH₃ | -(CH₂CH₂)C(O)OCH₃ | 1-morpholinosulphonyl-indol-3-yl |

**Table B4:**

| R5 | R6 | R7 |
|---|---|---|
| -CN | -CH₃ | 4-hydroxy-3,5-dimethylphenyl |
| -CN | -CH₃ | 4-methoxy-3,5-dimethylphenyl |
| -CN | -CH₃ | 4-carboxy-phenyl |
| -CN | -CH₃ | 2-methyl-4-hydroxy-phenyl |
| -CN | -CH₃ | 4-amino-phenyl |
| -CN | -CH₃ | 4-(2H-tetrazol-5-yl)-phenyl |
| -CN | -CH₃ | 4-morpholino-sulphonylamino-phenyl |
| -CN | -CH₃ | 4-methylsulphonylamino-phenyl |
| -CN | -CH₃ | pyridin-4-yl |
| -CN | -CH₃ | quinolin-4-yl |
| -CN | -CH₃ | 2-methyl-pyridin-4-yl |
| -CN | -CH₃ | 3-methyl-pyridin-4-yl |
| -CN | -CH₃ | 1-tolylsulphonyl-pyrrol-3-yl |
| -CN | -CH₃ | 1-tolylsulphonyl-indol-3-yl |
| -CN | -CH₃ | 1-phenylsulphonyl-indol-3-yl |
| -CN | -CH₃ | 1-methylsulphonyl-indol-3-yl |
| -CN | -CH₃ | 1-dimethylaminosulphonyl-indol-3-yl |
| -CN | -CH₃ | 1-morpholinosulphonyl-indol-3-yl |

The compounds according to the present invention can be prepared, for example, in an art-known manner, or in a manner described and shown as follows, or as disclosed in WO 02/48144, WO 03/014115, WO 03/014116, WO 03/014117 or WO 03/051877, or as described by way of example in the following examples, or analogously or similarly thereto.

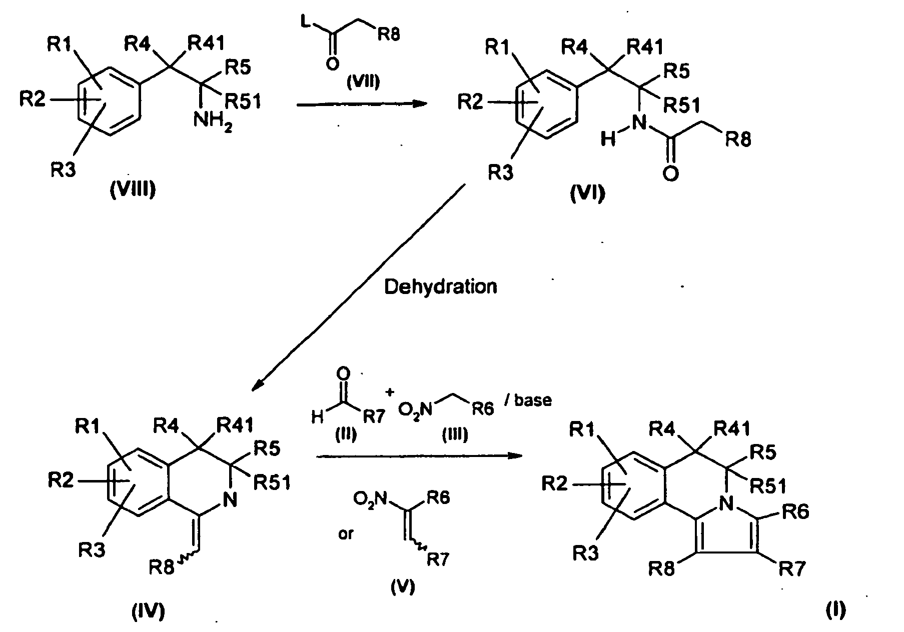

As shown in the scheme above, in a first reaction step compounds of formula VIII, in which R1, R2, R3, R4, R41, R5 and R51 have the meanings indicated above, are reacted with compounds of formula VII, in which R8 has the meanings indicated above and L is a suitable leaving group, for example chlorine or an acyloxy radical (e.g. the R8-CH₂-C(O)-O- radical), to give in the presence of a suitable organic or inorganic base corresponding compounds of formula VI.

Alternatively, compounds of formula VI are also accessible from compounds of formula VIII, in which R1, R2, R3, R4, R41, R5 and R51 have the meanings indicated above, and compounds of formula VII, in which R8 has the meanings indicated above and L is hydroxyl, by reaction with amide bond linking reagents known to the person skilled in the art. Exemplary amide bond linking reagents known to the person skilled in the art which may be mentioned are, for example, the carbodiimides (e.g. dicyclohexylcarbodiimide or, preferably, 1-ethyl-3-(3-dimethylaminopropyl)carbodiimide hydrochloride), azodicarboxylic acid derivatives (e.g. diethyl azodicarboxylate), uronium salts [e.g. O-(benzotriazol-1-yl)-N,N,N',N'-tetramethyluronium tetrafluoroborate or O-(benzotriazol-1yl)-N,N,N',N'-tetramthyl-uronium-hexafluorophosphatel and N,N'-carbonyldiimidazole. In the scope of this invention preferred amide bond linking reagents are uronium salts and, particularly, carbodiimides, preferably, 1-ethyl-3-(3-dimethylaminopropyl)carbodiimide hydrochloride.

Said reactions are carried out under conditions known to the person skilled in the art or as described exemplarily in the following examples.

As shown in the next step, compounds of the formula IV, in which R1, R2, R3, R4, R41, R5, R51 and R8 have the meanings indicated above, can be obtained by cyclocondensation of corresponding compounds of the formula VI. Said cyclocondensation reaction is carried out in a manner habitual per se to the person skilled in the art or as described by way of example in the following examples, according to Bischler-Napieralski (e.g. as described in J. Chem. Soc., 1956, 4280-4282) in the presence of a suitable condensing or dehydrating agent, such as, for example, polyphosphoric acid, phosphorus pentachloride, phosphorus pentoxide or phosphorus oxychloride, in a suitable inert solvent, e.g. in a chlorinated hydrocarbon such as chloroform, or in a cyclic hydrocarbon such as toluene or xylene, or another inert solvent such as acetonitrile, or without further solvent using an excess of condensing agent, at reduced temperature, or at room temperature, or at elevated temperature or at the boiling temperature of the solvent or condensing agent used.

Compounds of formula IV are converted either with compounds of formulae II, in which R7 has the meanings given above, and III, in which R6 is 1-6C-alkyl or 1-4C-alkyl substituted by 1-4C-alkoxycarbonyl, or with compounds of formula V, in which R7 has the meanings given above and R6 is 1-6C-alkyl or 1-4C-alkyl substituted by 1-4C-alkoxycarbonyl, optionally in a one pot synthesis and suitably in the presence of an inorganic or organic base (in particular a cyclic amine, e.g. piperidine) into the corresponding compounds of formula I.
Said conversion can be carried out as known to the skilled person or as described in the following examples or analogously or similarly thereto.

Compounds of formulae VIII, VII, III and II are commercially available or can be obtained in a manner known to the skilled person from his/her expert knowledge and/or from literature.

Compounds of formula V are known or are accessible by reaction of compounds of formula II with compounds of formula III in the presence of a suitable organic or inorganic base in a manner customary per se to the skilled person.

Compounds of formula I obtained can be converted into further compounds of formula I by methods known to one of ordinary skill in the art. More specifically, for example, from compounds of the formula I, in which
a.) R8, R61, R71, R74 or R76 are an ester group, the corresponding acids can be obtained by acidic or, particularly, alkaline hydrolysis;
b.) R8 is an ester group, the corresponding reduced forms thereof (e.g. the hydroxymethyl or methyl radicals) can be obtained by selective reduction reactions;
c.) R8 is a hydroxymethyl group obtainable according b.), the corresponding ester or ether derivatives -CH₂-O-R81 can be obtained by esterification or etherification reactions;
d.) R8 is an ester or carboxyl group, the corresponding amides can be obtained by amidification reactions;
e.) R6 is 1-4C-alkyl, particularly methyl, the corresponding halogenated, preferably chlorinated, groups can be obtained by halogenation reaction, particularly by reaction with a chlorination reagent such as sulfuryl chloride, thionyl chloride or N-chlorosuccinimide;
f.) R6 is 1-4C-alkyl substituted by halogen obtainable according e.), the corresponding derivatized 1-4C-alkyl radicals substituted by 1-4C-alkoxy, hydroxyl, halogen or-N(R611)R612 can be obtained by nucleophilic substitution reactions with suitable nucleophiles;
g.) R6 is 1-4C-alkyl substituted by hydroxyl obtainable according f.), the corresponding derivatized 1-4C-alkyl radicals substituted by 1-4C-alkoxycarbonyl can be obtained by oxidation and esterification reactions under suitable conditions;
h.) R6 is methyl, the corresponding oxidized forms thereof (e.g. the hydroxymethyl or formyl radicals) can be obtained stepwise or directly by selective oxidation reactions (e.g. with the aid of manganese dioxide to obtain the formyl radicals);
i.) R6 is formyl obtainable according h.), the corresponding aminated compounds can be obtained by reductive amination reaction;
j.) R6 is hydroxymethyl obtainable according h.), the corresponding fluorine compounds can be obtained by fluorination reaction;
k.) R6 is methyl, the corresponding amino compounds can be obtained by nitration reaction and subsequential reduction of the nitro compounds obtained.

The methods mentioned under a.) to k.) are expediently carried out analogously to the methods known to the person skilled in the art or as described by way of example in the following examples.

It is moreover known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The isolation and purification of the substances according to the invention is carried out in a manner known per se, e.g. by distilling off the solvent in vacuo and recrystallizing the resulting residue from a suitable solvent or subjecting it to one of the customary purification methods, such as, for example, column chromatography on suitable support material.

Salts are obtained by dissolving the free compound in a suitable solvent (e.g. a ketone, such as acetone, methyl ethyl ketone or methyl isobutyl ketone, an ether, such as diethyl ether, tetrahydrofuran or dioxane, a chlorinated hydrocarbon, such as methylene chloride or chloroform, or a low molecular weight aliphatic alcohol such as ethanol or isopropanol) which contains the desired acid or base, or to which the desired acid or base is then added. The salts are obtained by filtering, reprecipitating, precipitating with a nonsolvent for the addition salt or by evaporating the solvent. Salts obtained can be converted by alkalization or by acidification into the free compounds, which in turn can be converted into salts. In this way, pharmacologically intolerable salts can be converted into pharmacologically tolerable salts.

The person skilled in the art knows on the basis of his/her knowledge and on the basis of those synthesis routes, which are shown and described within the description of this invention, how to find other possible synthesis routes for compounds of the formula I. All these other possible synthesis routes are also part of this invention.

Having described the invention in detail, the scope of the present invention is not limited only to those described characteristics or embodiments. As will be apparent to persons skilled in the art, modifications, variations and adaptations to the described invention can be made on the base of the disclosure (e.g. the explicite, implicite or inherent disclosure) of the present invention without departing from the spirit and scope of this invention.

The following examples serve to illustrate the invention in greater detail without restricting it. Likewise, further compounds of the formula I, whose preparation is not explicitly described, can also be prepared in an analogous manner or in a manner familiar per se to the person skilled in the art using customary process techniques.

In the examples, m.p. stands for melting point, h for hour(s), min for minutes, conc. for concentrated, satd. for saturated, MS for mass spectrum, M for molecular ion.

Unless otherwise noted, if the exemplary compounds mentioned expressis verbis herein contain a chirality center, they are desribed illustratively as racemic mixtures herein, without restricting this invention thereto.

The compounds mentioned in the examples as well as their salts and stereoisomers are a preferred subject of the invention.

### Examples

### Final products

1. 2-(4-Hydrox-3,5-dimethyl-phenyl)-8,9-dimethoxy-3,5,5-trimethyl-5,6-dihydro-pyrrolo[2,1-a]isoquinoline-1-carboxylic acid ethyl ester
   Analogously to a procedure described by Meyer in Liebigs Ann. Chem. 1981, 9, 1534-1544, (6,7-dimethoxy-3,3-dimethyl-3,4-dihydro-2H-isoquinolin-1-ylidene)-acetic acid ethyl ester (compound A7) is reacted with nitro ethane and 4-hydroxy-3,5-dimethyl benzaldehyde to afford the title compound.
   MS (M+H) = 464.1; m.p. = 210 - 213 °C

The following examples (Examples 2-20) can be prepared in analogy to example 1 using the appropriate starting compound selected from the group consisting of the compounds A1 to A9. All aldehydes used are commercially available or can be prepared in analogy to published procedures. If nitro propane or 4-nitro butyric acid methyl ester is used instead of nitroethane, 3-ethyl-5,6-dihydropyrrolo[2,1-a]isoquinolines and 3-methoxycarbonylethyl-5,6-dihydro-pyrrolo[2,1-a]isoquinolines (e.g. 3-(8,9-dimethoxy-5,6-dihydro-pyrrol[2,1-a]isoquinolin-3-yl)propionic methyl esters), respectively are obtained.
2. 8,9-Dimethoxy-3,5,5-trimethyl-2-(3,4,5-trimethoxy-phenyl)-5,6-dihydro-pyrrolo[2,1-a]isoquinoline-1-carboxylic acid ethyl ester
   MS (M+H) = 510.4; m.p. = 52 - 56 °C
3. 2-[3-(4-Chloro-phenoxy)-phenyl]-8,9-dimethoxy-3,5,5-trimethyl-5,6-dihydro-pyrrolo[2,1-a]isoquinoline-1-carboxylic acid ethyl ester
   MS (M+H) = 546.2; m.p. = 61 - 64 °C
4. 2-(3-Dimethylamino-phenyl)-8,9-dimethoxy-3,5,5-trimethyl-5,6-dihydro-pyrrolo[2,1-a]isoquinoline-1-carboxylic acid ethyl ester
   MS (M+H) = 463.1; m.p. = 101 - 102 °C
5. (5RS)- (4-Hydroxy-3,5-dimethyl-phenyl)-8,9-dimethoxy-3,5-dimethyl-5,6-dihydro-pyrrolo[2,1-a]isoquinoline-1-carboxylic acid ethyl ester
   MS (M+H) = 450.2; m.p. = 158 -161 °C
6. (5RS)-5-Ethyl-2-(4-hydroxy-3,5-dimethyl-phenyl)-8,9-dimethoxy-3-methyl-5,6-dihydropyrrolo[2,1-a]isoquinoline-1-carboxylic acid ethyl ester
   MS (M+H) = 464.1; m.p. = 164 - 166 °C
7. (5RS)-2-Chlorophenyl-5-ethyl-8,9-dimethoxy-3-methyl-5,6-dihydro-pyrrolo[2,1-a]isoquinoline-1-carboxylic acid ethyl ester
   MS (M+H) = 454.2; m.p. = 121 -124 °C
8. (4aRS,8aRS)-cis-2-(4-hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
   MS (M+H) = 490.2; m.p. = 186 -192 °C
9. (5RS)-3-Ethyl-2-(4-hydroxy-3,5-dimethyl-phenyl)-8,9-dimethoxy-5-methyl-5,6-dihydropyrrolo[2,1-a]isoquinoline-1-carboxylic acid ethyl ester
   MS (M+H) = 464.1; m.p. = 188 -190 °C
10. (5RS)-8,9-Dimethoxy-3,5-dimethyl-2-(3,4,5-trimethoxy-phenyl)-5,6-dihydro-pyrrolo[2,1-a]isoquinoline-1-carboxylic acid ethyl ester
   MS (M+H) = 496.0; m.p. = 116 -118 °C
11. (5RS)-8,9-Dimethoxy-3,5-dimethyl-2-naphthalen-1-yl-5,6-dihydro-pyrrolo[2,1-a]isoquinoline-1-carboxylic acid ethyl ester
   MS (M+H) = 456.1; m.p. = 184 °C
12. (4aRS,8aRS)-cis-10,11-Dimethoxy-3-methyl-2-naphthalen-1-yl-4a,5,6,7,8,8a-hexahydropyrrolo[2,1-f]phenanthridine-1-carboxylic acid ethyl ester
   MS (M+H) = 496.1; m.p. = 189 -191 °C
13. (4aRS,8aRS)-cis-10,11-Dimethoxy-3-methyl-2-quinolin-4-yl-4a,5,6,7,8,8a-hexahydropyrrolo[2,1-f]phenanthridine-1-carboxylic acid ethyl ester
   MS (M+H) = 497.3; m.p. = 153 - 157 °C
14. (4aR,8aR)-10,11-Dimethoxy-3-methyl-2-quinolin-4-yl-4a,5,6,7,8,8a-hexahydro-pyrrolo[2,1-f]phenanthridine-1-carboxylic acid ethyl ester
   MS (M+H) = 497.3; oil
15. (4aR,8aR)-10,11-Dimethoxy-3-methyl-2-naphthalen-1-yl-4a,5,6,7,8,8a-hexahydro-pyrrolo[2,1-f]phenanthridine-1-carboxylic acid ethyl ester
   MS (M+H) = 496.1; m.p. = 212 - 216 °C
16. (4aR,8aR)-2-(4-Hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
   MS (M+H) = 490.2; m.p. = 203 - 206 °C
17. (5RS)-5-Ethyl-8,9-dimethoxy-3-methyl-2-naphthalen-1-yl-5,6-dihydro-pyrrolo[2,1-a]isoquinoline-1-carboxylic acid ethyl ester
   MS (M+H) = 470.1; oil
18. (5RS)-2-(4-Hydroxy-3,5-dimethyl-phenyl)-7,8,9-trimethoxy-3,5-dimethyl-5,6-dihydro-pyrrolo[2,1-a]isoquinoline-1-carboxylic acid ethyl ester
   MS (M+H) = 480.0; m.p. = 144 °C
19. 2-(4-Hydroxy-3,5-dimethyl-phenyl)-8,9-dimethoxy-5,6-dihydro-pyrrolo[2,1-a]isoquinoline-1,5-dicarboxylic acid 1-ethyl 5-methyl ester
   MS (M+H) = 494.1; m.p. = 92 - 97 °C
20. (5RS)-8,9-Dimethoxy-3-(2-methoxycarbonyl-ethyl)-5-methyl-2-naphthalen-1-yl-5,6-dihydropyrrolo[2,1-a]isoquinoline-1-carboxylic acid ethyl ester
   MS (M+H) = 528.1; m.p. = 56 - 59 °C
21. 2-(4-Hydroxy-3,5-dimethyl-phenyl)-8,9-dimethoxy-3-methyl-5,6-dihydro-pyrrolo[2,1-a]isoquinoline-1-carbornitrile
   Analogously to the procedure described for Example 1, (6,7-dimethoxy-3A-dihydro-2H-isoquinolin-1-ylidene)-acetonitrile (compound A8) is reacted with nitro ethane and 4-hydroxy-3,5-dimethyl benzaldehyde to afford 2-(4-hydroxy-3,5-dimethyl-phenyl)-8,9-dimethoxy-3-methyl-5,6-dihydropyrrolo[2,1-a]isoquinoline-1-carbonitrile as a colorless solid of m.p. 285 - 287 °C. The masts spectrum shows the molecular peak M+H at 388.5 Da.

The following examples (Nos. 22-28) can be prepared in analogy to example 21 using the appropriate starting compound A8 or A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydropyrrolo[2,1-a]isoquinolines, respectively are obtained.
22. 8,9-Dimethoxy-3-methyl-2-naphthalen-1-yl-5,6-dihydro-pyrrolo[2,1-a]isoquinoline-1-carbonitrile
   MS (M+H) = 395.2; m.p. = 226 - 229 °C
23. 8,9-Dimethoxy-3-methyl-2-quinolin-4-yl-5,6-dihydro-pyrrolo[2,1-a]isoquinoline-1-carbonitrile
   MS (M+H) = 396.3; m.p. = 239 -243 °C
24. 2-(1H-Indol-3-yl)-8,9-dimethoxy-3-methyl-5,6-dihydro-pyrrolo[2,1-a]isoquinoline-1-carbonitrile
   MS (M+H) = 384.3; m.p. = 304 - 307 °C
25. 2-(3,5-Di-tert-butyl-4-hydroxy-phenyl)-8,9-dimethoxy-3-methyl-5,6-dihydro-pyrrolo[2,1-a]isoquinoline-1-carbonitrile
   MS (M+H) = 473.1; m.p. = 250 - 252 °C
26. 8,9-Dimethoxy-3,5-dimethyl-2-pyridin-4-yl-5,6-dihydro-pyrrolo[2,1-a]isoquinoline-1-carbonitrile
   MS (M+H) = 360.3; m.p. = 253 - 254 °C
27. 3-[1-Cyano-2-(4-hydroxy-3,5-dimethyl)-8,9-dimethoxy-5-methyl-5,6-dihydro-pyrrolo[2,1-a]isoquinolin3-yl]-propionic acid methyl ester
   MS (M+H) = 475.2; m.p. = 208 - 209 °C
28. 2-(4-Hydroxy-3,5-dimethyl-phenyl)-8,9-dimethoxy-3,5-dimethyl-5,6-dihydro-pyrrolo[2,1-a]isoquinoline-1-carbonitrile
   MS (M+H) = 403.2; m.p. = 268 - 270 °C

The following examples (Nos. 29-59) can be prepared in analogy to example 21 using the appropriate starting compound, which can be prepared in an art-known manner, or analogously or similarly as described for A8 or A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinolines, respectively are obtained.
29. 3-(1-Cyano-8,9-dimethoxy-2-pyridin-4-yl-5,6-dihydro-pyrrolo[2,1-a]isoquinolin-3-yl)-propionic acid methyl ester
   MS (M+H) = 417.9; m.p. = 191 - 193 °C
30. 7-Fluoro-2-(4-hydroxy-3,5-dimethyl-phenyl)-8,9-dimethoxy-3,5-dimethyl-5,6-dihydro-pyrrolo[2,1-a]isoquinoline-1-carbonitrile
   MS (M+H) = 421.2; m.p. = 166 - 168 °C
31. 3-(1-Cyano-8,9-dimethoxy-2-quinolin-4-yl-5,6-dihydro-pyrrolo[2,1-a]isoquinolin-3-yl)-propionic acid methyl ester
   MS (M+H) = 467.9; m.p. = 232 - 234 °C
32. 3-[1-Cyano-2-(4-hydroxy-3,5-dimethyl-phenyl)-8,9-dimethoxy-5,6-dihydro-pyrrolo[2,1-a]isoquinolin-3-yl]-propionic acid methyl ester
   MS (M+H) = 461.0; m.p. = 217 - 219 °C
33. 8,9-Dimethoxy-2-(4-methoxy-3,5-dimethyl-phenyl)-3,5-dimethyl-5,6-dihydro-pyrrolo[2,1-a]isoquinoline-1-carbonitrile
   MS (M+H) = 417.3;
34. 2-(1H-Indol-5-yl)-8,9-dimethoxy-3-methyl-5,6-dihydro-pyrrolo[2,1-a]isoquinoline-1-carbonitrile
   MS (M+H) = 384.3;
35. 8,9-Dimethoxy-2-(4-methoxy-3,5-dimethyl-phenyl)-3-methyl-5,6-dihydro-pyrrolo[2,1-a]isoquinoline-1-carbonitrile
   MS (M+H) = 403.3;
36. 2-(1-Benzyl-2,3-dihydro-1H-indol-5-yl)-8,9-dimethoxy-3-methyl-5,6-dihydro-pyrrolo[2,1-a]isoquinoline-1-carbonitrile
   MS (M+H) = 476.1;
37. 8,9-Dimethoxy-3,5-dimethyl-2-[1-(toluene-4-sulfonyl)-1H-pyrrol-3-yl]-5,6-dihydro-pyrrolo[2,1-a]isoquinoline-1-carbonitrile
   MS (M+H) = 502.1;
38. 8,9-Dimethoxy-3,5-dimethyl-2-[1-(toluene-4-sulfonyl)-1H-indol-3-yl]-5,6-dihydro-pyrrolo[2,1-a]isoquinoline-1-carbonitrile
   MS (M+H) = 569.0;
39. 2-(1-Benzenesulfonyl-1H-indol-3-yl)-8,9-dimethoxy-3,5-dimethyl-5,6-dihydro-pyrrolo[2,1-a]isoquinoline-1-carbonitrile
   MS (M+H) = 537.7;
40. 2-(1-Methanesulfonyl-1H-indol-3-yl)-8,9-dimethoxy-3,5-dimethyl-5,6-dihydro-pyrrolo[2,1-a]isoquinoline-1-carbonitrile
   MS (M+H) = 475.8; m.p. = 219 - 221 °C
41. 8,9-Dimethoxy-3,5-dimethyl-2-(1-oxy-pyridin-4-yl)-5,6-dihydro-pyrrolo[2,1-a]isoquinoline-1-carbonitrile
   MS (M+H) = 375.8; m.p. = 279 - 282 °C
42. 7-Fluoro-8,9-dimethoxy-3,5-dimethyl-2-[1-(toluene-4-sulfonyl)-1H-indol-3-yl]-5,6-dihydropyrrolo[2,1-a]isoquinoline-1-carbonitrile
   MS (M+H) = 587.0;
43. 2-(2,3-Dihydro-1H-indol-5-yl)-8,9-dimethoxy-3-methyl-5,6-dihydro-pyrrolo[2,1-a]isoquinoline-1-carbonitrile
   MS (M+H) = 386.3;
44. 2-(4-Hydroxy-3,5-dimethyl-phenyl)-8,9-dimethoxy-5-methyl-3-morpholin-4-ylmethyl-5,6-dihydropyrrolo[2,1-a]isoquinoline-1-carbonitrile
   m.p. = 228 - 230 °C
45. 8,9-Dimethoxy-3,5-dimethyl-2-(2-methyl-pyridin-4-yl)-5,6-dihydro-pyrrolo[2,1-a]isoquinoline-1-carbonitrile
   MS (M+H) = 374.2; m.p. = 187 - 189 °C
46. 8,9-Dimethoxy-3,5-dimethyl-2-(4-nitro-phenyl)-5,6-dihydro-pyrrolo[2,1-a]isoquinoline-1-carbonitrile
   MS (M+H) = 403.7; m.p. = 206 - 207 °C
47. 4-(1-Cyano-8,9-dimethoxy-3,5-dimethyl-5,6-dihydro-pyrrolo[2,1-a]isoquinolin-2-yl)-benzoic acid
   MS (M+H) = 402.7; m.p. = 287 - 289 °C
48. 2-(4-Amino-phenyl)-8,9-dimethoxy-3,5-dimethyl-5,6-dihydro-pyrrolo[2,1-a]isoquinoline-1-carbonitrile
   MS (M+H) = 374.1; m.p. = 237 - 239 °C
49. 8,9-Dimethoxy-3,5-dimethyl-2-(3-methyl-pyridin-4-yl)-5,6-dihydro-pyrrolo[2,1-a]isoquinoline-1-carbonitrile
   MS (M+H) = 374.5; m.p. = 232 - 233 °C
50. 4-(1-Cyano-8-ethoxy-9-methoxy-3,5-dimethyl-5,6-dihydro-pyrrolo[2,1-a]isoquinolin-2-yl)-benzoic acid
   MS (M+H) = 417.2; m.p. = 274 - 277 °C
51. 2-(4-Hydroxy-2-methyl-phenyl)-8,9-dimethoxy-3,5-dimethyl-5,6-dihydro-pyrrolo[2,1-a]isoquinoline-1-carbonitrile
   MS (M+H) = 389.1; m.p. = 228 - 230 °C
52. 4-(1-Cyano-8,9-dimethoxy-3,5-dimethyl-5,6-dihydro-pyrrolo[2,1-a]isoquinolin-2-yl)-benzamide m.p. = 228 - 230 °C
53. 8-Ethoxy-2-(4-hydroxy-3,5-dimethyl-phenyl)-9-methoxy-3,5-dimethyl-5,6-dihydro-pyrrolo[2,1-a]isoquinoline-1-carbonitrile
   MS (M+H) = 417.2; m.p. = 232 - 234 °C
54. 3-(1-Cyano-8,9-dimethoxy-3,5-dimethyl-5,6-dihydro-pyrrolo[2,1-a]isoquinolin-2-yl)-indole-1-sulfonic acid dimethylamide
   MS (M+H) = 505.2; m.p. = 236 - 237 °C
55. 8,9-Dimethoxy-3,5-dimethyl-2-(2-methyl-1-oxy-pyridin-4-yl)-5,6-dihydro-pyrrolo[2,1-a]isoquinoline-1-carbonitrile
   MS (M+H) = 390.1; m.p. = 265 - 268 °C
56. 8,9-Dimethoxy-3,5-dimethyl-2-[1-(morpholine-4-sulfonyl)-1H-indol-3-yl]-5,6-dihydro-pyrrolo[2,1-a]isoquinoline-1-carbonitrile
   MS (M+H) = 574.1; m.p. = 210 - 212 °C
57. 8,9-Dimethoxy-3,5-dimethyl-2-[4-(2H-tetrazol-5-yl)-phenyl]-5,6-dihydro-pyrrolo[2,1-a]isoquinoline-1-carbonitrile
   MS (M+H) = 427.2; m. p. = 204 - 207 °C
58. Morpholine-4-sulfonic acid [4-(1-cyano-8,9-dimethoxy-3,5-dimethyl-5,6-dihydro-pyrrolo[2,1-a]isoquinolin-2-yl)-phenyl]-amide
   MS (M+H) = 523.1; m. p. = 223 - 225 °C
59. N-[4-(1-Cyano-8,9-dimethoxy-3,5-dimethy)-5,6-dihydro-pyrrolo[2,1-a]isoquinolin-2-yl)-phenyl]-methanesulfonamide
   MS (M+H) = 452.1; m.p. = 257 - 259 °C

The following examples (Examples 60-67) can be prepared in analogy to example 1 using the appropriate starting compound, which can be prepared in an art-known manner, or analogously or similarly as described for compounds A1 to A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinolines, respectively are obtained.
60. 5-Ethyl-2-(2-fluoro-3,4-dimethoxy-phenyl)-8,9-dimethoxy-3-methyl-5,6-dihydro-pyrrolo[2,1-a]isoquinoline-1-carboxylic acid ethyl ester
   MS (M+H) = 498.1;
61. 7-Chloro-8,9-dimethoxy-3,5-dimethyl-2-pyridin-4-yl-5,6-dihydro-pyrrolo[2,1-a]isoquinoline-1-carboxylic acid ethyl ester
   MS (M+H) = 441.3;
62. 7-Chloro-2-(4-hydroxy-3,5-dimethyl-phenyl)-8,9-dimethoxy-3,5-dimethyl-5,6-dihydro-pyrrolo[2,1-a]isoquinoline-1-carboxylic acid ethyl ester
   MS (M+H) = 484.0;
63. 7,8,9-Trimethoxy-3,5-dimethyl-2-pyridin-4-yl-5,6-dihydro-pyrrolo[2,1-a]isoquinoline-1-carboxylic acid ethyl ester
   MS (M+H) = 437.3;
64. 8,9-Dimethoxy-3-(2-methoxycarbonyl-ethyl)-5-methyl-2-quinolin-4-y-5,6-dihydro-pyrrolo[2,1-a]isoquinoline-1-carboxylic acid ethyl ester
   MS (M+H) = 529.3;
65. 2-(4-Hydroxy-3,5-dimethyl-phenyl)-8,9-dimethoxy-3,5-dimethyl-5,6-dihydro-pyrrolo[2,1-a]isoquinoline-1-carboxylic acid methyl ester
   MS (M+H) = 435.9; m.p. = 177 - 179 °C
66. 8,9-Dimethoxy-3,5-dimethyl-2-[1-(toluene-4-sulfonyl)-1H-indol-3-yl]-5,6-dihydro-pyrrolo[2,1-a]isoquinoline-1-carboxylic acid methyl ester
   MS (M+H) = 584.9; m.p. = 177 - 179 °C
67. 5-Cyano-2-(4-hydroxy-3,5-dimethyl-phenyl)-8,9-dimethoxy-3-methyl-5,6-dihydro-pyrrolo[2,1-a]isoquinoline-1-carboxylic acid ethyl ester
   MS (M+H) = 461.0;
68. 4-(8,9-Dimethoxy-1,3-dimethyl-5,6-dihydro-pyrrolo[2,1-a]isoquinolin-2-yl)-2,6-dimethyl-phenol MS (M+H) = 377.9; m.p. = 183 - 185 °C
   The title compound can be obtained via an analogous synthesis route as described for the Examples herein.
69. 8,9-Dimethoxy-3-(2-methoxycarbonyl-ethyl)-5-methyl-2-quinolin-4-yl-5,6-dihydro-pyrrolo[2,1-a]isoquinoline-1-carboxylic acid ethyl ester
   MS (M+H) = 407.9; m.p. = 176 -177 °C
   The title compound can be obtained from the corresponding ester compound, which is accessible analogously as described in Example 1 herein, by art-known saponification reaction.

Examplary compounds of formula lc mentioned as Examples 70-139 in Tables A1 to A4 can be prepared analogously or similarly to the described examples.

Exemplary compounds of formulae Id or Ie can be also prepared analogously or similarly to the described examples.

### Starting compounds

A1 (3RS)-(6,7-Dimethoxy-3-methyl-3,4-dihydro-2H-isoquinolin-1-ylidene)-acetic acid ethyl ester The title compound can be obtained by a Bischler-Napieralski reaction (Ber. 1893, 26, 1903) using N-(2-[4-methoxy-3-(2-methoxy-ethoxy)-phenyl]-ethyl)-malonamic acid ethyl ester (compound B1) as the starting material.

The following 3,4-Dihydro-1(2H)-isoquinolinylidene-derivatives A2 to A9 and also further relevant, non-explicitly described similar compounds can be prepared according to an analogous procedure using the appropriate starting compound B2 to B8, or an appropriate analogous compound:
A2 (3RS)-(3-Ethyl-6,7-dimethoxy-3,4-dihydro-2H-isoquinolin-1-ylidene)-acetic acid ethyl ester
A3 ((4aR,10bR)-8,9-Dimethoxy-1,3,4,4a,5,10b-hexyhydro-2H-phenanthridin-6-ylidene)-acetic acid ethyl ester
A4 ((4aRS,10bRS)-cis-8,9-Dimethoxy-1,3,4,4a,5,10b-hexyhydro-2H-phenanthridin-6-ylidene)-acetic acid ethyl ester
A5 1-Ethoxycarbonylmethylene-6,7-dimethoxy-1,2,3,4-tetrahydro-isoquinolie-3-carboxylic acid methyl ester
A6 (3RS)-(5,6,7-Trimethoxy-3-methyl-3,4-dihydro-2H-isoquinolin-1-ylidene)-acetic acid ethyl ester
A7 (6,7-Dimethoxy-3,3-dimethyl-3,4-dihydro-2H-isoquinolin-1-ylidene)-acetic acid ethyl ester
   The compound A19 is commercially available.
A8 (6,7-Dimethoxy-3,4-dihydro-2H-isoquinolin-1-ylidene)-acetonitrile
   The compound A8 can be prepared analogously to the above-described synthesis of compound A1 using the starting compound B7.
A9 (6,7-Dimethoxy-3-methyl-3,4-dihydro-2H-isoquinolin-1-ylidene)-acetonitrile
   The compound A9 can be prepared analogously to the above-described synthesis of compound A1 using the starting compound B8.

B1 N-[(RS)-2-(3,4-Dimethoxy-phenyl)-1-methyl-ethyl]-malonamic acid ethyl ester
   The title compound can be prepared by a reaction of (RS)-2-(3,4-Dimethoxy-phenyl)-1-methylethylamine (compound C1) with ethyl maloyl chloride in analogy to procedures in the literature (e.g. Benovsky et al., Tetrahedron lett. 1997, 38, 8475-8478).

The following amides B2 to B8 can be synthesized according an analogous procedure:
B2 N-[(RS)-1-(3,4-Dimethoxy-benzyl)-propyl]-malonamic acid ethyl ester
B3 N-[(1R,2R)-2-(3,4-Dimethoxy-phenyl)-cyclohexyl]-malonamic acid ethyl ester
B4 N-[(1RS,2RS)-cis-2-(3,4-Dimethoxy-phenyl)-cyclohexyl]-malonamic acid ethyl ester
B5 3-(3,4-Dimethoxy-phenyl)-2-(2-ethoxycarbonyl-ethanoylamino)-propionic acid methyl ester
B6 N-[(RS)-1-Methyl-2-(2,3,4-trimethoxy-phenyl)-ethyl]malonamic acid ethyl ester
B7 2-Cyano-N-[2-(3,4-dimethoxy-phenyl)-ethyl]-acetamide
   A solution of 10.0 g (55.1 mmol) of 2-(3,4-dimethoxy-phenyl)-ethylamine and 9.36 g (82.7 mmol) of ethyl cyano acetate is stirred at 100 °C for 15 h. The mixture is cooled to room temperature. The precipitate is filtered off and recrystallized from ethanol. 9.44 g (38.0 mmol, 60 %) of 2-cyano-N-[2-(3,4-dimethoxy-phenyl)-ethyl]-acetamide are obtained as a beige solid.
   MS (M+H) = 249.0, m.p. = 113-115 °C.
B8 2-Cyano-N-[2-(3,4-dimethoxy-phenyl)-1-methyl-ethyl]-acetamide
   Compound B8 can be prepared analogously to the synthesis of compound B7.

The appropriate starting compounds for the preparation of the compounds B1 to B8 are commercially available, or can be prepared as described below in the synthesis of the compound C1 or analogously or similarly thereto, or can be obtained in analogy to published procedures, e.g. the substituted 2-phenethyl amines can be prepared starting from the corresponding benzaldehydes (see also Shepard et al., J. Org. Chem. 1952, 17, 568).
C1 (RS)-2-(3,4-Dimethoxy-phenyl)-1-methyl-ethylamine
   The title compound can be prepared by a sequence described by Shepard et al. in J. Org. Chem. 1952, 17, 568.

### Commercial utility

### Commercial applicability

Intracellular levels of the second messengers cAMP and cGMP are regulated by both their rates of synthesis by cyclases and their hydrolysis by phosphodiesterases. Of the 11 phosphodiesterase (PDE) isoenzymes which are presently known, PDE10 was described for the first time in 1999 (Soderling SH, Bayuga SJ, Beavo JA. Isolation and characterization of a dual-substrate phosphodiesterase gene family: PDE10A. Proc Natl Acad Sci U S A. 1999 Jun 8;96(12):7071-6; Fujishige K, Kotera J, Michibata H, Yuasa K, Takebayashi S, Okumura K, Omori K. Cloning and characterization of a novel human phosphodiesterase that hydrolyzes both cAMP and cGMP (PDE10A). J Biol Chem. 1999 Jun 25;274(26):18438-45; Loughney K, Snyder PB, Uher L, Rosman GJ, Ferguson K, Florio VA. Isolation and characterization of PDE10A, a novel human 3', 5'-cyclic nucleotide phosphodiesterase. Gene. 1999 Jun 24;234(1):109-17). The first gene of this new PDE subfamily was designated PDE10A and the first splice variant was described as PDE10A1, according to the current nomenclature. Due to alternative splicing, other splice variants of PDE10A exist and have been described in the subsequent years (Kotera J, Fujishige K, Yuasa K, Omori K. Characterization and phosphorylation of PDE10A2, a novel alternative splice variant of human phosphodiesterase that hydrolyzes cAMP and cGMP. Biochem Biophys Res Commun. 1999 Aug 11;261(3):551-7; Fujishige K, Kotera J, Omori K. Striatum- and testis-specific phosphodiesterase PDE10A isolation and characterization of a rat PDE10A. Eur J Biochem. 1999 Dec;266(3):1118-27; Fujishige K, Kotera J, Yuasa K, Omori K. The human phosphodiesterase PDE10A gene genomic organization and evolutionary relatedness with other PDEs containing GAF domains. Eur J Biochem. 2000 Oct;267(19):5943-51). PDE10A has been described as a cyclic nucleotide phosphodiesterase exhibiting properties of a cAMP PDE and a cAMP-inhibited cGMP PDE.

Individual representatives of the PDE10 isoenzyme are characterized by being particularly prominently expressed in specific areas of the brain (striatum, putamen, caudate nucleus, cerebellum, thalamus), in testis, in the thyroid gland, in the pituitary gland, in kidney and in placenta.

Increased expression levels in a broad variety of tumor cell lines and tissues, namely of the lung, breast, pancreas, brain, prostate and ovar indicates that PDE10 may play an important role in tumor cell growth and/or survival under conditions of elevated cAMP and/or cGMP generation.

Increased expression levels and activities of PDE10A have been also found in testis suggesting that PDE10A may contribute to spermatogenesis (Fujishige K et al., Eur J Biochem. 1999, 266:1118-27). Certain PDE inhibitors, namely e.g. PDE3 or PDE11A inhibitors, are known to augment glucose-induced insulin secretion and thus may be useful for treating diabetes (see e.g. WO 03/077949).

The compounds according to the invention have miscellaneous valuable pharmacological properties which make them commercially utilizable.

Thus, for example, the compounds according to this invention are PDE inhibitors.

Yet thus, for example, the compounds according to the invention are potent PDE10 inhibitors, some of which are apparently selective (by >100 fold) among other PDE isoenzymes, wherein these selective compounds are particularly preferred in the context of the present invention. The compounds according to the invention therefore can be employed as therapeutic agents for the treatment or prophylaxis of diseases in human and veterinary medicine.

Due to their potent and selective PDE10 inhibitory activity, the compounds according to the present invention may be, of potential value in treating disorders of the central nervous system, in particular neurologic and psychiatric disorders, for example those mentioned in EP 1250923 and/or, in more particular, psychotic disorders, anxiety disorders, mood disorders or episodes, drug addiction, movement disorders or disorders comprising deficient cognition as a symptom (e.g. dementia, Parkinson's disease or Alzheimer's disease).

Furthermore, the compounds according to the present invention may be, , of potential value in treating certain disorders of the central nervous system, in particular neurologic and psychiatric disorders, for example those mentioned generically, specifically or exemplarily in EP 1250923, US 2003/0008806 and/or US 2003/0018047, such as, for example, anxiety or psychotic disorders, movement disorders, obsessive/compulsive disorders, drug addictions, cognition deficiency disorders, mood disorders or mood episodes, or neurodegenerative disorders.

In this context, examples of anxiety disorders, which may be treated by the compounds according to the present invention, include, without being limited thereto, panic disorder, agoraphobia, a specific phobia, social phobia, obsessive-compulsive disorder, post-traumatic stress disorder, acute stress disorder, or generalized anxiety disorder.

Examples of psychotic disorders, which may be treated by the compounds according to the present invention, include, without being limited thereto, schizophrenia (for example of the paranoid, disorganized, catatonic, undifferentiated, or residual type), schizophreniform disorder, schizoaffective disorder (for example of the delusional type or the depressive type), delusional disorder, substance-induced psychotic disorder (for example psychosis induced by alcohol, amphetamine, cannabis, cocaine, hallucinogens, inhalants, opioids, or phencyclidine), personality disorder of the paranoid type, or personality disorder of the schizoid type.

Examples of movement disorders, which may be treated by the compounds according to the present invention, include, without being limited thereto, Parkinson's disease, or restless leg syndrome.

Examples of obsessive/compulsive disorders, which may be treated by the compounds according to the present invention, include, without being limited thereto, Tourette's syndrome, or other tic disorders. Examples of drug addictions, which may be treated by the compounds according to the present invention, include, without being limited thereto, an alcohol, amphetamine, cocaine, or opiate addiction.

Examples of cognition deficiency disorders, which may be treated by the compounds according to the present invention, include, without being limited thereto, Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia, delirium, amnestic disorder, post-traumatic stress disorder, mental retardation, a learning disorder, for example reading disorder, mathematics disorder, or a disorder of written expression, attention-deficit/hyperactivity disorder, or age-related cognitive decline.

Examples of mood disorders or mood episodes, which may be treated by the compounds according to the present invention, include, without being limited thereto, a major depressive episode of the mild, moderate or severe type, a manic or mixed mood episode, a hypomanic mood episode, a depressive episode with a typical features, a depressive episode with melancholic features, a depressive episode with catatonic features, a mood episode with postpartum onset, post-stroke depression, major depressive disorder, dysthymic disorder, minor depressive disorder, premenstrual dysphoric disorder, post-psychotic depressive disorder of schizophrenia, a major depressive disorder superimposed on a psychotic disorder such as delusional disorder or schizophrenia, a bipolar disorder (for example bipolar I disorder, bipolar II disorder), or cyclothymic disorder.

Examples of neurodegenerative disorders, which may be treated by the compounds according to the present invention, include, without being limited thereto, Parkinson's disease, Huntington's disease, dementia (for example Alzheimer's disease, multi-infarct dementia, AIDS-related dementia, or Fronto temperal Dementia), neurodegeneration associated with cerebral trauma, neurodegeneration associated with stroke, neurodegeneration associated with cerebral infarct, hypoglycemia-induced neurodegeneration, neurodegeneration associated with epileptic seizure, neurodegeneration associated with neurotoxin poisoning, or multi-system atrophy.

Yet in this context, the compounds according to the present invention may be of potential value for treating diseases or conditions, in which abnormal function of the basal ganglia has been implicated. Thus, abnormal function of the basal ganglia may be involved in disregulated motoric, appetitive and/or cognitive processes. Exemplary neuropsychiatric conditions, in which abnormal function of the basal ganglia has been implicated, are mentioned e.g. in EP 1250923, US 2003/0008806 and/or US 2003/0018047, such as e.g. psychosis, attention-deficit/hyperactivity disorder (ADHD) and related attentional disorders, depression, obsessive conpulsive disorders including Tourette's syndrome and other tic disorders, and substance abuse. Several neurological disorders including Parkinson's disease, restless leg syndrom and Huntington's disease can be also linked to basal ganglia dysfunction.

Still yet in this context, the compounds according to the present invention may be of potential value for improving cognition, powers of concentration, learning skills or hypermesia, in particular if the disorder is a symptom of dementia.

Yet furthermore, the compounds according to the present invention may be, , of potential value for regulating fertility, e.g. via reducing spermatogenesis and/or via reducing sperm motility.

Still yet furthermore, the compounds according to the present invention may be, , of potential value for treating diabetes, such as, for example, typ II diabetes, e.g. via augmenting glucose-induced insulin secretion.

A special interest in the compounds according to the present invention lies in their use in therapy of schizophrenia.

Another special interest in the compounds according to the present invention lies in their use in the therapy of psychotic disorders.

Another special interest in the compounds according to the present invention lies in their use in the therapy of drug addictions.

The invention further relates to the compounds according to the invention for use in the treatment or prophylaxis of diseases, in particular said diseases and/or disorders.

The invention likewise relates to the use of the compounds according to the invention in the manufacture of pharmaceutical compositions which are employed for the treatment of said diseases or disorders.

The invention further relates to pharmaceutical compositions for the treatment or prophylaxis of the said diseases and/or disorders, which pharmaceutical compositions comprise one or more of the compounds according to the invention.

The present invention further relates to pharmaceutical compositions comprising one or more of the compounds according to this invention and a pharmaceutically acceptable carrier or diluent.

The present invention further relates to combinations comprising one or more of the compounds according to this invention and pharmaceutically acceptable auxiliaries, excipients or vehicles, e.g. for use in the treatment of those conditions mentioned above.

The present invention further relates to the use of the compounds according to this invention for the production of pharmaceutical compositions which can be used use in therapy of disorders responsive to inhibiting of PDE, such as e.g. PDE10.

The present invention further relates to compounds according to this invention having PDE, particularly PDE10, inhibiting properties.

The present invention further relates to pharmaceutical combinations or compositions according to this invention having PDE10 inhibiting properties.

The invention further relates to the use of a pharmaceutical composition comprising one or more of the compounds according to this invention as sole active ingredient(s) and a pharmaceutically acceptable carrier or diluent in the manufacture of pharmaceutical products for therapy, amelioration or prophylaxis of the illnesses, diseases, disorders or conditions mentioned above.

The invention furthermore relates to a commercial product which consists of a customary secondary packaging means, a primary packaging means (for example an ampoule or a blister pack) which contains a pharmaceutical composition, and, if desired, a patient information leaflet, with the pharmaceutical composition exhibiting an antagonistic effect toward type 10 cyclic nucleotide phosphodiesterases (PDE10) and leading to the attenuation of the symptoms of diseases and/or disorders which are associated with type 10 cyclic nucleotide phosphodiesterases, and with reference being made, on the secondary packaging means and/or on the patient information leaflet of the commercial product, to the suitability of the pharmaceutical composition for use in the prophylaxis or treatment of diseases and/or disorders which are associated with type 10 cyclic nucleotide phosphodiesterases, and with the pharmaceutical composition comprising one or more compounds according to this invention. The secondary packaging means, the primary packaging means containing the pharmaceutical composition and the patient information leaflet otherwise correspond to what the skilled person would regard as being the standard for drugs of this nature.

The pharmaceutical compositions according to this invention are produced using methods with which the skilled person is familiar. When employed in pharmaceutical compositions, the compounds according to the invention (= active compounds) are either used as such or, preferably, in combination with suitable pharmaceutical auxiliaries or formulating agents, for example in the form of tablets, coated (e.g. sugar-coated) tablets, capsules, caplets, suppositories, patches (e.g. as TTS), plasters, emulsions, suspensions, gels or solutions, with the content of active compound advantageously being between 0.1 and 95%, and where, by the appropriate choice of the auxiliaries, a pharmaceutical administration form (e.g. a delayed release form or an enteric form) exactly suited to the active compound and/or to the desired onset of action can be achieved.

The person skilled in the art is familiar, on the basis of his/her knowledge, with auxiliaries, vehicles, formulating agents, carriers, diluents, adjuvants or excipients which are suitable to be used for the desired pharmaceutical formulations, preparations or compositions. Beside solvents, gel-forming agents, suppository bases, tablet auxiliaries and other active carriers, it is possible to use, for example, antioxidants, dispersants, emulsifiers, antifoams, flavor corrigents, preservatives, solubilizers, colorants or, in particular, permeation promoters and complexing agents (e.g. cyclodextrines).

The administration of the pharmaceutical compositions according to the invention may be performed in any of the generally accepted modes of administration available in the art. Illustrative examples of suitable modes of administration include intravenous, inhalative, oral, nasal, parenteral, topical, transdermal and rectal delivery. Oral or intravenous delivery are preferred.

The pharmaceutical compositions according to the invention are prepared by processes known per se. For producing the drugs, the compounds according to the invention (= active compounds) are preferably mixed with suitable pharmaceutical auxiliary substances and further processed into suitable medicinal formulations. Suitable medicinal formulations which may be mentioned by way of example are powders, emulsions, suspensions, sprays, oils, ointments, greasy ointments, creams, pastes, gels and solutions.

The required dosage of the active compounds according to this invention can vary depending on the mode of administration, the particular condition to be treated and the effect desired. In general, satisfactory results are indicated to be obtained systemically at daily dosages of from about 0.01 to about 100 mg/kg body weight, conveniently administered, for example, in divided doses up to four times a day or in retard form.

The optimal dose and manner of administration of the active compounds necessary in each case can easily be determined by any person skilled in the art on the basis of his/her expert knowledge.

Depending upon the particular disease, to be treated or prevented, additional therapeutic active agents, which are normally administered to treat or prevent that disease, may optionally be coadministered separately, simultaneously, sequentially or chronologically staggered with the compounds according to this invention. As used herein, additional therapeutic agents that are normally administered to treat or prevent a particular disease are known as appropriate for the disease being treated.

The person skilled in the art is aware on the base of his/her expert knowledge of the total daily dosage(s) of the additional therapeutic agent(s) coadministered. Said total daily dosage(s) can vary within a wide range.

### Biological investigations

Methods to determine the activity and selectivity of a phosphodiesterase inhibitor are known to the person skilled in the art. In this connection it may be mentioned, for example, the methods described by Thompson et al. (Adv Cycl Nucl Res 10: 69-92, 1979), Giembycz et al. (Br J Pharmacol 118: 1945-1958, 1996) and the phosphodiesterase scintillation proximity assay of Amersham Pharmacia Biotech.

### Inhibiting the activity of PDE10A

The PDE10A is cloned into pCR2.1-Topo (Invitrogen) via PCR from human whole brain cDNA using primers OZ 353 (5'- ACCATGTTGACAGATGAAAAAGTGAAGGC -3') and OZ 317 (5'-TCAATCTTCAGATGCAGCTGCC -3'). The ORF encoding for the PDE10A is cut with EcoRV and BamHI and subcloned into Smal and Bgl II of the expression vector pBP9 (Clontech). The encoded protein represents the PDE10A1 (GenBank Acc.-# AB020593) truncated at its N-terminus at aa 14.

The recombinant baculoviruses are prepared by means of homologous recombination in Sf9 insect cells. The expression plasmids are cotransfected with Bac-N-Blue (Invitrogen) or Baculo-Gold DNA (Pharmingen) using a standard protocol (Pharmingen). Wildtype virus-free recombinant virus supernatants are selected using plaque assay methods. After that, high-titre virus supernatants are prepared by amplifying 3 times. PDE10A1 is expressed in Sf21 cells by infecting 2x10⁶ cells/ml with an MOI (multiplicity of infection) between 1 and 10 in serum-free SF900 medium (Life Technologies. Paisley, UK). Cells are cultured at 28°C, typically for 48 hours, after which they are pelleted for 5-10 min at 1000 g and 4°C. In spinner flasks, cells are cultured at a rotational speed of 75 rpm. The SF21 insect cells are resuspended, at a concentration of approx. 1x10⁷ cells/ml, in ice-cold (4°C) homogenization buffer (20 mM Tris, pH 8.2, containing the following additions: 140 mM NaCl, 3.8 mM KCl, 1 mM EGTA, 1 mM MgCl₂, 10 mM β-mercaptoethanol, 2 mM benzamidine, 0.4 mM Pefabloc, 10 µM leupeptin, 10 µM pepstatin A, 5 µM trypsin inhibitor) and disrupted by ultrasonication on ice. The homogenate is then centrifuged for 10 min at 1000 g (4 °C) and the supernatant is stored at - 80 °C until subsequent use (see below). The protein content is determined by the Bradford method (BioRad, Munich) using BSA as the standard.

The PDE10A activity is inhibited by said compounds in a modified SPA (scintillation proximity assay) test, supplied by Amersham Pharmacia Biotech (see procedural instructions "Phosphodiesterase [3H]cAMP SPA enzyme assay, code TRKQ 7090"), carried out in 96-well microtitre plates (MTPs). The test volume was 100 µl and contained 20 mM Tris buffer (pH 7.4), 0.1 mg of BSA (bovine serum albumin)/ml, 5 mM Mg²⁺, 0.5 µM cAMP (including about 50,000 cpm of [3H]cAMP), 1 µl of the respective substance dilution in DMSO and sufficient recombinant PDE10A1 (1000xg supernatant, see above) to ensure that 15-20% of cAMP was converted under said experimental conditions. After a preincubation of 5 min at 37°C, the reaction is started by adding a substrate (cAMP) and the assays are incubated for a further 15 min; after that, they are stopped by adding SPA beads (50 µl). In accordance with the manufacturer's instructions, the SPA beads have previously been resuspended in water and diluted 1:3 (v/v) and added to IBMX (3 mM). After the beads have been sedimented (> 30 min), the MTPs are analyzed in commercially available measuring appliances and the corresponding IC₅₀ values of the compounds for the inhibition of PDE10A activity are determined from concentration-effect curves by means of non-linear regression.

Representative inhibitory values [inhibitory concentration as -logIC₅₀ (mol/l)] which are determined for the compounds according to the invention are shown in the following table 1, in which the numbers of the compounds correspond to the numbers of the examples.

Particular interesting compounds according to this invention are those compounds mentioned in table 1 below.

**Table 1: Inhibition of PDE10A activity**

| Compounds | -log IC₅₀ |
|---|---|
| 5, 6, 8, 9, 11, 12, 13, 18, 20, 12, 13, 18, 20, 21, 23, 24, 26 to 35, 37 to 54, 56 to 62, 64 to 67 | The inhibitory values of the mentioned Examples lie in the range in the range from 7.01 to 9.58 |

## Claims

1. Compounds of formula I 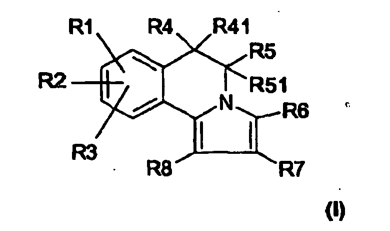 in which
R1 is halogen, nitro, amino, mono-or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 is hydrogen or 1-4C-alkoxy, or
R2 and R3 bound to the benzo ring moiety in ortho-position to each other together form a.1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together fonn a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂O-R411, in which
R411 is hydrogen, 1-4C-alkyl, 1-4C-alkoxy-2-4-alkyl or 1-4C-alkylcarbonyl,
R41 is hydrogen or 14C-alkyl,
R5 is fluorine or 1-4C-alkyl, and
R51 is hydrogen or 14C-alkyl,
or
R4 is hydrogen, fluorine, chlorine or 1-4C-alkyl,
R41 is hydrogen or 1-4C-alkyl,
R5 is fluorine, 1-4C-alkyl, trifluoromethyl, cyclopropyl, cyano, 1-4C-alkoxycarbonyl or
-CH₂-O-R511, in which
R511 is hydrogen, 1-4C-alkyl, 1-4C-alkoxy-2-4-alkyl or 1-4C-alkylcarbonyl, and
R51 is hydrogen or 1-4C-alkyl,
or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen, atom, to which R611 and R612 are bound, and, optionally, one further heteroatom selected from a group consisting of nitrogen, oxygen and sulfur, and optionally substituted by R613 on a ring nitrogen atom,
in which
R613 is 1-4C-alkyl, 3-7C-cycloalkyl, 3-7C-cycloalkyl-1-4C-alkyl, hydroxy-2-4-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R76-substituted Het2, naphthyl, or R76- and/or R77-substituted naphthyl, in which
Het2 is either
a monocyclic or fused bicyclic 5- to 10-membered heteroaryl radical comprising one to three heteroatoms, each of which is selected from a group consisting of nitrogen, oxygen and sulfur,
or
a fused bicyclic 9- or 10-membered, partially saturated heterocyclic ring radical containing a benzene ring and comprising one or two heteroatoms, each of which is selected from a group consisting of nitrogen, oxygen and sulfur,
or
N-oxy-pyridyl,
R71 is hydroxyl, halogen, nitro, cyano, trifluoromethyl, 14C-alkyl, 1-4C-alkoxy, amino, mono-or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, completely or predominantly fluorine-substituted 1-4C-alkoxy, mono- or di-1-4C-alkylaminocarbonyl, carbamoyl, tetrazolyl, or - N(H)S(O)₂-N(R712)R713, in which
aryl is phenyl or R711-substituted phenyl, in which
R711 is halogen,1-4C-alkyl, 1-4C-alkoxy, nitro or cyano,
R712 is 1-4C-alkyl, and
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl, phenyloxy, phenyl-1-4C-alkyl, arylsulphonyl, 1-4C-alkylsulphonyl, or -S(O)₂-N(R712)R713,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is 1-4C-alkyl, phenyl, 2-4C-alkinyl, cyano, -CH₂-O-R81, phenylcarbonyl, -C(O)-N(R82)R83 or -C(O)-OR9, in which
R81 is hydrogen, 1-4C-alkyl, 1-4C-alkoxy 2-4C-alkyl or 1-4C-alkylcarbonyl,
R82 is hydrogen, 1-4C-alkyl, 3-7C-cycloalkyl, 3-7C-cycloalkyl-1-4C-alkyl, phenyl or phenyl-1-4C-alkyl, and
R83 is hydrogen or 1-4C-alkyl, or
R82 and R83 together and with inclusion of the nitrogen atom, to which they are bound, form a heterocyclic ring radical selected from the group consisting of pyrrollidinyl, piperidinyl, morpholinyl or N-(1-4C-alkyl)-piperazinyl,
R9 is hydrogen or 1-4C-alkyl;
and the salts, stereoisomers, hydrates and hydrates of the salts of these compounds.

2. Compounds of formula I according to claim 1,
In which
R1 is hydroxy, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 Is 1-4C-elkoxy, or
R2 and R3 bound to the benzo ring moiety In ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
and none of R1, R2 and R3 is bound to the 10-position of the pyrrolo[2.1-a]isoquinollne ring,
R4 is hydrogen or 1-4C-alkyl,
R41 is hydrogen or 1-4C-alkyl,
R5 is 1-4C-alkyl, cyano or 1-4C-alkoxycarbonyl, and
R51 is hydrogen or 1-4C-alkyl,
or
R4 and R5 together form a 1-4C-alkylene bridge and R41 and R51 are both hydrogen,
R6 is 1-6C-alkyl, or 1-4C-alkyl substituted by R61, in which
R61 is 1-4C-alkoxycarbonyl or -N(R611)R612, in which
R611 is 1-4C-alkyl, and
R612 is 1-4C-alkyl, or
R611 and R612 together and with inclusion of the nitrogen atom to which they are bound form a radical Het1, in which
Het1 is pyrroildin-1-yl, piperidin-1-yl, morpholin-1-yl, or N-(1-4C-alkyl)-piperazinyl,
R7 is Het2, R71- and/or R72- and/or R73-substituted phenyl, R74-substituted Het2, or naphthyl, in which
Het2 is either
a monocyclic or fused bicyclic 5- to 10-membered heteroaryl radical comprising one to three heteroatoms, each of which is selected from a group consisting of nitrogen, oxygen and sulfur,
or
a fused bicyclic 9- or 10-membered, partially saturated heterocyclic ring radical containing a benzene ring and comprising one or two heteroatoms, each of which is selected from a group consisting of nitrogen, oxygen and sulfur,
or
N-oxy-pyridyl,
R71 is hydroxyl, halogen, nitro, cyano, trifluoromethyl, 1-4C-alkyl, 1-4C-alkoxy, amino, mono- or di-1-4C-alkylamino, 1-4C-alkylsulphonylamino, 1-4C-alkoxycarbonyl, carboxyl, aryloxy, completely or predominantly fluorine-substituted 1-4C-alkoxy, mono- or di-1-4C-alkylaminocarbonyl, carbamoyl, tetrazolyl, or -N(H)S(O)₂N(R712)R713, in which
aryl Is phenyl or R711-substituted phenyl, in which
R711 is halogen or 1-4C-alkyl,
R712 is 1-4C-alkyl, and
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl,
R72 is halogen, 1-4C-alkyl or 1-4C-alkoxy,
R73 is 1-4C-alkyl or 1-4C-alkoxy, .
R74 is 1-4C-alkyl, phenyl-1-4C-alkyl, arylsulphonyl, 1-4C-alkylsulphonyl, or -S(O)₂-N(R712)R713,
R8 is 1-4C-alkyl, cyano, or -C(O)-OR9, in which
R9 is hydrogen or 1-4C-alkyl;
and the salts, stereoisomers, hydrates and hydrates of the salts of these compounds.

3. Compounds of formula I according to claim 1,
in which
R1 is bound to the 8-position of the pyrrolo[2.1-a]isoquinoline ring, and is 1-4C-alkoxy,
R2 is bound to the 7-position of the pyrrolo[2.1-a]isoquinoline ring, and is hydrogen, halogen or 1-4C-alkoxy,
R3 is bound to the 9-position of the pyrrolo[2.1-a]isoquinoline ring, and is 1-4C-alkoxy,
R4 is hydrogen,
R41 is hydrogen,
R5 is 1-4C-alkyl, cyano or 1-4C-alkoxycarbonyl, and
R51 is hydrogen or 1-4C-alkyl,
or
R4 and R5 together form a 3-4C-alkylene bridge and R41 and R51 are both hydrogen,
R6 is 1-4C-alkyl, or 1-4C-alkyl substituted by R61, in which
R61 is 1-4C-alkoxycarbonyl or -N(R611)R612, in which
R611 and R612 together and with inclusion of the nitrogen atom to which they are bound form a radical Het1, in which
Het1 is morpholin-1-yl,
R7 is Het2, R71- and/or R72- and/or R73-substituted phenyl, R74-substituted Het2, or naphthyl, in which
Het2 is either
a monocyclic or fused bicyclic 5- to 10-membered heteroaryl radical comprising one to three heteroatoms, each of which is selected from a group consisting of nitrogen, oxygen and sulfur,
or
a fused bicyclic 9- or 10-membered, partially saturated heterocyclic ring radical containing a benzene ring and comprising one or two heteroatoms, each of which is selected from a group consisting of nitrogen, oxygen and sulfur,
or
N-oxy-pyridyl,
R71 is hydroxyl, halogen, nitro, 1-4C-alkyl, 1-4C-alkoxy, amino, mono- or di-1-4C-alkylamino, 1-4C-alkylsulphonylamino, carboxyl, aryloxy, mono- or di-1-4C-alkylaminocarbonyl, carbamoyl, tetrazolyl, or -N(H)S(O)₂-N(R712)R713, in which
aryl is phenyl or R711-substituted phenyl, in which
R711 is halogen or 1-4C-alkyl,
R712 is 1-4C-alkyl, and
R713 is 1-4C-alkyl, or
R712 and R713 together and with inclusion of the nitrogen atom to which they are bound form a radical Het3, In which
Het3 is morpholin-4-yl,
R72 is halogen, 1-4C-alkyl or 1-4C-alkoxy,
R73 Is 1-4C-alkyl or 1-4C-alkoxy,
R74 is 1-4C-alkyl, phenyl-1-4C-alkyl, arylsulphonyl, 1-4C-alkylsulphonyl, or -S(O)₂-N(R712)R713,
R8 is 1-4C-alkyl, cyano, or -C(O)-OR9, in which
R9 is hydrogen or 1-4C-alkyl;
and the salts, stereoisomers, hydrates and hydrates of the salts of these compounds.

4. Compounds of formula I according to claim 1,
in which
either, In a first independent embodiment,
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy,
R4 is hydrogen,
R41 is hydrogen,
R5 is 1-2C-alkyl or cyano, and
R51 is hydrogen,
or
R4 and R5 together form a tetramethylene bridge and R41 and R51 are both hydrogen,
R6 is 1-2C-alkyl, or 1-2C-alkyl substituted by R61, in which
R61 is 1-2C-alkoxycarbonyl or -N(R611)R612, In which
R611 and R612 together and with inclusion of the nitrogen atom to which they are bound form a radical Het1, in which
Het1 is morpholin-1-yl,
R7 is naphthyl, 4-hydroxy-3,5-dimethylphenyl, 4-methoxy-3,5-dimethylphenyl, 4-carboxy-phenyl, 4-carbamoyl-phenyl, 2-methyl-4-hydroxy-phenyl, 4-amino-phenyl, 4-(2H-tetrazol-5-yl)-phenyl, 4-morpholino-sulphonylamino-phenyl, 4-methylsulphonylamino-phenyl, or 2-fluoro-3,4-dimethoxyphenyl,
pyridyl, indolyl, quinolinyl, indolinyl,
2-methyl-pyridin-4-yl, 3-methyl-pyridin-4-yl, or
N-(R74)-Het2, in which
Het2 is pyrrolyl or indolyl,
R7R74 is arylsulphonyl, 1-2C-alkylsulphonyl, or -S(O)₂-N(R712)R713, in which
aryl is phenyl, or R711-substituted phenyl, In which
R711 Is 1-2C-alkyl,
R712 is 1-2C-alkyl, and
R713 is 1-2C-alkyl, or
R712 and R713 together and with inclusion of the nitrogen atom to which they are bound form a radical Het3, in which
Het3 is morpholin-4-yl, and
R8 is cyano;
or, in a second independent embodiment,
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine.
R3 is bound to the 9-position of the pyrrolo[2-1-a]isoquinoline ring, and is 1-20-alkoxy.
R4 is hydrogen,
R41 Is hydrogen,
R5 is 1-2C-alkyl or cyano, and
R51 is hydrogen,
or
R4 and R5 together form a tetramethylene bridge and R41 and R51 are both hydrogen,
R6 is 1-2C-alkyl, or 1-2C-alkyl substituted by R61, In which
R61 is 1-2C-alkoxycarbonyl or -N(R611)R612, in which
R611 and R612 together and with inclusion of the nitrogen atom to which they are bound form a radical Het1, in which
Het1 is morpholin-1-yl,
R7 is naphthyl, 4-hydroxy-3,5dimethylphenyl, 4-methoxy-3,5-dimethylphenyl, 4-carboxy-phenyl, 4-carbamoyl-phenyl, 2-methyl-4-hydroxy-phenyl, 4-amino-phenyl, 4-(2H-tetrazol-5-yl)-phenyl, 4-morpholino-sulphonylamino-phenyl, 4-methylsulphonylamino-phenyl, or 2-fluoro-3,4-dimethoxyphenyl,
pyridyl, indolyl, quinolinyl, indolinyl,
2-methyl-pyridin-4-yl, 3-methyl-pyridin-4-yl, or
N-(R74)-Het2, in which
Het2 is pyrrolyl or indolyl,
R74 is arylsulphonyl, 1-2C-alkylsulphonyl, or -S(O)₂-N(R712)R7713, in which
aryl is phenyl, or R711-substituted phenyl, in which
R711 is 1-2C-alkyl,
R712 is 1-2C-alkyl, and
R713 is 1-2C-alkyl, or
R712 and R713 together and with inclusion of the nitrogen atom to which they are bound form a radical Het3, in which
Het3 is morpholin-4-yl, and
R8 is -C(O)-OR9, In which
R9 is 1-2C-alkyl;
and the salts, stereoisomers, hydrates and hydrates of the salts of these compounds.

5. Compounds of formula I according to claim 1,
in which
either, in a first independent embodiment,
R1 is bound to the 8-position of the pyrrolo[2.1-a]isoquinoline ring, and is methoxy,
R2 is bound to the 7-position of the pyrrolo[2.1-a]isoquinoline ring, and is hydrogen or fluorine,
R3 is bound to the 9-posltion of the pyrrolo[2.1-a]isoquinoline ring, and is methoxy,
R4 is hydrogen,
R41 is hydrogen,
R5 is methyl or cyano,
R51 is hydrogen,
R6 is methyl, ethyl or 2-methoxycarbonylethyl,
R7 is 4-hydroxy-3,6-dimethylphenyl, 4-methoxy-3,5-dimethylphenyl, 4-carboxy-phenyl, 2-methyl-4-hydroxy-phenyl, 4-amino-phenyl, 4-(2H-tetrazol-5-yl)-phenyl, 4-morpholino-sulphonylamino-phenyl, 4-methylsulphonylamino-phenyl,
pyridyl, quinolinyl,
2-methyl-pyridin-4-yl, 3-methyl-pyridin-4-yl,
1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonyl-indol-3-yl, and
R8 is cyano;
or, in a second independent embodiment,
R1 is bound to the 8-position of the pyrrolo[2.1-a]isoquinoline ring, and is methoxy,
R2 is bound to the 7-position of the pyrrolo[2.1-a]isoquinoline ring, and is hydrogen or fluorine,
R3 is bound to the 9-position of the pyrrolo[2.1-a]isoquinoline ring, and is methoxy,
R4 is hydrogen,
R41 is hydrogen,
R5 is methyl or cyano,
R51 is hydrogen,
R6 is methyl, ethyl or 2-methoxycarbonylethyl,
R7 is 4-hydroxy-3,5-dimethylphenyl, 4-methoxy-3,5-dimethylphenyl, 4-carboxy-phenyl, 2-methyl-4-hydroxy-phenyl, 4-amino-phenyl, 4-(2H-tetrazol-5-yl)-phenyl, 4-morpholino-sulphonylamino-phenyl, 4-methylsulphonylamino-phenyl,
pyridyl, quinolinyl,
2-methyl-pyridin-4-yl, 3-methyl-pyridin-4-yl,
1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonylindol-3-yl, and
R8 Is -C(O)-OR9, in which
R9 is methyl or ethyl;
and the salts, stereoisomers, hydrates and hydrates of the salts of these compounds.

6. Compounds of formula I according to claim 1,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is fluorine,
R3 is bound to the 9-position of the pyrrolo[2.1-a]isoquinoline ring, and is 1-2C-alkoxy, such as e.g. methoxy,
R4 is hydrogen,
R41 Is hydrogen.
R5 is methyl or cyano,
R51 is hydrogen,
R6 is methyl, ethyl or 2-methoxycarbonylethyl,
R7 is 4-hydroxy-3,5-methylphenyl, 4-methoxy-3,5-methylphenyl, 4-carboxy-phenyl, 2-methyl-4-hydroxy-phenyl, 4-amino-phenyl, 4-(2H-tetrazol-5-yl)-phenyl, 4-morpholino-sulphonylamino-phenyl, 4-methylsulphonylamino-phenyl,
pyridyl, quinolinyl,
2-methyl-pyridin-4-yl, 3-methyl-pyridin-4-yl,
1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonylindol-3-yl,
R8 is cyano;
and the salts, stereoisomers, hydrates and hydrates of the salts of these compounds.

7. Compounds of formula I according to claim 1,
in which
R1 is bound to the 8-position of the pyrrolo[2.1-a]isoquinoline ring, and is methoxy,
R2 is bound to the 7-position of the pyrrolo[2.1-a]isoquinoline ring, and is fluorine,
R3 is bound to the 9-position of the pyrrolo[2.1-a]isoquinoline ring, and is methoxy,
R4 is hydrogen,
R41 is hydrogen,
R5 is methyl,
R51 is hydrogen,
R6 is methyl,
R7 is 4-hydroxy-3,5-dimethylphenyl, 4-methoxy-3,5-dimethylphenyl, 4-carboxy-phenyl, 2-methyl-4-hydroxy-phenyl, 4-amino-phenyl, 4-(2H-tetrazol-5-phenyl), 4-morpholino-sulphonylamino-phenyl, 4-methylsulphonylamino-phenyl,
pyridyl, quinolinyl,
2-methyl-pyridin-4-yl, 3-methyl-pyridin-4-yl,
1-tolylsulphonyl-pyrrol-3-yl, 1-tolylsulphonyl-indol-3-yl, 1-phenylsulphonyl-indol-3-yl, 1-methylsulphonyl-indol-3-yl, 1-dimethylaminosulphonyl-indol-3-yl, or 1-morpholinosulphonylindol-3-yl,
R8 is cyano;
and the salts, stereoisomers, hydrates and hydrates of the salts of these compounds.

8. Compounds of formula I according to claim 1,
in which
R1 is halogen or 1-2C-alkoxy,
R2 is hydrogen or 1-2C-alkoxy,
R3 is 1-2C-alkoxy,
R4 is hydrogen,
R41 is hydrogen,
R5 is 1-2C-alkyl,
R51 is hydrogen,
R6 is methyl, ethyl or methoxycabonylethyl,
R7 is phenyl, Het2, R71- and/or R72-and/or R73-substituted phenyl, or naphthyl, in which
Het2 is a heteroaryl radical selected from the group consisting of furanyl, thiophenyl, pyrrolyl pyridinyl, quinolyl, indolyl, benzothiophenyl and benzofuranyl,
R71 is hydroxyl, chlorine, methoxy, dimethylamino, or aryloxy, in which
aryl is R711-substituted phenyl, in which
R711 is chlorine,
R72 is methyl, tert-butyl or methoxy,
R73 is methyl, tert-butyl or methoxy,
R8 is cyano,
and the salts, stereoisomers, hydrates and hydrates of the salts of these compounds.

9. Compounds according to claim 1, which are of formulae Ia or Ib, 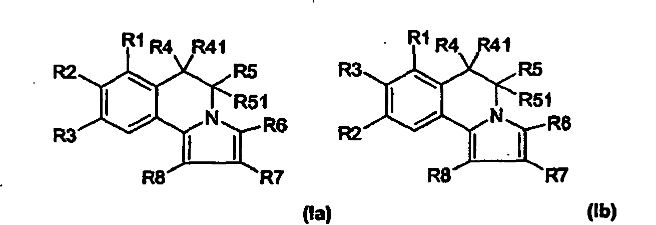 in which,
as a first alternative,
R1 is hydrogen.
R2 is chlorine or fluorine,
R3 is methoxy or ethoxy,
or, as a second alternative,
R1 is hydrogen,
R2 is methoxy or ethoxy,
R3 is methoxy or ethoxy,
or, as a third alternative,
R1 is methoxy or ethoxy,
R2 is chlorine or fluorine,
R3 is methoxy or ethoxy,
or, as a fourth alternative,
R1 is chlorine or fluorine,
R2 is methoxy or ethoxy,
R3 is methoxy or ethoxy,
or, as a fifth alternative,
R1 is methoxy or ethoxy,
R2 is methoxy or ethoxy,
R3 is methoxy or ethoxy,
R4 is hydrogen,
R41 is hydrogen,
R5 is methyl,
R51 is hydrogen,
R6 is methyl, ethyl or methoxycarbonylethyl,
R7 is Het2, R75-substituted Het2, or 4-hydroxy-3,5-dimethyl-phenyl, in which
Het2 is pyridinyl or quinolinyl,
R75 is 1-4C-alkyl,
R8 is cyano,
and the salts, stereoisomers, hydrates and hydrates of the salts of these compounds.

10. Compounds according to any of the preceding claims,
In which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy, such as e.g. methoxy,
and
R4 is hydrogen,
R41 is hydrogen,
R5 is 1-2C-alkyl or cyano,
R51 is hydrogen,
and
R8 is cyano,
and the salts, stereoisomers, hydrates and hydrates of the salts of these compounds.

11. Compounds according to any of the claims 1 to 9,
in which
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine,
R3 is bound to the 9-position of the pyrrolo[2.1-a]isoquinoline ring, and is 1-2C-alkoxy, such as e.g. methoxy,
and
R4 is hydrogen,
R41 is hydrogen,
R5 is 1-2C-alkyl or cyano,
R51 is hydrogen,
and
R8 is cyano,
and the salts, stereoisomers, hydrates and hydrates of the salts of these compounds.

12. A compound according to any of the claims 1 to 9,
wherein said compound is from formula Ia as defined in claim 9, in which
R2 is methoxy,
R3 is methoxy,
R4 is hydrogen,
R41 is hydrogen,
R51 is hydrogen,
and in which R1, R5, R8 and R8 have any one of the meanings 1.) to 75.) specified in the following table:
| | R1 | R5 | R6 | R8 |
|---|---|---|---|---|
| 1.) | hydrogen | methyl | methyl | cyano |
| 2.) | hydrogen | methyl | methyl | ethoxycarbonyl |
| 3.) | hydrogen | methyl | 2-methoxycarbonytethyl | cyano |
| 4.) | hydrogen | methyl | 2-methoxycarbonylethyl | ethoxycarbonyl |
| 7.) | fluorine | methyl | methyl | cyano |
| 8.) | fluorine | methyl | methyl | ethoxycarbonyl |
| 9.) | fluorine | methyl | 2-methoxycarbonylethyl | cyano |
| 10.) | fluorine | methyl | 2-methoxycarbonylethyl | ethoxycarbonyl |
| 15.) | hydrogen | cyano | methyl | cyano |
| 16.) | hydrogen | cyano | methyl | ethoxycarbonyl |
| 17.) | hydrogen | cyano | 2-methoxycarbonylethyl | cyano |
| 18.) | hydrogen | cyano | 2-methoxycarbonylethyl | ethoxycarbonyl |
| 19.) | fluorine | cyano | methyl | cyano |
| 20.) | fluorine | cyano | methyl | ethoxycarbonyl |
| 21.) | fluorine | cyano | 2-methoxycarbonylethyl | cyano |
| 22.) | fluorine | cyano | 2-methoxycarbonytethyl | ethoxycarbonyl |
| 23.) | chlorine | methyl | methyl | cyano |
| 24.) | chlorine | methyl | methyl | ethoxycarbonyl |
| 25.) | chlorine | methyl | 2-methoxycarimnytethyl | cyano |
| 26.) | chlorine | methyl | 2-methoxycarbonylethyl | ethoxycarbonyl |
| 31.) | chlorine | cyano | methyl | cyano |
| 32.) | chlorine | cyano | methyl | ethoxycarbonyl |
| 33.) | chlorine | cyano | 2-methoxycarbonylethyl | cyano |
| 34.) | chlorine | cyano | 2-methoxycarbonviethyl | ethoxycarbonyl |
| 35.) | hydrogen | methyl | methyl | methoxycarbonyl |
| 36.) | hydrogen | methyl | 2-methoxycarbonylethyl | methoxycarbonyl |
| 37.) | fluorine | methyl | methyl | methoxycarbonyl |
| 38.) | fluorine | methyl | 2-methoxycarbonylethyt | methoxycarbonyl |
| 41.) | hydrogen | cyano | methyl | methoxycarbonyl |
| 42.) | hydrogen | cyano | 2-methoxycarbonylethyl | methoxycarbonyl |
| 43.) | fluorine | cyano | methyl | methoxycarbonyl |
| 44.) | fluorine | cyano | 2-methoxycarbonytethyl | methoxycarbonyl |
| 45.) | chlorine | methyl | methyl | methoxycarbonyl |
| 46.) | chlorine | methyl | 2-methoxycarbonylethyl | methoxycarbonyl |
| 49.) | chlorine | cyano | methyl | methoxycarbonyl |
| 50.) | chlorine | cyano | 2-methoxycarbonylethyl | methoxycarbonyl |
| 51.) | hydrogen | methyl | ethyl | cyano |
| 52.) | hydrogen | methyl | ethyl | ethoxycarbonyl |
| 54.) | fluorine | methyl | ethyl | cyano |
| 55.) | fluorine | methyl | ethyl | ethoxycarbonyl |
| 58.) | hydrogen | cyano | ethyl | cyano |
| 59.) | hydrogen | cyano | ethyl | ethoxycarbonyl |
| 60.) | fluorine | cyano | ethyl | cyano |
| 61.) | fluorine | cyano | ethyl | ethoxycarbonyl |
| 62.) | chlorine | methyl | ethyl | cyano |
| 63.) | chlorine | methyl | ethyl | ethoxycarbonyl |
| 66.) | chlorine | cyano | ethyl | cyano |
| 67.) | chlorine | cyano | ethyl | ethoxycarbonyl) |
| 68.) | hydrogen | methyl | ethyl | methoxycarbonyl |
| 69.) | fluorine | methyl | ethyl | methoxycarbonyl |
| 71.) | hydrogen | cyano | ethyl | methoxycarbonyl |
| 72) | fluorine | cyano | ethyl | methoxycarbonyl |
| 73.) | chlorine | methyl | ethyl | methoxycarbonyl |
| 75.) | chlorine | cyano | ethyl | methoxycarbonyl |
or a salt, stereoisomer, hydrate or hydrate of a salt of this compound.

13. A compound according to claim 1, which is selected from the group consisting of:
1. 2-(4-Hydroxy-3,5-dimethyl-phenyl)-8,9-dimethoxy-3,5,5-trimethyl-5,6-dihydro-pyrrolo[2,1-α]isaquinoline-1-carboxylic acid ethyl ester
2. 8,9-Dimethoxy-3,5,5-trimethyl-2-(3,4,5-trimethoxy-phenyl)-5,6-dihydro-pyrrolo[2,1-α]isoquinoline-1-carboxylic acid ethyl ester .
3. 2-[3-(4-Chloro-phenoxy)-phenyl]-8,9-dimethoxy-3,5,5-trimethyl-5,6-dihydro-pyrrolo[2,1-a]isoquinoline-1-carboxylic add ethyl ester
4. 2-(3-Dimethylamino-phenyl)-8,9-dimethoxy-3,5,5-methyl-5,8-dihydro-pyrrolo[2,1-a]isoquinoline-1-carboxylic-acid ethyl ester
5. (5RS)- (4-Hydroxy-3,5-dimethyl-phenyl)-8,9-dimethoxy-3,5-dimethyl-5,8-dihydro-pyrrolo[2,1-a]isoquinoline-1-carboxylic acid ethyl ester
6. (5RS)-5-Ethyl-2-(4-hydroxy-3,5-dimethyl-phenyl)-8,9-dimethoxy-3-methyl-5,8-dihydropyrrolo[2,1-a]isoquinoline-1-carboxylic add ethyl ester
7. (5RS)-2-Chloro-5-ethyl-8,9-dimethyl-3-methyl-5,6-dihydro-pyrrolo[2,1-a]isoquinoline-1-carboxylic acid ethyl ester
8. (4aRS,8aRS)-ds-2-(4-hydroxy-3,5-dimethyl)-10,11-dimethoxy-3-methyl-4a,5,6,7,8a-hexahydro-pyrrolo[2,1-f]phenanthridine-1-carboxylic add ethyl ester
9. (5RS)-3-Ethyl-2-(4-hydroxy-3,5-dimethyl-phenyl)-8,9-dimethoxy-5-methyl-5,8-dihydropyrrolo[2.1-a]isoquinoline-1-carboxylic add ethyl ester
10. (5RS)-8,9-Dimethoxy-3,5-dimethyl-2-(3,4,5-trimethoxy-phenyl)-5,6-dihydro-pyrrolo[2,1-a]Isoquinoline-1-carboxylic acid ethyl ester
11. (5RS)-8,9-Dimethoxy-3,5-dimethyl-2-naphthalen-1-yl-5,6-dihydro-pyrrolo[2, 1-a]isoquinoline-1-carboxylic add ethyl ester
12. (4aRS,8aRS)-cis-10,11-Dimethoxy-3-methyl-2-naphthalen-1-yl-4a,5,6,7,8,8a-hexahydropyrrolo[2,1-f]phenanthridine-1-carboxylic add ethyl ester
13. (4aRS,8aRS)-cis-10,11-Dimethoxy-3-methyl-2-quinolin-4-yl-4a,5,6,7,8,8a-hexahydropyrrolo[2,1-f]phenanthridine-1-carboxylic add ethyl ester
14. (4aR,8aR)-10,11-Dimethoxy-3-methyl-2-quinolin-4-yl-4a,5,6,7,8,8a-hexahydro-pyrrolo[2,1-f]phenanthridine-1-carboxylic acid ethyl ester
15. (4aR,8aR)-10,11-Dimethoxy-3-methyl-2-naphthaten-1-yl-4a,5,8,7,8,8a-hexahydro-pyrrolo[2,1-f]phenanthridine-1-carboxylic acid ethyl ester
16. (4aR,8aR)-2-(4-Hydroxy-3,5-dimethyl-phenyl)-10,11-dimethoxy-methyl-4a,5,8,7,8,8a-hexahydro-pyrrolo[2,1-f]phenanthridine-1-carboxylic add ethyl ester
17. (5RS)-5-Ethyl-8,9-dimethoxy-3-methyl-2-aphthalen-1-yl-5,6-dihydro-pyrrolo[2,1-a]isoquinolin-1-carboxylic acid ethyl ester
18. (5RS)-2-(4-Hydroxy-3,5-dimethyl-phenyl)-7,8,9-trimethoxy-3,5-dimethyl-5,6-dihydropyrrolo[2,1-a]isoquinoline-1-carboxylic add ethyl ester
19. 2-(4-Hydroxy-3,5-dimethyl-phenyl)-8,9-dimethoxy-5,6-dihydro-pyrrolo[2,1-a]isoquinoline-1,5-dicarboxylic acid 1-ethyl 5-methyl ester
20. (5RS)-8,8-Dimethoxy-3-(2-methoxycarbonyl-ethyl)-5-methyl-2-naphthalen-1-yl-5,6-dihydropyrrolo[2,1-a]Isoquinolino-1-carboxylic acid ethyl ester
26. 8,9-Dimethoxy-3,5-dimethyl-2-pyridin-4-yl-5,6-dihydro-pyrrolo[2,1-a]isoquinoline-1-carbonitrile
27. 3-{1-Cyano-2-(4-hydroxy-3,5-dimethyl)-8,8-dimethoxy-5-methyl-5,8-dihydro-pyrrolo[2,1-a]Isoquino-3-yl]-propionic add methyl ester
28. 2-(4-Hydroxy-3,5-dimethyl-phenyl)-8,9-dimethoxy-3,5-dimethyl-5,6-dihydro-pyrrolo[2,1-a]isoquinoline-1-carbonitrile
or a salt, stereoisomer, hydrate or hydrate of a salt thereof.

14. A compound according to claim 1, which is selected from the group consisting of:
1. 2-(4-Hydroxy-3,5-dimethyl-phenyl)-8,9-dimethoxy-3,5,5-trimethyl-5,6-dihydro-pyrrolo[2,1-α]isoquinoline-1-carboxylic acid ethyl ester
2. 8,9-Dimethoxy-3,5,5-trimethyl-2-(3,4,5-trimethoxy-phenyl)-5,8-dihydro-pyrrolo[2,1-a]isoquinoline-1-carboxylic acid ethyl ester
3. 2-[3-(4-Chloro-phenoxy)-phenyl]-8,9-dimethoxy-3,5,5-trimethyl-5,6-dihydro-pyrrolo[2,1-a]isoquinoline-1-carboxylic acid ethyl ester
4. 2-(3-Dimethylamino-phenyl)-8,9-dimethoxy-3,5,5-trimethyl-5,6-dihydro-pyrrolo[2,1-a]isoquiniline-1-carboxylic acid ethyl ester
5. (5RS)- (4-hydroxy-3,5-dimethyl-phenyl)-8,9-dimethoxy-3,5-dimethyl-5.6-dihydro-pyrrolo[2,1-a]Isoquinoline-1-carboxylic add ethyl ester
6. (5RS)-5-Ethyl-2-(4-hydroxy-3,5-dimethyl-phenyl)-8,9-dimethoxy-3-methyl-5,8-dihydropyrolo[2,1-a]isoquinoline-1-carboxylic acid ethyl ester
7. (5RS)-2-Chloro-5-ethyl-8,8-dimethoxy-3-methyl-5,8-dihydro-pyrrolo[2,1-a]isoqulnoline-1-carboxylic acid ethyl ester
8. (4aRS,8aRS)-cis-2-(4-hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
9. (5RS)-3-Ethyl-2-(4-hydroxy-3,5-dimethyl-phenyl)-8,9-dimethoxy-5-methyl-5,8-dihydropyrrolo[2,1-a]isoquinoline-1-carboxylic acid ethyl ester
10. (5RS)-8,9-Dimethoxy-3,5-dimethyl-2-(3,4,5-trimethoxy-phenyl)-5,8-dihydro-pyrrolo[2,1-a]isoquinoline-1-carboxylic acid ethyl ester
11. (5RS)-8,9-Dimethoxy-3,5-dimethyl-2-nephthalen-1-yl-5,6-dihydro-pyrrolo[2,1-a]isoquinoline-1-carboxylic acid ethyl ester
12 (4aRS,8aRS)-cis-10.11-Dimethoxy-3-methyl-2-naphthalen-1-yl-4a,5,6,7,8,8a-hexahydropyrrolo[2,1-f]phenanthridine-1-carboxylic acid ethyl ester
13. (4aRS,8aRS)-cis-10,11-Dimethoxy-3-methyl-2-quinolin-4-yl-4a,5,6,7,8,8a-hexahydropyrrolo[2,1-f]phenanthridine-1-carboxylic acid ethyl ester
14. (4aR,8aR)-10,11-Dimethoxy-3-methyl-2-quinolin-4-yl-4a,5,6,7,8,8a-hexahydro-pyrrolo[2,1-f]phenanthridine-1-carboxylic acid ethyl ester
15. (4aR,8aR)-10,10,11-Dimethoxy-3-methyl-2-naphthalen-1-yl-4a,5,8,7,8,8a-hexahydro-pyrrolo[2,1-f]phenanthridine-1-carboxylic acid ethyl ester
16. (4aR,8aR)-2-(4-Hydroxy-3,5-dimethyl-phenyl)-10,11-dimethoxy-3-methyl-4a,5,8,7,8,8a-hexahydro-pyrrolo[2,1-f]phenanthridine-1-carboxylic add ethyl ester
17. (5RS)-5-Ethyl-8,9-dimethoxy-3-methyl-2-naphthalen-1-yl-5,8-dihydro-pyrrolo[2,1-a]isoquinoline-1-carboxylic acid ethyl ester
18. (5RS)-2-(4-Hydroxy-3,5-dimethyl-phenyl)-7,8,9-trimethoxy-3,5-dimethyl-5,8-dihydropyrrolo[2,1-a]isoquinoline-1-carboxylic acid ethyl ester
19. 2-(4-Hydroxy-3,5-dimethyl-phenyl)-8,9-dimethoxy-5,6-dihydro-pyrrolo[2,1-a]isoquinoline-1,5-dicarboxylic acid 1-ethyl 5-methyl ester
20. (5RS)-8,9-Dimethoxy-3-(2-methoxycarbonyl-ethyl)-5-methyl-2-naphthalen-1-yl-5,6-dihydropyrrolo[2,1-a]isoquinoline-1-carboxylic acid ethyl ester
26. 8,9-Dimethoxy-3,5-dimethyl-2-pyridin-4-yl-5,8-dihydro-pyrrolo[2,1-a]isoquinoline-1-carbonitrile
27. 3-[1-Cyano-2-(4-hydroxy-3,5-dimethyl)-8,9-dimethoxy-5-methyl-5,6-dihydro-pyrrolo[2,1-a]isoquinolin3-yl]-propionic acid methyl ester
28. 2-(4-Hydroxy-3,5-dimethyl-phenyl)-8,9-dimethoxy-3,5-dimethyl-5,8-dihydro-pyrrolo[2,1-a]isoquinoline-1-carbonitrile
30. 7-Fluoro-2-(4-hydroxy-3,5-dimethyl-phenyl)-8,9-dimethoxy-3,5-dimethyl-5,8-dihydropyrrolo[2,1-a]isoquinolin-1-carbonitrile
33. 8,9-Dimethoxy-2-(4-methoxy-3,5-dimethyl-phenyl)-3,5-dimethyl-5,6-dihydro-pyrrolo[2,1-a]Isoquinoline-1-carbonitrile
37. 8,9-Dimethoxy-3,5-dimethyl-2-[1-(toluene-4-sulfonyl)-1H-pyrrolo-3-yl]-5,8-dihydro-pyrrolo[2,1-a]isoquinoline-1-carbonitrile
38. 8,9-Dimethoxy-3,5-dimethyl-2-[1-(toluene-4-sulfonyl)-1H-indol-3-yl]-5,6-dihydro-pyrrolo[2,1-a]isoquinoline-1-carbonitrile
39. 2-(1-Benzenesulfonyl-1H-indol-3-yl)-8,9-dimethoxy-3,5-dimethyl-5,6-dihydro-pyrrolo[2,1-a]isoquinoline-1-carbonitrile
40. 2-(1-Methanesulfonyl-1H-indol-3-yl)-8,9-dimethoxy-3,5-dimethyl-5,8-dihydro-pyrrolo[2,1-a]isoquinoline-1-carbonitrile
41. 8,9-Dimethoxy-3,5-dimethyl-2-(1-oxy-pyridin-4-yl)-5,8-dihydro-pyrrolo[2,1-a]isoquinoline-1-carbonibile
42. 7-Fluoro-8,9-dimethoxy-3,5-dimethyl-2-[1-(toluene-4-sulfonyl)-1H-indol-3-yl]-5,8-dihydropyrrolo[2,1-a]isoquinoline-1-carbonitrile
44. 2-(4-Hydroxy-3,5-dimethyl-phenyl)-8,9-dimethoxy-5-methyl-3-morpholin-4-ylmethyl-5,6-dihydro-pyrrolo[2,1-a]isoquinoline-1-carbonitrile
45. 8,9-Dimethoxy-3,5-dimethyl-2-(2-methyl-pyridin-4-yl)-5,8-dihydro-pyrrolo[2,1-a]isoquinoline-1-carbonitrile
46. 8,9-Dimethoxy-3,5-dimethyl-2-(44,phenyl)-5,6-dihydro-pyrrolo[2,1-a]isoquinoline-1-carbonitrile
47. 4-(1-Cyano-8,9-dimethoxy-3,5-dimethyl-5,6-dihydropyrrolo[2,1-a]isoquinolin-2-yl)-benzoic
48. 2-(4-Amino-phenyl)-8,9-dimethoxy-3,5-dimethyl-5,8-dihydro-pyrrolo[2,1-a]isoquinoline-1-carbonitrile
49. 8,9-Dimethoxy-3,5-dimethyl-2-(3-methyl-pyridin-4-yl)5,6-dihydro-pyrrolo[2,1-a]Isoquinoline-1-carbonitrile
50. 4-(1-Cyano-ethoxy-9-methoxy-3,5-dimethyl-5,8-dihydro-pyrrolo[2,1-a]isoquinolin-2-yl)-benzoic add
51. 2-(4-Hydroxy-2-methyl-phenyl)-8-methoxy-3,5-dimethyl-5,6-dihydro-pyrrolo[2,1-a]isoquinoline-1-carbonitrile
52. 4-(1-Cyano-8,9-dimethoxy-3,5-dimethyl-5,8-dihydro-pyrrolo[2,1-a]isoquinolin-2-yl)-benzamide
53. 8-Elhoxy-2-(4-hydroxy-3,5-dimethyl-phenyl)-9-methoxy-3,5-dimethyl-5,6-dihydro-pyrrolo[2,1-a]isoquinoline-1-carbonitrile
54. 3-(1-Cyano-8,9-dimethoxy-3,5-dimethyl-5,6-dihydro-pyrrolo[2,1-a]isoquinolin-2-yl)-indole-1-sulfonic acid dimethylamide
55. 8,9-Dimethoxy-3,5-dimethyl-2-(2-methyl-1-oxy-pyridin-4-yl)-5,6-dihydro-pyrrolo[2,1-a]isoquinoline-1-carbonitrile
56. 8,9-Dimethoxy-3,5-dimethyl-2-[1-(morpholin-4-sulfonyl)-1H-indol-3-yl]-5,6-dihydropyrrolo[2,1-a]isoquinoline-1-carbonitrile
57. 8,9-Dimethoxy-3,5-dimethyl-2-[4-(2H-tetrazol-5-yl)-phenyl]-5,6-dihydro-pyrrolo[2,1-a]isoquinoline-1-carbonitrile
58. Morpholine-4-sulfonic add [4-(1-cyano-8,9-dimethoxy-3,5-dimethyl-5,6-dihydro-pyrrolo[2,1-a]isoquinolin-2-yl)-phenyl]-amide
59. N-[4-(1-Cyano-8,9-dimethoxy-3,5-dimethyl-5,6-dihydro-pyrrolo[2,1-a]isoquinolin-2-yl)-phenyl]-methanesulfonamide
60. 5-Ethyl-2-(2-fluoro-3,4-dimethoxy-phenyl)-8,9-dimethoxy-3-methyl-5,6-dihydro-pyrrolo[2,1-a]isoquinoline-1-carboxylic add ethyl ester
61. 7-Chloro-8,9-dimethoxy-3,5-dimethyl-2-pyridin-4-yl-5,6-dihydro-pyrrolo[2,1-a]isoquinoline carboxylic acid ethyl ester
62. 7-Chloro-2-(4-hydroxy-3,5-dimethyl-phenyl)-8,9-dimethoxy-3,5-dimethyl-5,6-dihydropyrrolo[2,1-a]Isoquinoline-1-carboxylic acid ethyl ester
63. 7,8,9-Trimethoxy-3,5-dimethyl-2-pyridin-4-yl-5,6-dihydro-pyrrolo[2,1-a]isoquinoline-1-carboxylic add ethyl ester
65. 2-(4-Hydroxy-3,5-dimethyl-phenyl)-8,9-dimethoxy-3,5-dimethyl-5,6-dihydro-pyrrolo[2,1-a]isoquinoline-1-carboxylic acid methyl ester
66. 8,9-Dimethoxy-3,5-dimethyl-2-[1-(toluene-4-Sulfonyl)-1H-indol-3-yl]-5,8-dihydro-pyrrolo[2,1-a]isoquinoline-1-carboxylic add methyl ester
67. 5-Cyano-2-(4-hydroxy-3,5-dimethyl-phenyl)-8,9-dimethoxy-3-methyl-5,6-dihydro-pyrrolo[2,1-a]isoquinoline-1-carboxylic acid ethyl ester
or a salt, stereoisomer, hydrate or hydrate of a salt thereof.

15. A compound according to any of claim 1-14 for use in therapy.

16. Use of a compound according to any of claims 1-14 in the manufacture of pharmaceutical compositions for the treatment of neurologic and/or psychiatric disorders.

17. A pharmaceutical composition comprising as an active ingredient an effective amount of at least one of the compounds according to claim 1 together with suitable pharmaceutical auxiliaries and/or excipients.

## Patentansprüche

1. Verbindungen der Formel I 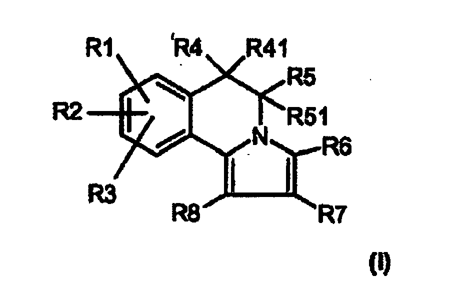 worin
R1 für Halogen, Nitro, Amino, Mono- oder Di-1-4C-alkylamino, 1-4C-Alkyl, Hydroxyl, 1-4C-Alkoxy, 1-4C-Alkoxy-2-4C-alkoxy, 3-7C-Cycloalkoxy, 3-7C-Cycloalkylmethoxy oder vollständig oder überwiegend fluorsubstituiertes 1-4C-Alkoxy steht,
R2 für Wasserstoff, Halogen oder 1-4C-Alkoxy steht und
R3 für Wasserstoff oder 1-4C-Alkoxy steht oder
R2 und R3 in ortho-Stellung zueinander an die Benzoringgruppierung gebunden sind und gemeinsam eine 1-2C-Alkylendioxybrücke bilden oder
R2 und R3 in ortho-Stellung zueinander an die Benzoringgruppierung gebunden sind und gemeinsam eine vollständig oder überwiegend fluorsubstituierte 1-2C-Alkylendioxybrücke bilden oder
R1 und R2 in ortho-Stellung zueinander an die Benzoringgruppierung gebunden sind und gemeinsam eine 1-2C-Alkylendioxybrücke bilden und R3 für Wasserstoff steht oder
R1 und R2 in ortho-Stellung zueinander an die Benzoringgruppierung gebunden sind und gemeinsam eine vollständig oder überwiegend fluorsubstituierte 1-2C-Alkylendioxybrücke bilden und R3 für Wasserstoff steht,
R4 für Wasserstoff, Fluor, Chlor, 1-4C-Alkyl, Trifluormethyl, Cyclopropyl, Cyano, 1-4C-Alkoxycarbonyl oder -CH₂-O-R411 steht, wobei
R411 für Wasserstoff, 1-4C-Alkyl, 1-4C-Alkoxy-2-4C-alkyl oder 1-4C-Alkylcarbonyl steht,
R41 für Wasserstoff oder 1-4C-Alkyl steht,
R5 für Fluor oder 1-4C-Alkyl steht und
R51 für Wasserstoff oder 1-4C-Alkyl steht
oder
R4 für Wasserstoff, Fluor, Chlor oder 1-4C-Alkyl steht,
R41 für Wasserstoff oder 1-4C-Alkyl steht,
R5 für Fluor, 1-4C-Alkyl, Trifluormethyl, Cyclopropyl, Cyano, 1-4C-Alkoxycarbonyl oder -CH₂-O-R511 steht, wobei
R511 für Wasserstoff, 1-4C-Alkyl, 1-4C-Alkoxy-2-4C-alkyl oder 1-4C-Alkylcarbonyl steht und
R51 für Wasserstoff oder 1-4C-Alkyl steht
oder
R4 und R5 gemeinsam eine 1-4C-Alkylenbrücke bilden und R41 und R51 beide für Wasserstoff stehen,
R6 für 1-6C-Alkyl, Amino, Formyl oder durch R61 substituiertes 1-4C-Alkyl steht, wobei
R61 für 1-4C-Alkoxycarbonyl, Carboxyl, 1-4C-Alkoxy, Hydroxyl, Halogen oder -N(R611)R612 steht, wobei
R611 für Wasserstoff, 1-4C-Alkyl, 3-7C-Cycloalkyl oder 3-7C-Cycloalkyl-1-4C-alkyl steht und
R612 für Wasserstoff oder 1-4C-Alkyl steht oder
R611 und R612 gemeinsam und unter Einbeziehung des Stickstoffatoms, an das sie gebunden sind, einen Rest Het1 bilden, wobei
Het1 für einen 5- bis 7-gliedrigen gesättigten heterocylischen Ringrest, der ein Stickstoffatom, an das R611 und R612 gebunden sind, und gegebenenfalls ein weiteres Heteroatom ausgewählt aus der Gruppe bestehend aus Stickstoff, Sauerstoff und Schwefel enthält und gegebenenfalls an einem Ringstickstoffatom durch R613 substituiert ist, steht, wobei
R613 für 1-4C-Alkyl, 3-7C-Cycloalkyl, 3-7C-Cycloalkyl-1-4C-alkyl, Hydroxy-2-4C-alkyl, 1-4C-Alkoxy-2-4C-alkyl, Amino-2-4C-alkyl, Mono- oder Di-1-4C-alkylamino-2-4C-alkyl, Formyl, Pyridyl oder Pyrimidinyl steht,
R7 für Phenyl, Het2, R71- und/oder R72- und/oder R73-substituiertes Phenyl, R74- und/oder R75-substituiertes Het2, Naphthyl oder R76-und/oder R77-substituiertes Naphthyl steht, wobei
Het2 entweder für einen monocyclischen oder kondensierten bicyclischen 5- bis 10-gliedrigen Heteroarylrest mit einem bis drei Heteroatomen, die jeweils aus der Gruppe bestehend aus Stickstoff, Sauerstoff und Schwefel ausgewählt sind,
oder
einen kondensierten bicyclischen 9- oder 10- gliedrigen teilweise gesättigten heterocyclischen Ringrest mit einem Benzolring und mit einem oder zwei Heteroatomen, die jeweils aus der Gruppe bestehend aus Stickstoff, Sauerstoff und Schwefel ausgewählt sind,
oder
N-Oxypyridyl steht,
R71 für Hydroxyl, Halogen, Nitro, Cyano, Trifluormethyl, 1-4C-Alkyl, 1-4C-Alkoxy, Amino, Mono- oder Di-1-4C-alkylamino, 1-4C-Alkylsulfonylamino, Arylsulfonylamino, 1-4C-Alkoxycarbonyl, Carboxyl, 1-4C-Alkylthio, Aryloxy-2-4C-alkoxy, Aryloxy-1-4C-alkyl, Aryloxy, Aryl-1-4C-alkoxy, Aryl, 1-4C-Alkoxy-2-4C-alkoxy, 1-4C-Alkoxy-1-4C-alkyl, Hydroxy-2-4C-alkoxy, Amino-2-4C-alkoxy, Mono- oder Di-1-4C-alkylamino-2-4C-alkoxy, vollständig oder überwiegend fluorsubstituiertes 1-4C-Alkoxy, Mono- oder Di-1-4C-alkylaminocarbonyl, Carbamoyl, Tetrazolyl oder -N(H)S(O)₂-N(R712)R713 steht, wobei
Aryl für Phenyl oder R711-substituiertes Phenyl steht, wobei
R711 für Halogen, 1-4C-Alkyl, 1-4C-Alkoxy, Nitro oder Cyano steht,
R712 für 1-4C-Alkyl steht und
R713 für 1-4C-Alkyl steht oder
R712 und R713 gemeinsam und unter Einbeziehung des Stickstoffatoms, an das sie gebunden sind, einen Rest Het3 bilden, wobei
Het3 für Pyrrolidin-1-yl, Piperidin-1-yl oder Morpholin-4-yl steht,
R72 für Halogen, 1-4C-Alkyl, 1-4C-Alkoxy oder 1-4C-Alkoxycarbonyl steht,
R73 für 1-4C-Alkyl oder 1-4C-Alkoxy steht,
R74 für Halogen, 1-4C-Alkyl, Trifluormethyl, 1-4C-Alkoxy, Cyano, Amino, Mono- oder Di-1-4C-alkylamino, 1-4C-Alkoxycarbonyl, Morpholino, Carboxyl, Nitro, Phenyl, Phenyloxy, Phenyl-1-4C-alkyl, Arylsulfonyl, 1-4C-Alkylsulfonyl oder -S(O)₂-N(R712)R713 steht,
R75 für 1-4C-Alkyl oder Halogen steht,
R76 für Halogen, Hydroxyl, 1-4C-Alkyl, 1-4C-Alkoxy, Carboxyl oder 1-4C-Alkoxycarbonyl steht,
R77 für 1-4C-Alkyl oder 1-4C-Alkoxy steht,
R8 für 1-4C-Alkyl, Phenyl, 2-4C-Alkinyl, Cyano, -CH₂-O-R81, Phenylcarbonyl, -C(O)-N(R82)R83 oder -C(O)-OR9 steht, wobei
R81 für Wasserstoff, 1-4C-Alkyl, 1-4C-Alkoxy-2-4C-alkyl oder 1-4C-Alkylcarbonyl steht,
R82 für Wasserstoff, 1-4C-Alkyl, 3-7C-Cycloalkyl, 3-7C-Cycloalkyl-1-4C-alkyl, Phenyl oder Phenyl-1-4C-alkyl steht und
R83 für Wasserstoff oder 1-4C-Alkyl steht oder
R82 und R83 gemeinsam und unter Einbeziehung des Stickstoffatoms, an das sie gebunden sind, einen heterocyclischen Ringrest ausgewählt aus der aus Pyrrolidinyl, Piperidinyl, Morpholinyl und N-(1-4C-Alkyl)piperazinyl bestehenden Gruppe bilden,
R9 für Wasserstoff oder 1-4C-Alkyl steht, und die Salze, Stereoisomere, Hydrate und Hydrate der Salze dieser Verbindungen.

2. Verbindungen der Formel I nach Anspruch 1,
worin
R1 für Hydroxyl, 1-4C-Alkoxy, 1-4C-Alkoxy-2-4C-alkoxy, 3-7C-Cycloalkoxy, 3-7C-Cycloalkylmethoxy oder vollständig oder überwiegend fluorsubstituiertes 1-4C-Alkoxy steht,
R2 für Wasserstoff, Halogen oder 1-4C-Alkoxy steht und
R3 für 1-4C-Alkoxy steht oder
R2 und R3 in ortho-Stellung zueinander an die Benzoringgruppierung gebunden sind und gemeinsam eine 1-2C-Alkylendioxybrücke bilden oder
R2 und R3 in ortho-Stellung zueinander an die Benzoringgruppierung gebunden sind und gemeinsam eine vollständig oder überwiegend fluorsubstituierte 1-2C-Alkylendioxybrücke bilden oder
R1 und R2 in ortho-Stellung zueinander an die Benzoringgruppierung gebunden sind und gemeinsam eine 1-2C-Alkylendioxybrücke bilden und R3 für Wasserstoff steht oder
R1 und R2 in ortho-Stellung zueinander an die Benzoringgruppierung gebunden sind und gemeinsam eine vollständig oder überwiegend fluorsubstituierte 1-2C-Alkylendioxybrücke bilden und R3 für Wasserstoff steht
und keine der Gruppen R1, R2 und R3 an die 10-Position des Pyrrolo[2.1-a]isochinolinrings gebunden ist,
R4 für Wasserstoff oder 1-4C-Alkyl steht,
R41 für Wasserstoff oder 1-4C-Alkyl steht,
R5 für 1-4C-Alkyl, Cyano oder 1-4C-Alkoxycarbonyl steht, und
R51 für Wasserstoff oder 1-4C-Alkyl steht
oder
R4 und R5 gemeinsam eine 1-4C-Alkylenbrücke bilden und R41 und R51 beide für Wasserstoff stehen,
R6 für 1-6C-Alkyl oder durch R61 substituiertes 1-4C-Alkyl steht, wobei
R61 für 1-4C-Alkoxycarbonyl oder -N(R611)R612 steht, wobei
R611 für 1-4C-Alkyl steht und
R612 für 1-4C-Alkyl steht oder
R611 und R612 gemeinsam und unter Einbeziehung des Stickstoffatoms, an das sie gebunden sind, einen Rest Het1 bilden, wobei
Het1 für Pyrrolidin-1-yl, Piperidin-1-yl, Morpholin-1-yl oder N-(1-4C-Alkyl)piperazinyl steht,
R7 für Het2, R71- und/oder R72- und/oder R73-substituiertes Phenyl, R74-substituiertes Het2 oder Naphthyl steht, wobei
Het2 entweder für
einen monocyclischen oder kondensierten bicyclischen 5- bis 10-gliedrigen Heteroarylrest mit einem bis drei Heteroatomen, die jeweils aus der Gruppe bestehend aus Stickstoff, Sauerstoff und Schwefel ausgewählt sind,
oder
einen kondensierten bicyclischen 9- oder 10- gliedrigen teilweise gesättigten heterocyclischen Ringrest mit einem Benzolring und mit einem oder zwei Heteroatomen, die jeweils aus der Gruppe bestehend aus Stickstoff, Sauerstoff und Schwefel ausgewählt sind,
oder
N-Oxypyridyl steht,
R71 für Hydroxyl, Halogen, Nitro, Cyano, Trifluormethyl, 1-4C-Alkyl, 1-4C-Alkoxy, Amino, Mono- oder Di-1-4C-alkylamino, 1-4C-Alkylsulfonylamino, 1-4C-Alkoxycarbonyl, Carboxyl, Aryloxy, vollständig oder überwiegend fluorsubstituiertes 1-4C-Alkoxy, Mono- oder Di-1-4C-alkylaminocarbonyl, Carbamoyl, Tetrazolyl oder -N(H)S(O)₂-N(R712)R713 steht, wobei
Aryl für Phenyl oder R711-substituiertes Phenyl steht, wobei
R711 für Halogen oder 1-4C-Alkyl steht,
R712 für 1-4C-Alkyl steht und
R713 für 1-4C-Alkyl steht oder
R712 und R713 gemeinsam und unter Einbeziehung des Stickstoffatoms, an das sie gebunden sind, einen Rest Het3 bilden, wobei
Het3 für Pyrrolidin-1-yl, Piperidin-1-yl oder Morpholin-4-yl steht,
R72 für Halogen, 1-4C-Alkyl oder 1-4C-Alkoxy steht,
R73 für 1-4C-Alkyl oder 1-4C-Alkoxy steht,
R74 für 1-4C-Alkyl, Phenyl-1-4C-alkyl, Arylsulfonyl, 1-4C-Alkylsulfonyl oder -S(O)₂-N(R712)R713 steht,
R8 für 1-4C-Alkyl, Cyano oder -C(O)-OR9 steht,
wobei
R9 für Wasserstoff oder 1-4C-Alkyl steht, und die Salze, Stereoisomere, Hydrate und Hydrate der Salze dieser Verbindungen.

3. Verbindungen der Formel I nach Anspruch 1,
worin
R1 an die 8-Position des Pyrrolo[2.1-a]-isochinolinrings gebunden ist und für 1-4C-Alkoxy steht,
R2 an die 7-Position des Pyrrolo[2.1-a]-isochinolinrings gebunden ist und für Wasserstoff, Halogen oder 1-4C-Alkoxy steht,
R3 an die 9-Position des Pyrrolo[2.1-a]-isochinolinrings gebunden ist und für 1-4C-Alkoxy steht,
R4 für Wasserstoff steht,
R41 für Wasserstoff steht,
R5 für 1-4C-Alkyl, Cyano oder 1-4C-Alkoxycarbonyl steht, und
R51 für Wasserstoff oder 1-4C-Alkyl steht
oder
R4 und R5 gemeinsam eine 3-4C-Alkylenbrücke bilden und R41 und R51 beide für Wasserstoff stehen,
R6 für 1-4C-Alkyl oder durch R61 substituiertes 1-4C-Alkyl steht, wobei
R61 für 1-4C-Alkoxycarbonyl oder -N(R611)R612 steht, wobei
R611 und R612 gemeinsam und unter Einbeziehung des Stickstoffatoms, an das sie gebunden sind, einen Rest Het1 bilden, wobei
Het1 für Morpholin-1-yl steht,
R7 für Het2, R71- und/oder R72- und/oder R73- substituiertes Phenyl, R74-substituiertes Het2 oder Naphthyl steht, wobei
Het2 entweder für
einen monocyclischen oder kondensierten bicyclischen 5- bis 10-gliedrigen Heteroarylrest mit einem bis drei Heteroatomen, die jeweils aus der Gruppe bestehend aus Stickstoff, Sauerstoff und Schwefel ausgewählt sind,
oder
einen kondensierten bicyclischen 9- oder 10- gliedrigen teilweise gesättigten heterocyclischen Ringrest mit einem Benzolring und mit einem oder zwei Heteroatomen, die jeweils aus der Gruppe bestehend aus Stickstoff, Sauerstoff und Schwefel ausgewählt sind,
oder
N-Oxypyridyl steht,
R71 für Hydroxyl, Halogen, Nitro, 1-4C-Alkyl, 1-4C-Alkoxy, Amino, Mono- oder Di-1-4C-alkylamino, 1-4C-Alkylsulfonylamino, Carboxyl, Aryloxy, Mono- oder Di-1-4C-alkylaminocarbonyl, Carbamoyl, Tetrazolyl oder -N(H)S(O)₂-N(R712)R steht, wobei
Aryl für Phenyl oder R711-substituiertes Phenyl steht, wobei
R711 für Halogen oder 1-4C-Alkyl steht,
R712 für 1-4C-Alkyl steht und
R713 für 1-4C-Alkyl steht oder
R712 und R713 gemeinsam und unter Einbeziehung des Stickstoffatoms, an das sie gebunden sind, einen Rest Het3 bilden, wobei
Het3 für Morpholin-4-yl steht,
R72 für Halogen, 1-4C-Alkyl oder 1-4C-Alkoxy steht,
R73 für 1-4C-Alkyl oder 1-4C-Alkoxy steht,
R74 für 1-4C-Alkyl, Phenyl-1-4C-alkyl, Arylsulfonyl, 1-4C-Alkylsulfonyl oder -S(O)₂-N(R712)R713 steht,
R8 für 1-4C-Alkyl, Cyano oder -C(O)-OR9 steht,
wobei
R9 für Wasserstoff oder 1-4C-Alkyl steht,
und die Salze, Stereoisomere, Hydrate und Hydrate der Salze dieser Verbindungen.

4. Verbindungen der Formel I nach Anspruch 1,
worin
entweder, in einer ersten unabhängigen Ausführungsform,
R1 an die 8-Position des Pyrrolo[2.1-a]-isochinolinrings gebunden ist und für 1-2C-Alkoxy steht,
R2 an die 7-Position des Pyrrolo[2.1-a]-isochinolinrings gebunden ist und für Wasserstoff, Chlor oder Fluor steht,
R3 an die 9-Position des Pyrrolo[2.1-a]-isochinolinrings gebunden ist und für 1-2C-Alkoxy steht,
R4 für Wasserstoff steht,
R41 für Wasserstoff steht,
R5 für 1-2C-Alkyl oder Cyano steht und
R51 für Wasserstoff steht,
oder
R4 und R5 gemeinsam eine Tetramethylenbrücke bilden und R41 und R51 beide für Wasserstoff stehen,
R6 für 1-2C-Alkyl oder durch R61 substituiertes 1-2C-Alkyl steht, wobei
R61 für 1-2C-Alkoxycarbonyl oder -N(R611)R612 steht, wobei
R611 und R612 gemeinsam und unter Einbeziehung des Stickstoffatoms, an das sie gebunden sind, einen Rest Het1 bilden, wobei
Het1 für Morpholin-1-yl steht,
R7 für Naphthyl, 4-Hydroxy-3,5-dimethylphenyl, 4-Methoxy-3,5-dimethylphenyl, 4-Carboxyphenyl, 4-Carbamoylphenyl, 2-Methyl-4-hydroxyphenyl, 4-Aminophenyl, 4-(2H-Tetrazol-5-yl-phenyl, 4-Morpholinosulfonylaminophenyl, 4-Methylsulfonylaminophenyl oder 2-Fluor-3,4-dimethoxyphenyl,
Pyridyl, Indolyl, Chinolinyl, Indolinyl, 2-Methylpyridin-4-yl, 3-Methylpyridin-4-yl oder
N-(R74)-Het2 steht, wobei
Het2 für Pyrrolyl oder Indolyl steht,
R74 für Arylsulfonyl, 1-2C-Alkylsulfonyl oder -S(O)₂-N(R712)R713 steht, wobei
Aryl für Phenyl oder R711-substituiertes Phenyl steht, wobei
R711 für 1-2C-Alkyl steht,
R712 für 1-2C-Alkyl steht und
R713 für 1-2C-Alkyl steht oder
R712 und R713 gemeinsam und unter Einbeziehung des Stickstoffatoms, an das sie gebunden sind, einen Rest Het3 bilden, wobei
Het3 für Morpholin-4-yl steht und
R8 für Cyano steht;
oder, in einer zweiten unabhängigen Ausführungsform,
R1 an die 8-Position des Pyrrolo[2.1-a]-isochinolinrings gebunden ist und für 1-2C-Alkoxy steht,
R2 an die 7-Position des Pyrrolo[2.1-a]-isochinolinrings gebunden ist und für Wasserstoff, Chlor oder Fluor steht,
R3 an die 9-Position des Pyrrolo[2.1-a]-isochinolinrings gebunden ist und für 1-2C-Alkoxy steht,
R4 für Wasserstoff steht,
R41 für Wasserstoff steht,
R5 für 1-2C-Alkyl oder Cyano steht und
R51 für Wasserstoff steht,
oder
R4 und R5 gemeinsam eine Tetramethylenbrücke bilden und R41 und R51 beide für Wasserstoff stehen,
R6 für 1-2C-Alkyl oder durch R61 substituiertes 1-2C-Alkyl steht, wobei
R61 für 1-2C-Alkoxycarbonyl oder -N(R611)R612 steht, wobei
R611 und R612 gemeinsam und unter Einbeziehung des Stickstoffatoms, an das sie gebunden sind, einen Rest Het1 bilden, wobei
Het1 für Morpholin-1-yl steht,
R7 für Naphthyl, 4-Hydroxy-3,5-dimethylphenyl, 4-Methoxy-3,5-dimethylphenyl, 4-Carboxyphenyl, 4-Carbamoylphenyl, 2-Methyl-4-hydroxyphenyl, 4-Aminophenyl, 4-(2H-Tetrazol-5-yl)-phenyl, 4-Morpholinosulfonylaminophenyl, 4-Methylsulfonylaminophenyl oder 2-Fluor-3,4-dimethoxyphenyl,
Pyridyl, Indolyl, Chinolinyl, Indolinyl, 2-Methylpyridin-4-yl, 3-Methylpyridin-4-yl oder
N-(R74)-Het2 steht, wobei
Het2 für Pyrrolyl oder Indolyl steht,
R74 für Arylsulfonyl, 1-2C-Alkylsulfonyl oder -S(O)₂-N(R712)R713 steht, wobei
Aryl für Phenyl oder R711-substituiertes Phenyl steht, wobei
R711 für 1-2C-Alkyl steht,
R712 für 1-2C-Alkyl steht und
R713 für 1-2C-Alkyl steht oder
R712 und R713 gemeinsam und unter Einbeziehung des Stickstoffatoms, an das sie gebunden sind, einen Rest Het3 bilden, wobei
Het3 für Morpholin-4-yl steht und
R8 für -C(O)-OR9 steht, wobei
R9 für 1-2C-Alkyl steht;
und die Salze, Stereoisomere, Hydrate und Hydrate der Salze dieser Verbindungen.

5. Verbindungen der Formel I nach Anspruch 1,
worin
entweder, in einer ersten unabhängigen Ausführungsform,
R1 an die 8-Position des Pyrrolo[2.1-a]-isochinolinrings gebunden ist und für Methoxy steht,
R2 an die 7-Position des Pyrrolo[2.1-a]-isochinolinrings gebunden ist und für Wasserstoff oder Fluor steht,
R3 an die 9-Position des Pyrrolo[2.1-a]-isochinolinrings gebunden ist und für Methoxy steht,
R4 für Wasserstoff steht,
R41 für Wasserstoff steht,
R5 für Methyl oder Cyano steht,
R51 für Wasserstoff steht,
R6 für Methyl, Ethyl oder 2-Methoxycarbonylethyl steht,
R7 für 4-Hydroxy-3,5-dimethylphenyl, 4-Methoxy-3,5-dimethylphenyl, 4-Carboxyphenyl, 2-Methyl-4-hydroxyphenyl, 4-Aminophenyl, 4-(2H-Tetrazol-5-yl)phenyl, 4-Morpholinosulfonylaminophenyl, 4-Methylsulfonylaminophenyl, Pyridyl, Chinolinyl,
2-Methylpyridin-4-yl, 3-Methylpyridin-4-yl, 1-Tolylsulfonylpyrrol-3-yl, 1-Tolylsulfonylindol-3-yl, 1-Phenylsulfonylindol-3-yl, 1-Methylsulfonylindol-3-yl, 1-Dimethylaminosulfonylindol-3-yl oder 1-Morpholinosulfonylindol-3-yl steht und
R8 für Cyano steht;
oder, in einer zweiten unabhängigen Ausführungsform,
R1 an die 8-Position des Pyrrolo[2.1-a]-isochinolinrings gebunden ist und für Methoxy steht,
R2 an die 7-Position des Pyrrolo[2.1-a]-isochinolinrings gebunden ist und für Wasserstoff oder Fluor steht,
R3 an die 9-Position des Pyrrolo[2.1-a]-isochinolinrings gebunden ist und für Methoxy steht,
R4 für Wasserstoff steht,
R41 für Wasserstoff steht,
R5 für Methyl oder Cyano steht,
R51 für Wasserstoff steht,
R6 für Methyl, Ethyl oder 2-Methoxycarbonylethyl steht,
R7 für 4-Hydroxy-3,5-dimethylphenyl, 4-Methoxy-3,5-dimethylphenyl, 4-Carboxyphenyl, 2-Methyl-4-hydroxyphenyl, 4-Aminophenyl, 4-(2H-Tetrazol-5-yl)phenyl, 4-Morpholinosulfonylaminophenyl, 4-Methylsulfonylaminophenyl, Pyridyl, Chinolinyl, 2-Methylpyridin-4-yl, 3-Methylpyridin-4-yl, 1-Tolylsulfonylpyrrol-3-yl, 1-Tolylsulfonylindol-3-yl, 1-Phenylsulfonylindol-3-yl, 1-Methylsulfonylindol-3-yl, 1-Dimethylaminosulfonylindol-3-yl oder 1-Morpholinosulfonylindol-3-yl steht und
R8 für -C(O)-OR9 steht, wobei
R9 für Methyl oder Ethyl steht;
und die Salze, Stereoisomere, Hydrate und Hydrate der Salze dieser Verbindungen.

6. Verbindungen der Formel I nach Anspruch 1,
worin
R1 an die 8-Position des Pyrrolo[2.1-a]-isochinolinrings gebunden ist und für 1-2C-Alkoxy wie z.B. Methoxy steht,
R2 an die 7-Position des Pyrrolo[2.1-a]-isochinolinrings gebunden ist und für Fluor steht,
R3 an die 9-Position des Pyrrolo[2.1-a]-isochinolinrings gebunden ist und für 1-2C-Alkoxy wie z.B. Methoxy steht,
R4 für Wasserstoff steht,
R41 für Wasserstoff steht,
R5 für Methyl oder Cyano steht,
R51 für Wasserstoff steht,
R6 für Methyl, Ethyl oder 2-Methoxycarbonylethyl steht,
R7 für 4-Hydroxy-3,5-dimethylphenyl, 4-Methoxy-3,5-dimethylphenyl, 4-Carboxyphenyl, 2-Methyl-4-hydroxyphenyl, 4-Aminophenyl, 4-(2H-Tetrazol-5-yl)phenyl, 4-Morpholinosulfonylaminophenyl, 4-Methylsulfonylaminophenyl, Pyridyl, Chinolinyl, 2-Methylpyridin-4-yl, 3-Methylpyridin-4-yl, 1-Tolylsulfonylpyrrol-3-yl, 1-Tolylsulfonylindol-3-yl, 1-Phenylsulfonylindol-3-yl, 1-Methylsulfonylindol-3-yl, 1-Dimethylaminosulfonylindol-3-yl oder 1-Morpholinosulfonylindol-3-yl steht,
R8 für Cyano steht;
und die Salze, Stereoisomere, Hydrate und Hydrate der Salze dieser Verbindungen.

7. Verbindungen der Formel I nach Anspruch 1,
worin
R1 an die 8-Position des Pyrrolo[2.1-a]-isochinolinrings gebunden ist und für Methoxy steht,
R2 an die 7-Position des Pyrrolo[2.1-a]-isochinolinrings gebunden ist und für Fluor steht,
R3 an die 9-Position des Pyrrolo[2.1-a]-isochinolinrings gebunden ist und für Methoxy steht,
R4 für Wasserstoff steht,
R41 für Wasserstoff steht,
R5 für Methyl steht,
R51 für Wasserstoff steht,
R6 für Methyl steht,
R7 für 4-Hydroxy-3,5-dimethylphenyl, 4-Methoxy-3,5-dimethylphenyl, 4-Carboxyphenyl, 2-Methyl-4-hydroxyphenyl, 4-Aminophenyl, 4-(2H-Tetrazol-5-yl)phenyl, 4-Morpholinosulfonylaminophenyl, 4-Methylsulfonylaminophenyl, Pyridyl, Chinolinyl, 2-Methylpyridin-4-yl, 3-Methylpyridin-4-yl, 1-Tolylsulfonylpyrrol-3-yl, 1-Tolylsulfonylindol-3-yl, 1-Phenylsulfonylindol-3-yl, 1-Methylsulfonylindol-3-yl, 1-Dimethylaminosulfonylindol-3-yl oder 1-Morpholinosulfonylindol-3-yl steht,
R8 für Cyano steht;
und die Salze, Stereoisomere, Hydrate und Hydrate der Salze dieser Verbindungen.

8. Verbindungen der Formel I nach Anspruch 1,
worin
R1 für Wasserstoff oder 1-2C-Alkoxy steht,
R2 für Wasserstoff oder 1-2C-Alkoxy steht,
R3 für 1-2C-Alkoxy steht,
R4 für Wasserstoff steht,
R41 für Wasserstoff steht,
R5 für 1-2C-Alkyl steht,
R51 für Wasserstoff steht,
R6 für Methyl, Ethyl oder Methoxycarbonylethyl steht,
R7 für Phenyl, Het2, R71- und/oder R72- und/oder R73-substituiertes Phenyl oder für Naphthyl steht, wobei
Het2 für einen Heteroarylrest ausgewählt aus der Gruppe bestehend aus Furanyl, Thiophenyl, Pyrrolyl, Pyridinyl, Chinolyl, Indolyl, Benzothiophenyl und Benzofuranyl steht,
R71 für Hydroxyl, Chlor, Methoxy, Dimethylamino oder Aryloxy steht, wobei
Aryl für R711-substituiertes Phenyl steht, wobei
R711 für Chlor steht,
R72 für Methyl, tert.-Butyl oder Methoxy steht,
R73 für Methyl, tert.-Butyl oder Methoxy steht,
R8 für Cyano steht,
und die Salze, Stereoisomere, Hydrate und Hydrate der Salze dieser Verbindungen.

9. Verbindungen nach Anspruch 1 mit der Formel Ia
oder Ib 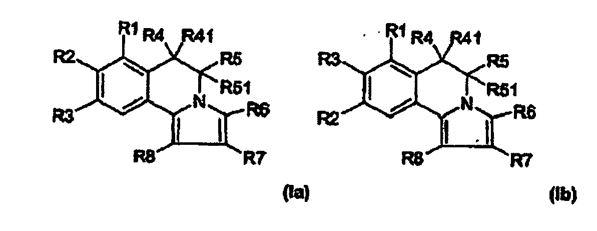 worin,
als erste Alternative,
R1 für Wasserstoff steht,
R2 für Chlor oder Fluor steht,
R3 für Methoxy oder Ethoxy steht,
oder, als zweite Alternative,
R1 für Wasserstoff steht,
R2 für Methoxy oder Ethoxy steht,
R3 für Methoxy oder Ethoxy steht,
oder, als dritte Alternative,
R1 für Methoxy oder Ethoxy steht,
R2 für Chlor oder Fluor steht,
R3 für Methoxy oder Ethoxy steht,
oder, als vierte Alternative,
R1 für Chlor oder Fluor steht,
R2 für Methoxy oder Ethoxy steht,
R3 für Methoxy oder Ethoxy steht,
oder, als fünfte Alternative,
R1 für Methoxy oder Ethoxy steht,
R2 für Methoxy oder Ethoxy steht,
R3 für Methoxy oder Ethoxy steht,
R4 für Wasserstoff steht,
R41 für Wasserstoff steht,
R5 für Methyl steht,
R51 für Wasserstoff steht,
R6 für Methyl, Ethyl oder Methoxycarbonylethyl steht,
R7 für Het2, R75-substituiertes Het2 oder 4-Hydroxy-3,5-dimethylphenyl steht, wobei
Het2 für Pyridinyl oder Chinolinyl steht,
R75 für 1-4C-Alkyl steht,
R8 für Cyano steht,
und die Salze, Stereoisomere, Hydrate und Hydrate der Salze dieser Verbindungen.

10. Verbindungen nach einem der vorhergehenden Ansprüche,
worin
R1 an die 8-Position des Pyrrolo[2.1-a]-isochinolinrings gebunden ist und für 1-2C-Alkoxy wie z.B. Methoxy steht,
R2 an die 7-Position des Pyrrolo[2.1-a]-isochinolinrings gebunden ist und für Wasserstoff, Chlor oder Fluor steht,
R3 an die 9-Position des Pyrrolo[2.1-a]-isochinolinrings gebunden ist und für 1-2C-Alkoxy wie z.B. Methoxy steht,
und
R4 für Wasserstoff steht,
R41 für Wasserstoff steht,
R5 für 1-2C-Alkyl oder Cyano steht,
R51 für Wasserstoff steht,
und
R8 für Cyano steht
und die Salze, Stereoisomere, Hydrate und Hydrate der Salze dieser Verbindungen.

11. Verbindungen nach einem der Ansprüche 1 bis 9,
worin
R1 an die 8-Position des Pyrrolo[2.1-a]-isochinolinrings gebunden ist und für 1-2C-Alkoxy wie z.B. Methoxy steht,
R2 an die 7-Position des Pyrrolo[2.1-a]-isochinolinrings gebunden ist und für Chlor oder Fluor steht,
R3 an die 9-Position des Pyrrolo[2.1-a]-isochinolinrings gebunden ist und für 1-2C-Alkoxy wie z.B. Methoxy steht,
und
R4 für Wasserstoff steht,
R41 für Wasserstoff steht,
R5 für 1-2C-Alkyl oder Cyano steht,
R51 für Wasserstoff steht,
und
R8 für Cyano steht
und die Salze, Stereoisomere, Hydrate und Hydrate der Salze dieser Verbindungen.

12. Verbindungen nach einem der Ansprüche 1 bis 9,
worin die Verbindungen die wie in Anspruch 9 definierte Formel Ia aufweisen, wobei
R2 für Methoxy steht,
R3 für Methoxy steht,
R4 für Wasserstoff steht,
R41 für Wasserstoff steht,
R51 für Wasserstoff steht,
und wobei R1, R5, R6 und R8 eine der in der folgenden Tabelle angeführten Bedeutungen 1.) bis 75.) haben
| | R1 | R5 | R6 | R8 |
|---|---|---|---|---|
| 1.) | Wasserstoff | Methyl | Methyl | Cyano |
| 2.) | Wasserstoff | Methyl | Methyl | Ethoxycarbonyl |
| 3.) | Wasserstoff | Methyl | 2-Methoxycarbonylethyl | Cyano |
| 4.) | Wasserstoff | Methyl | 2-Methoxycarbonylethyl | ethoxycarbonyl |
| 7.) | Fluor | Methyl | Methyl | Cyano |
| 8.) | Fluor | Methyl | Ethyl | Ethoxycarbonyl |
| 9.) | Fluor | Methyl | 2-Methoxycarbonylethyl | Cyano |
| 10.) | Fluor | Methyl | 2-Methoxycarbonylethyl | Ethoxycarbonyl |
| 15.) | Wasserstoff | Cyano | Methyl | Cyano |
| 16.) | Wasserstoff | Cyano | Methyl | Ethoxycarbonyl |
| 17.) | Wasserstoff | Cyano | 2-Methoxycarbonylethyl | Cyano |
| 16.) | Wasserstoff | Cyano | 2.Methoxycarbonylethyl | Ethoxycarbonyl |
| 19. | Fluor | Cyano | Methyl | Cyano |
| 20. | Fluor | Cyano | Methyl | Ethoxycarbonyl |
| 21.) | Fluor | Cyano | 2-Methylcarbonylethyl | Cyano |
| 22.) | Fluor | Cyano | 2-Methoxycarbonylethyl | Ethoxycarbonyl |
| 23.) | Chlor | Methyl | Methyl | Cyano |
| 24.) | Chlor | Methyl | Methyl | Ethoxycarbonyl |
| 24.) | Chlor | Methyl | 2-Methoxycarbonylethyl | Cyano |
| 26.) | Chlor | Methyl | 2-Methoxycarbonylethyl | Ethoxycarbonyl |
| 31.) | Chlor | Cyano | Methyl | Cyano |
| 32.) | Chlor | Cyano | Methyl | Ethoxycarbonyl |
| 33.) | Chlor | Cyano | 2-Methoxycarbonylethyl | Cyano |
| 34.) | Chlor | Cyano | 2-Methoxycarbonylethyl | Ethoxycarbonyl |
| 35.) | Wasserstoff | Methyl | Methyl | Methoxycarbonyl |
| 36.) | Wasserstoff | Methyl | 2-Methoxycarbonylethyl | Methoxycarbonyl |
| 37.) | Fluor | Methyl | Methyl | Methoxycarbonyl |
| 38.) | Fluor | Methyl | 2-Methoxycarbonylethyl | Methoxycarbonyl |
| 41.) | Wasserstoff | Cyano | Methyl | Methoxycarbonyl |
| 42.) | Wasserstoff | Cyano | 2-Methoxycarbonylethyl | Methoxycarbonyl |
| 43.) | Fluor | Cyano | Methyl | Methoxycarbonyl |
| 44.) | Fluor | Cyano | 2-Methoxycarbonylethyl | Methoxycarbonyl |
| 45.) | Chlor | Methyl | Methyl | Methoxycarbonyl |
| 46.) | Chlor | Methyl | 2-Methoxycarbonylethyl | Methoxycarbonyl |
| 49.) | Chlor | Cyano | Methyl | Methoxycarbonyl |
| 50.) | Chlor | Cyano | 2-Methoxycarbonylethyl | Methoxycarbonyl |
| 61.) | Wasserstoff | Methyl | Ethyl | Cyano |
| 52.) | Wasserstoff | Methyl | Ethyl | Ethoxycarbonyl |
| 54.) | Fluor | Methyl | Ethyl | Cyano |
| 55.) | Fluor | Methyl | Ethyl | Ethoxycarbonyl |
| 58.) | Wasserstoff | Cyano | Ethyl | Cyano |
| 59.) | Wasserstoff | Cyano | Ethyl | Ethoxycarbonyl |
| 60.) | Fluor | Cyano | Ethyl | Cyano |
| 61.) | Fluor | Cyano | Ethyl | Ethoxycarbonyl |
| 62.) | Chlor | Methyl | Ethyl | Cyano |
| 63.) | Chlor | Methyl | Ethyl | Ethoxycarbonyl |
| 66.) | Chlor | Cyano | Ethyl | Cyano, |
| 67.) | Chlor | Cyano | Ethyl | Ethoxycarbonyl |
| 68.) | Wasserstoff | Methyl | Ethyl | Methoxycarbonyl |
| 69.) | Fluor | Methyl | Ethyl | Methoxycarbonyl |
| 71.) | Wasserstoff | Cyano | Ethyl | Methoxycarbonyl |
| 72.) | Fluor | Cyano | Ethyl | Methoxycarbonyl |
| 73.) | Chlor | Methyl | Ethyl | Methoxycarbonyl |
| 75.) | Chlor | Cyano | Ethyl | Methoxycarbonyl |
oder die Salze, Stereoisomere, Hydrate und Hydrate der Salze dieser Verbindungen.

13. Verbindungen nach Anspruch 1, ausgewählt aus der Gruppe bestehend aus
1. 2-(4-Hydroxy-3,5-dimethylphenyl)-8,9-dimethoxy-3,5,5-trimethyl-5,6-dihydropyrrolo[2,1-a]-isochinolin-1-carbonsäureethylester
2. 8,9-Dimethoxy-3,5,5-trimethyl-2-(3,4,5-trimethoxyphenyl)-5,6-dihydropyrrolo[2,1-a]-isochinolin-1-carbonsäureethylester
3. 2-[3-(4-Chlorphenoxy)phenyl]-8,9-dimethoxy-3,5,5-trimethyl-5,6-dihydropyrrolo[2,1-a]isochinolin-1-carbonsäureethylester
4. 2-(3-Dimethylaminophenyl)-8,9-dimethoxy-3,5,5-trimethyl-5,6-dihydropyrrolo[2,1-a]isochinolin-1-carbonsäureethylester
5. (5RS)-(4-Hydroxy-3,5-dimethylphenyl)-8,9-dimethoxy-3,5-dimethyl-5,6-dihydropyrrolo[2,1-a]-isochinolin-1-carbonsäureethylester
6. (5RS)-5-Ethyl-2-(4-hydroxy-3,5-dimethylphenyl)-8,9-dimethoxy-3-methyl-5,6-dihydropyrrolo[2,1-a]-isochinolin-1-carbonsäureethylester
7. (5RS)-2-Chlor-5-ethyl-8,9-dimethoxy-3-methyl-5,6-dihydropyrrolo[2,1-a]isochinolin-1-carbonsäureethylester
8. (4aRS,8aRS)-cis-2-(4-Hydroxy-3,5-dimethylphenyl)-10,11-dimethoxy-3-methyl-4a,5,6,7,8,8a-hexahydropyrrolo[2,1-f]phenanthridin-1-carbonsäureethylester
9. (5RS)-3-Ethyl-2-(4-hydroxy-3,5-dimethylphenyl)-8,9-dimethoxy-5-methyl-5,6-dihydropyrrolo[2,1-a]-isochinolin-1-carbonsäureethylester
10. (5RS)-8,9-Dimethoxy-3,5-dimethyl-2-(3,4,5-trimethoxyphenyl)-5,6-dihydropyrrolo[2,1-a]-isochinolin-1-carbonsäureethylester
11. (5RS)-8,9-Dimethoxy-3,5-dimethyl-2-naphthalin-1-yl-5,6-dihydropyrrolo[2,1-a]isochinolin-1-carbonsäureethylester
12. (4aRS,8aRS)-cis-10,11-Dimethoxy-3-methyl-2-naphthalin-1-yl-4a,5,6,7,8,8a-hexahydropyrrolo-[2,1-f]phenanthridin-1-carbonsäureethylester
13. (4aRS,8aRS)-cis-10,11-Dimethoxy-3-methyl-2-chinolin-4-yl-4a,5,6,7,8,8a-hexahydropyrrolo[2,1-f]-phenanthridin-1-carbonsäureethylester
14. (4aR,8aR)-10,11-Dimethoxy-3-methyl-2-chinolin-4-yl-4a,5,6,7,8,8a-hexahydropyrrolo[2,1-f]phenanthridin-1-carbonsäureethylester
15. (4aR,8aR)-10,11-Dimethoxy-3-methyl-2-naphthalin-1-yl-4a,5,6,7,8,8a-hexahydropyrrolo[2,1-f]phenanthridin-1-carbonsäureethylester
16. (4aR,8aR)-2-(4-Hydroxy-3,5-dimethylphenyl)-10,11-dimethoxy-3-methyl-4a,5,6,7,8,8a-hexahydropyrrolo-[2,1-f]phenanthridin-1-carbonsäureethylester
17. (5RS)-5-Ethyl-8,9-dimethoxy-3-methyl-2-naphthalin-1-yl-5,6-dihydropyrrolo[2,1-a]isochinolin-1-carbonsäureethylester
18. (5RS)-2-(4-Hydroxy-3,5-dimethylphenyl)-7,8,9-trimethoxy-3,5-dimethyl-5,6-dihydropyrrolo[2,1-a]-isochinolin-1-carbonsäureethylester
19. 2-(4-Hydroxy-3,5-dimethylphenyl)-8,9-dimethoxy-5,6-dihydropyrrolo[2,1-a]isochinolin-1,5-dicarbonsäure-1-ethyl-5-methylester
20. (5RS)-8,9-Dimethoxy-3-(2-methoxycarbonylethyl)-5-methyl-2-naphthalin-1-yl-5,6-dihydropyrrolo[2,1-a]isochinolin-1-carbonsäureethylester
26. 8,9-Dimethoxy-3,5-dimethyl-2-pyridin-4-yl-5,6-dihydropyrrolo[2,1-a]isochinolin-1-carbonitril
27. 3-[1-Cyano-2-(4-hydroxy-3,5-dimethyl)-8,9-dimethoxy-5-methyl-5,6-dihydropyrrolo[2,1-a]-isochinolin-3-yl]propionsäuremethylester
28. 2-(4-Hydroxy-3,5-dimethylphenyl)-8,9-dimethoxy-3,5-dimethyl-5,6-dihydropyrrolo[2,1-a]isochinolin-1-carbonitril
und die Salze, Stereoisomere, Hydrate und Hydrate der Salze davon.

14. Verbindungen nach Anspruch 1, ausgewählt aus der Gruppe bestehend aus
1. 2-(4-Hydroxy-3,5-dimethylphenyl)-8,9-dimethoxy-3,5,5-trimethyl-5,6-dihydropyrrolo[2,1-a]-isochinolin-1-carbonsäureethylester
2. 8,9-Dimethoxy-3,5,5-trimethyl-2-(3,4,5-trimethoxyphenyl)-5,6-dihydro-pyrrolo[2,1-a]-isochinolin-1-carbonsäureethylester
3. 2-[3-(4-Chlorphenoxy)phenyl]-8,9-dimethoxy-3,5,5-trimethyl-5,6-dihydropyrrolo[2,1-a]isochinolin-1-carbonsäureethylester
4. 2-(3-Dimethylaminophenyl)-8,9-dimethoxy-3,5,5-trimethyl-5,6-dihydropyrrolo[2,1-a]isochinolin-1-carbonsäureethylester
5. (5RS)-(4-Hydroxy-3,5-dimethylphenyl)-8,9-dimethoxy-3,5-dimethyl-5,6-dihydropyrrolo[2,1-a]-isochinolin-1-carbonsäureethylester
6. (5RS)-5-Ethyl-2-(4-hydroxy-3,5-dimethylphenyl)-8,9-dimethoxy-3-methyl-5,6-dihydropyrrolo[2,1-a]-isochinolin-1-carbonsäureethylester
7. (5RS)-2-Chlor-5-ethyl-8,9-dimethoxy-3-methyl-5,6-dihydropyrrolo[2,1-a]isochinolin-1-carbonsäureethylester
8. (4aRS,8aRS)-cis-2-(4-Hydroxy-3,5-dimethylphenyl)-10,11-dimethoxy-3-methyl-4a,5,6,7,8,8a-hexahydropyrrolo[2,1-f]phenanthridin-1-carbonsäureethylester
9. (5RS)-3-Ethyl-2-(4-hydroxy-3,5-dimethylphenyl)-8,9-dimethoxy-5-methyl-5,6-dihydropyrrolo[2,1-a]-isochinolin-1-carbonsäureethylester
10. (5RS)-8,9-Dimethoxy-3,5-dimethyl-2-(3,4,5-trimethoxyphenyl)-5,6-dihydropyrrolo[2,1-a]-isochinolin-1-carbonsäureethylester
11. (5RS)-8,9-Dimethoxy-3,5-dimethyl-2-naphthalin-1-yl-5,6-dihydropyrrolo[2,1-a]isochinolin-1-carbonsäureethylester
12. (4aRS,8aRS)-cis-10,11-Dimethoxy-3-methyl-2-naphthalin-1-yl-4a,5,6,7,8,8a-hexahydropyrrolo-[2,1-f]phenanthridin-1-carbonsäureethylester
13. (4aRS,8aRS)-cis-10,11-Dimethoxy-3-methyl-2-chinolin-4-yl-4a,5,6,7,8,8a-hexahydropyrrolo[2,1-f]-phenanthridin-1-carbonsäureethylester
14. (4aR,8aR)-10,11-Dimethoxy-3-methyl-2-chinolin-4-yl-4a,5,6,7,8,8a-hexahydropyrrolo[2,1-f]phenanthridin-1-carbonsäureethylester
15. (4aR,8aR)-10,11-Dimethoxy-3-methyl-2-naphthalin-1-yl-4a,5,6,7,8,8a-hexahydropyrrolo[2,1-f]phenanthridin-1-carbonsäureethylester
16. (4aR,8aR)-2-(4-Hydroxy-3,5-dimethylphenyl)-10,11-dimethoxy-3-methyl-4a,5,6,7,8,8a-hexahydropyrrolo-[2,1-f]phenanthridin-1-carbonsäureethylester
17. (5RS)-5-Ethyl-8,9-dimethoxy-3-methyl-2-naphthalin-1-yl-5,6-dihydropyrrolo[2,1-a]isochinolin-1-carbonsäureethylester
18. (5RS)-2-(4-Hydroxy-3,5-dimethylphenyl)-7,8,9-trimethoxy-3,5-dimethyl-5,6-dihydropyrrolo[2,1-a]-isochinolin-1-carbonsäureethylester
19. 2-(4-Hydroxy-3,5-dimethylphenyl)-8,9-dimethoxy-5,6-dihydropyrrolo[2,1-a]isochinolin-1,5-dicarbonsäure-1-ethyl-5-methylester
20. (5RS)-8,9-Dimethoxy-3-(2-methoxycarbonylethyl)-5-methyl-2-naphthalin-1-yl-5,6-dihydropyrrolo[2,1-a]isochinolin-1-carbonsäureethylester
26. 8,9-Dimethoxy-3,5-dimethyl-2-pyridin-4-yl-5,6-dihydropyrrolo[2,1-a]isochinolin-1-carbonitril
27. 3-[1-Cyano-2-(4-hydroxy-3,5-dimethyl)-8,9-dimethoxy-5-methyl-5,6-dihydropyrrolo[2,1-a]-isochinolin-3-yl]propionsäuremethylester
28. 2-(4-Hydroxy-3,5-dimethylphenyl)-8,9-dimethoxy-3,5-dimethyl-5,6-dihydropyrrolo[2,1-a]isochinolin-1-carbonitril
30. 7-Fluor-2-(4-hydroxy-3,5-dimethylphenyl)-8,9-dimethoxy-3,5-dimethyl-5,6-dihydropyrrolo[2,1-a]-isochinolin-1-carbonitril
33. 8,9-Dimethoxy-2-(4-methoxy-3,5-dimethylphenyl)-3,5-dimethyl-5,6-dihydropyrrolo[2,1-a]isochinolin-1-carbonitril
37. 8,9-Dimethoxy-3,5-dimethyl-2-[1-(toluol-4-sulfonyl)-1H-pyrrol-3-yl]-5,6-dihydropyrrolo[2,1-a]-isochinolin-1-carbonitril
38. 8,9-Dimethoxy-3,5-dimethyl-2-[1-(toluol-4-sulfonyl)-1H-indol-3-yl]-5,6-dihydropyrrolo[2,1-a] - isochinolin-1-carbonitril
39. 2-(1-Benzolsulfonyl-1H-indol-3-yl)-8,9-dimethoxy-3,5-dimethyl-5,6-dihydropyrrolo[2,1-a]isochinolin-1-carbonitril
40. 2-(1-Methansulfonyl-1H-indol-3-yl)-8,9-dimethoxy-3,5-dimethyl-5,6-dihydropyrrolo[2,1-a]isochinolin-1-carbonitril
41. 8,9-Dimethoxy-3,5-dimethyl-2-(1-oxypyridin-4-yl)-5,6-dihydropyrrolo[2,1-a]isochinolin-1-carbonitril
42. 7-Fluor-8,9-dimethoxy-3,5-dimethyl-2-[1-(toluol-4-sulfonyl)-1H-indol-3-yl]-5,6-dihydropyrrolo[2,1-a]isochinolin-1-carbonitril
44. 2-(4-Hydroxy-3,5-dimethylphenyl)-8,9-dimethoxy-5-methyl-3-morpholin-4-ylmethyl-5,6-dihydropyrrolo-[2,1-a]isochinolin-1-carbonitril
45. 8,9-Dimethoxy-3,5-dimethyl-2-(2-methyl-pyridin-4-yl)-5,6-dihydropyrrolo[2,1-a]isochinolin-1-carbonitril
46. 8,9-Dimethoxy-3,5-dimethyl-2-(4-nitrophenyl)-5,6-dihydropyrrolo[2,1-a]isochinolin-1-carbonitril
47. 4-(1-Cyano-8,9-dimethoxy-3,5-dimethyl-5,6-dihydropyrrolo[2,1-a]isochinolin-2-yl)benzoesäure
48. 2-(4-Aminophenyl)-8,9-dimethoxy-3,5-dimethyl-5,6-dihydropyrrolo[2,1-a]isochinolin-1-carbonitril
49. 8,9-Dimethoxy-3,5-dimethyl-2-(3-methylpyridin-4-yl)-5,6-dihydropyrrolo[2,1-a]isochinolin-1-carbonitril
50. 4-(1-Cyano-8-ethoxy-9-methoxy-3,5-dimethyl-5,6-dihydropyrrolo[2,1-a]isochinolin-2-yl)benzoesäure
51. 2-(4-Hydroxy-2-methylphenyl)-8,9-dimethoxy-3,5-dimethyl-5,6-dihydropyrrolo[2,1-a]isochinolin-1-carbonitril
52. 4-(1-Cyano-8,9-dimethoxy-3,5-dimethyl-5,6-dihydropyrrolo[2,1-a]isochinolin-2-yl)benzamid
53. 8-Ethoxy-2-(4-hydroxy-3,5-dimethylphenyl)-9-methoxy-3,5-dimethyl-5,6-dihydropyrrolo[2,1-a]-isochinolin-1-carbonitril
54. 3-(1-Cyano-8,9-dimethoxy-3,5-dimethyl-5,6-dihydropyrrolo[2,1-a]isochinolin-2-yl)indol-1-schwefelsäuredimethylamid
55. 8,9-Dimethoxy-3,5-dimethyl-2-(2-methyl-1-oxypyridin-4-yl)-5,6-dihydropyrrolo[2,1-a]isochinolin-1-carbonitril
56. 8,9-Dimethoxy-3,5-dimethyl-2-[1-(morpholin-4-sulfonyl)-1H-indol-3-yl]-5,6-dihydropyrrolo[2,1-a]isochinolin-1-carbonitril
57. 8,9-Dimethoxy-3,5-dimethyl-2-[4-(2H-tetrazol-5-yl)phenyl]-5,6-dihydropyrrolo[2,1-a]isochinolin-1-carbonitril
58. Morpholin-4-sulfonsäure-[4-(1-cyano-8,9-dimethoxy-3,5-dimethyl-5,6-dihydropyrrolo[2,1-a]isochinolin-2-yl)phenyl]amid
59. N-[4-(1-Cyano-8,9-dimethoxy-3,5-dimethyl-5,6-dihydropyrrolo[2,1-a]isochinolin-2-yl)phenyl]-methansulfonamid
60. 5-Ethyl-2-(2-fluor-3,4-dimethoxyphenyl)-8,9-dimethoxy-3-methyl-5,6-dihydropyrrolo[2,1-a]-isochinolin-1-carbonsäureethylester
61. 7-Chlor-8,9-dimethoxy-3,5-dimethyl-2-pyridin-4-yl-5,6-dihydropyrrolo[2,1-a]isochinolin-1-carbonsäureethylester
62. 7-Chlor-2-(4-hydroxy-3,5-dimethylphenyl)-8,9-dimethoxy-3,5-dimethyl-5,6-dihydropyrrolo[2,1-a]-isochinolin-1-carbonsäureethylester
63. 7,8,9-Trimethoxy-3,5-dimethyl-2-pyridin-4-yl-5,6-dihydropyrrolo[2,1-a]isochinolin-1-carbonsäureethylester
65. 2-(4-Hydroxy-3,5-dimethylphenyl)-8,9-dimethoxy-3,5-dimethyl-5,6-dihydropyrrolo[2,1-a]isochinolin-1-carbonsäuremethylester
66. 8,9-Dimethoxy-3,5-dimethyl-2-[1-(toluol-4-sulfonyl)-1H-indol-3-yl]-5,6-dihydropyrrolo[2,1-a]-isochinolin-1-carbonsäuremethylester
67. 5-Cyano-2-(4-hydroxy-3,5-dimethyl-phenyl)-8,9-dimethoxy-3-methyl-5,6-dihydropyrrolo[2,1-a]-isochinolin-1-carbonsäureethylester und die Salze, Stereoisomere, Hydrate und Hydrate der Salze davon.

15. Verbindung nach einem der Ansprüche 1 bis 14 zur Verwendung in der Therapie.

16. Verwendung einer Verbindung nach einem der Ansprüche 1 bis 14 zur Herstellung von pharmazeutischen Zusammensetzungen zur Behandlung von neurologischen und/oder psychiatrischen Störungen.

17. Pharmazeutische Zusammensetzung, die als Wirkstoff eine wirksame Menge mindestens einer der Verbindungen nach Anspruch 1 zusammen mit geeigneten pharmazeutischen Hilfsstoffen und/oder Trägerstoffen enthält.

## Revendications

1. Composés de formule I 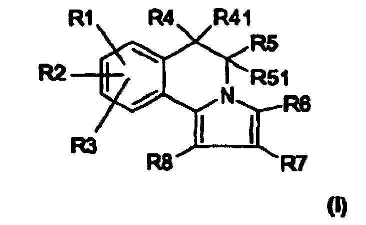 dans laquelle :
R1 représente halogène, nitro, amino, mono-1-4C-alkylamino ou di-1-4C-alkylamino, 1-4C-alkyle, hydroxyle, 1-4C-alcoxy, 1-4C-alcoxy-2-4C-alcoxy, 3-7C-cycloalcoxy, 3-7C-cycloalkylméthoxy, ou 1-4C-alcoxy totalement ou de manière prédominante substitué par fluor,
R2 représente hydrogène, halogène ou 1-4C-alcoxy, et
R3 représente hydrogène ou 1-4C-alcoxy, ou
R2 et R3 liés au fragment avec le cycle benzo en position ortho l'un par rapport à l'autre forment ensemble un pont 1-2C-alkylènedioxy, ou
R2 et R3 liés au fragment avec le cycle benzo en position ortho l'un par rapport à l'autre forment ensemble un pont 1-2C-alkylènedioxy totalement ou de manière prédominante substitué par fluor, ou
R1 et R2 liés au fragment avec le cycle benzo en position ortho l'un par rapport à l'autre forment ensemble un pont 1-2C-alkylènedioxy et R3 représente hydrogène, ou
R1 et R2 liés au fragment avec le cycle benzo en position ortho l'un par rapport à l'autre forment ensemble un pont 1-2C-alkylènedioxy totalement ou de manière prédominante substitué par fluor et R3 représente hydrogène,
R4 représente hydrogène, fluor, chlore, 1-4C-alkyle, trifluorométhyle, cyclopropyle, cyano, 1-4C-alcoxycarbonyle ou -CH₂-O-R411, où
R411 représente hydrogène, 1-4C-alkyle, 1-4C-alcoxy-2-4C-alkyle ou 1-4C-alkylcarbonyle,
R41 représente hydrogène ou 1-4C-alkyle,
R5 représente fluor ou 1-4C-alkyle, et
R51 représente hydrogène ou 1-4C-alkyle,
ou
R4 représente hydrogène, fluor, chlore ou 1-4C-alkyle,
R41 représente hydrogène ou 1-4C-alkyle,
R5 représente fluor, 1-4C-alkyle, trifluorométhyle, cyclopropyle, cyano, 1-4C-alcoxycarbonyle ou -CH₂-O-R511, où
R511 représente hydrogène, 1-4C-alkyle, 1-4C-alcoxy-2-4C-alkyle ou 1-4C-alkylcarbonyle, et
R51 représente hydrogène ou 1-4C-alkyle,
ou
R4 et R5 forment ensemble un pont 1-4C-alkylène et R41 et R51 représentent tous les deux hydrogène,
R6 représente 1-6C-alkyle, amino, formyle, ou 1-4C-alkyle substitué par R61, où
R61 représente 1-4C-alcoxycarbonyle, carboxyle, 1-4C-alcoxy, hydroxyle, halogène ou -N(R611)R612, où
R611 représente hydrogène, 1-4C-alkyle, 3-7C-cycloalkyle ou 3-7C-cycloalkyl-1-4C-alkyle, et
R612 représente hydrogène ou 1-4C-alkyle, ou
R611 et R612 ensemble et en incluant l'atome d'azote auquel ils sont liés forment un radical Het1, dans lequel
Het1 représente un radical hétérocyclique saturé de 5 à 7 chaînons, comprenant un atome d'azote, auquel R611 et R612 sont liés et, éventuellement, un autre hétéroatome choisi dans un groupe constitué par azote, oxygène et soufre et éventuellement substitué par R613 sur un atome d'azote du cycle,
où
R613 représente 1-4C-alkyle, 3-7C-cycloalkyle, 3-7C-cycloalkyl-1-4C-alkyle, hydroxy-2-4C-alkyle, 1-4C-alcoxy-2-4C-alkyle, amino-2-4C-alkyle, mono-1-4C-alkylamino-2-4C-alkyle ou di-1-4C-alkylamino-2-4C-alkyle, formyle, pyridyle ou pyrimidinyle,
R7 représente phényle, Het2, phényle substitué par R71 et/ou R72 et/ou R73, Het2 substitué par R74 et/ou R75, naphtyle, ou naphtyle substitué par R76 et/ou R77, où
Het2 représente
un radical hétéroaryle de 5 à 10 chaînons, monocyclique ou bicyclique condensé comprenant un à trois hétéroatomes, dont chacun est choisi dans un groupe constitué par azote, oxygène et soufre,
ou
un radical hétérocyclique partiellement saturé, bicyclique condensé, de 9 ou 10 chaînons contenant un cycle benzène et comprenant un ou deux hétéroatomes, dont chacun est choisi dans un groupe constitué par azote, oxygène et soufre,
ou
N-oxypyridyle,
R71 représente hydroxyle, halogène, nitro, cyano,
trifluorométhyle, 1-4C-alkyle, 1-4C-alcoxy, amino, mono-1-4C-alkylamino ou di-1-4C-alkylamino, 1-4C-alkylsulfonylamino, arylsulfonylamino, 1-4C-alcoxycarbonyle, carboxyle, 1-4C-alkylthio, aryloxy-2-4C-alcoxy, aryloxy-1-4C-alkyle, aryloxy, aryl-1-4C-alcoxy, aryle, 1-4C-alcoxy-2-4C-alcoxy, 1-4C-alcoxy-1-4C-alkyle, hydroxy-2-4C-alcoxy, amino-2-4C-alcoxy, mono-1-4C-alkylamino-2-4C-alcoxy ou di-1-4C-alkylamino-2-4C-alcoxy, 1-4C-alcoxy complètement ou de manière prédominante substitué par fluor, mono-1-4C-alkylaminocarbonyle
ou di-1-4C-alkylaminocarbonyle, carbamoyle, tétrazolyle, ou -N(H)S(O)₂-N(R712)R713, où aryle représente phényle ou phényle substitué par
R711, où
R711 représente halogène, 1-4C-alkyle, 1-4C-alcoxy, nitro ou cyano,
R712 représente 1-4C-alkyle, et
R713 représente 1-4C-alkyle, ou
R712 et R713 ensemble et en incluant l'atome d'azote auquel ils sont liés forment un radical Het3, dans lequel
Het3 représente pyrrolidin-1-yle, pipéridin-1-yle ou morpholin-4-yle,
R72 représente halogène, 1-4C-alkyle, 1-4C-alcoxy ou 1-4C-alcoxycarbonyle,
R73 représente 1-4C-alkyle ou 1-4C-alcoxy,
R74 représente halogène, 1-4C-alkyle, trifluorométhyle, 1-4C-alcoxy, cyano, amino, mono-1-4C-alkylamino ou di-1-4C-alkylamino, 1-4C-alcoxycarbonyle, morpholino, carboxyle, nitro, phényle, phényloxy, phényl-1-4C-alkyle, arylsulfonyle, 1-4C-alkylsulfonyle, ou -S(O)₂-N(R712)R713,
R75 représente 1-4C-alkyle ou halogène,
R76 représente halogène, hydroxyle, 1-4C-alkyle, 1-4C-alcoxy, carboxyle ou 1-4C-alcoxycarbonyle,
R77 représente 1-4C-alkyle ou 1-4C-alcoxy,
R8 représente 1-4C-alkyle, phényle, 2-4C-alcynyle, cyano, -CH₂-O-R81, phénylcarbonyle, -C(O)-N(R82)R83 ou -C(O)-OR9, où
R81 représente hydrogène, 1-4C-alkyle, 1-4C-alcoxy-2-4C-alkyle ou 1-4C-alkylcarbonyle,
R82 représente hydrogène, 1-4C-alkyle, 3-7C-cycloalkyle, 3-7C-cycloalkyl-1-4C-alkyle, phényle ou phényl-1-4C-alkyle, et
R83 représente hydrogène ou 1-4C-alkyle, ou
R82 et R83 ensemble et en incluant l'atome d'azote auquel ils sont liés forment un radical hétérocyclique choisi dans le groupe constitué par pyrrolidinyle, pipéridinyle, morpholinyle ou N-(1-4C-alkyl)-pipérazinyle,
R9 représente hydrogène ou 1-4C-alkyle ;
et les sels, stéréo-isomères, hydrates et hydrates des sels de ces composés.

2. Composés de formule I selon la revendication 1,
où
R1 représente hydroxyle, 1-4C-alcoxy, 1-4C-alcoxy-2-4C-alcoxy, 3-7C-cycloalcoxy, 3-7C-cycloalkylméthoxy, ou 1-4C-alcoxy complètement ou de manière prédominante substitué par fluor,
R2 représente hydrogène, halogène ou 1-4C-alcoxy, et
R3 représente 1-4C-alcoxy, ou
R2 et R3 liés au fragment avec le cycle benzo en position ortho l'un par rapport à l'autre forment ensemble un pont 1-2C-alkylènedioxy, ou
R2 et R3 liés au fragment avec le cycle benzo en position ortho l'un par rapport à l'autre forment ensemble un pont 1-2C-alkylènedioxy totalement ou de manière prédominante substitué par fluor, ou
R1 et R2 liés au fragment avec le cycle benzo en position ortho l'un par rapport à l'autre forment ensemble un pont 1-2C-alkylènedioxy et R3 représente hydrogène, ou
R1 et R2 liés au fragment avec le cycle benzo en position ortho l'un par rapport à l'autre forment ensemble un pont 1-2C-alkylènedioxy totalement ou de manière prédominante substitué par fluor et R3 représente hydrogène,
et aucun radical parmi R1, R2 et R3 n'est lié en 10ème position du cycle pyrrolo[2,1-a]isoquinoléine,
R4 représente hydrogène ou 1-4C-alkyle,
R41 représente hydrogène ou 1-4C-alkyle,
R5 représente 1-4C-alkyle, cyano ou 1-4C-alcoxycarbonyle, et
R51 représente hydrogène ou 1-4C-alkyle,
ou
R4 et R5 forment ensemble un pont 1-4C-alkylène et R41 et R51 représentent tous les deux hydrogène,
R6 représente 1-6C-alkyle, ou 1-4C-alkyle substitué par R61, où
R61 représente 1-4C-alcoxycarbonyle ou -N(R611)R612, où
R611 représente 1-4C-alkyle, et
R612 représente 1-4C-alkyle, ou
R611 et R612 ensemble et en incluant l'atome d'azote auquel ils sont liés forment un radical Het1, dans lequel
Het1 représente pyrrolidin-1-yle, pipéridin-1-yle, morpholin-1-yle, ou N-(1-4C-alkyl)-pipérazinyle,
R7 représente Het2, phényle substitué par R71 et/ou R72 et/ou R73, Het2 substitué par R74, ou naphtyle, où
Het2 représente
un radical hétéroaryle de 5 à 10 chaînons, monocyclique ou bicyclique condensé comprenant un à trois hétéroatomes, dont chacun est choisi dans un groupe constitué par azote, oxygène et soufre, ou
un radical hétérocyclique partiellement saturé, bicyclique condensé, de 9 ou 10 chaînons contenant un cycle benzène et comprenant un ou deux hétéroatomes, dont chacun est choisi dans un groupe constitué par azote, oxygène et soufre,
ou
N-oxypyridyle,
R71 représente hydroxyle, halogène, nitro, cyano, trifluorométhyle, 1-4C-alkyle, 1-4C-alcoxy, amino, mono-1-4C-alkylamino ou di-1-4C-alkylamino, 1-4C-alkylsulfonylamino, 1-4C-alcoxycarbonyle, carboxyle, aryloxy, 1-4C-alcoxy complètement ou de manière prédominante substitué par fluor, mono-1-4C-alkylaminocarbonyle ou di-1-4C-alkylaminocarbonyle, carbamoyle, tétrazolyle, ou -N(H)S(O)₂-N(R712)R713, où
aryle représente phényle ou phényle substitué par
R711, où
R711 représente halogène ou 1-4C-alkyle,
R712 représente 1-4C-alkyle, et
R713 représente 1-4C-alkyle, ou
R712 et R713 ensemble et en incluant l'atome d'azote auquel ils sont liés forment un radical Het3, dans lequel
Het3 représente pyrrolidin-1-yle, pipéridin-1-yle ou morpholin-4-yle,
R72 représente halogène, 1-4C-alkyle ou 1-4C-alcoxy,
R73 représente 1-4C-alkyle ou 1-4C-alcoxy,
R74 représente 1-4C-alkyle, phényl-1-4C-alkyle, arylsulfonyle, 1-4C-alkylsulfonyle, ou -S(O)₂-N(R712)R713,
R8 représente 1-4C-alkyle, cyano, ou -C(O)-OR9, où
R9 représente hydrogène ou 1-4C-alkyle ;
et les sels, stéréo-isomères, hydrates et hydrates des sels de ces composés.

3. Composés de formule I selon la revendication 1,
où
R1 est lié en 8ème position du cycle pyrrolo[2,1-a]isoquinoléine et représente 1-4C-alcoxy,
R2 est lié en 7ème position du cycle pyrrolo[2,1-a]isoquinoléine, et représente hydrogène, halogène ou 1-4C-alcoxy,
R3 est lié en 9ème position du cycle pyrrolo[2,1-a]isoquinoléine et représente 1-4C-alcoxy,
R4 représente hydrogène,
R41 représente hydrogène,
R5 représente 1-4C-alkyle, cyano ou 1-4C-alcoxycarbonyle, et
R51 représente hydrogène ou 1-4C-alkyle,
ou
R4 et R5 forment ensemble un pont 3-4C-alkylène et R41 et R51 représentent tous les deux hydrogène,
R6 représente 1-4C-alkyle, ou 1-4C-alkyle substitué par R61, où
R61 représente 1-4C-alcoxycarbonyle ou -N(R611)R612,
où
R611 et R612 ensemble et en incluant l'atome d'azote auquel ils sont liés forment un radical Het1, dans lequel
Het1 représente morpholin-1-yle,
R7 représente Het2, phényle substitué par R71 et/ou R72 et/ou R73, Het2 substitué par R74, ou naphtyle, où
Het2 représente
un radical hétéroaryle de 5 à 10 chaînons, monocyclique ou bicyclique condensé comprenant un à trois hétéroatomes, dont chacun est choisi dans un groupe constitué par azote, oxygène et soufre, ou
un radical hétérocyclique partiellement saturé, bicyclique condensé, de 9 ou 10 chaînons contenant un cycle benzène et comprenant un ou deux hétéroatomes, dont chacun est choisi dans un groupe constitué par azote, oxygène et soufre,
ou
N-oxypyridyle,
R71 représente hydroxyle, halogène, nitro, 1-4C-alkyle, 1-4C-alcoxy, amino, mono-1-4C-alkylamino ou di-1-4C-alkylamino, 1-4C-alkylsulfonylamino, carboxyle, aryloxy, mono-1-4C-alkylaminocarbonyle ou di-1-4C-alkylaminocarbonyle, carbamoyle, tétrazolyle, ou -N(H)S(O)₂-N(R712)R713, où
aryle représente phényle ou phényle substitué par
R711, où
R711 représente halogène ou 1-4C-alkyle,
R712 représente 1-4C-alkyle, et
R713 représente 1-4C-alkyle, ou
R712 et R713 ensemble et en incluant l'atome d'azote auquel ils sont liés forment un radical Het3, dans lequel
Het3 représente morpholin-4-yle,
R72 représente halogène, 1-4C-alkyle ou 1-4C-alcoxy,
R73 représente 1-4C-alkyle ou 1-4C-alcoxy,
R74 représente 1-4C-alkyle, phényl-1-4C-alkyle, arylsulfonyle, 1-4C-alkylsulfonyle, ou -S(O)₂-N(R712)R713,
R8 représente 1-4C-alkyle, cyano, ou -C(O)-OR9, où
R9 représente hydrogène ou 1-4C-alkyle ;
et les sels, stéréo-isomères, hydrates et hydrates des sels de ces composés.

4. Composés de formule I selon la revendication 1,
où
soit, dans une première forme de réalisation indépendante,
R1 est lié en 8ème position du cycle pyrrolo[2,1-a]isoquinoléine et représente 1-2C-alcoxy,
R2 est lié en 7ème position du cycle pyrrolo[2,1-a]isoquinoléine, et représente hydrogène, chlore ou fluor,
R3 est lié en 9ème position du cycle pyrrolo[2,1-a]isoquinoléine et représente 1-2C-alcoxy,
R4 représente hydrogène,
R41 représente hydrogène,
R5 représente 1-2C-alkyle ou cyano, et
R51 représente hydrogène,
ou
R4 et R5 forment ensemble un pont tétraméthylène et R41 et R51 représentent tous les deux hydrogène,
R6 représente 1-2C-alkyle, ou 1-2C-alkyle substitué par R61, où
R61 représente 1-2C-alcoxycarbonyle ou -N(R611)R612, où
R611 et R612 ensemble et en incluant l'atome d'azote auquel ils sont liés forment un radical Het1, dans lequel
Het1 représente morpholin-1-yle,
R7 représente naphtyle, 4-hydroxy-3,5-diméthylphényle, 4-méthoxy-3,5-diméthylphényle, 4-carboxyphényle, 4-carbamoylphényle, 2-méthyl-4-hydroxyphényle, 4-aminophényle, 4-(2H-tétrazol-5-yl)-phényle, 4-morpholinosulfonylaminophényle, 4-méthylsulfonylaminophényle, ou 2-fluoro-3,4-diméthoxyphényle,
pyridyle, indolyle, quinoléinyle, indolinyle, 2-méthylpyridin-4-yle, 3-méthylpyridin-4-yle, ou N-(R74)-Het2, où
Het2 représente pyrrolyle ou indolyle,
R74 représente arylsulfonyle, 1-2C-alkylsulfonyle, ou -S(O)₂-N(R712)R713, où
aryle représente phényle ou phényle substitué par
R711, où
R711 représente 1-2C-alkyle,
R712 représente 1-2C-alkyle, et
R713 représente 1-2C-alkyle, ou
R712 et R713 ensemble et en incluant l'atome d'azote auquel ils sont liés forment un radical Het3, dans lequel
Het3 représente morpholin-4-yle, et
R8 représente cyano ;
soit, dans une deuxième forme de réalisation indépendante,
R1 est lié en 8ème position du cycle pyrrolo[2,1-a]isoquinoléine et représente 1-2C-alcoxy,
R2 est lié en 7ème position du cycle pyrrolo[2,1-a]isoquinoléine, et représente hydrogène, chlore ou fluor,
R3 est lié en 9ème position du cycle pyrrolo[2,1-a]isoquinoléine et représente 1-2C-alcoxy,
R4 représente hydrogène,
R41 représente hydrogène,
R5 représente 1-2C-alkyle ou cyano, et
R51 représente hydrogène,
ou
R4 et R5 forment ensemble un pont tétraméthylène et R41 et R51 représentent tous les deux hydrogène,
R6 représente 1-2C-alkyle, ou 1-2C-alkyle substitué par R61, où
R61 représente 1-2C-alcoxycarbonyle ou -N(R611)R612, où
R611 et R612 ensemble et en incluant l'atome d'azote auquel ils sont liés forment un radical Het1, dans lequel
Het1 représente morpholin-1-yle,
R7 représente naphtyle, 4-hydroxy-3,5-diméthylphényle, 4-méthoxy-3,5-diméthylphényle, 4-carboxyphényle, 4-carbamoylphényle, 2-méthyl-4-hydroxyphényle, 4-aminophényle, 4-(2H-tétrazol-5-yl)-phényle, 4-morpholinosulfonylaminophényle, 4-méthylsulfonylaminophényle, ou 2-fluoro-3,4-diméthoxyphényle,
pyridyle, indolyle, quinoléinyle, indolinyle, 2-méthylpyridin-4-yle, 3-méthylpyridin-4-yle, ou N-(R74)-Het2, où
Het2 représente pyrrolyle ou indolyle,
R74 représente arylsulfonyle, 1-2C-alkylsulfonyle, ou -S(O)₂-N(R712)R713, où
aryle représente phényle ou phényle substitué par
R711, où
R711 représente 1-2C-alkyle,
R712 représente 1-2C-alkyle, et
R713 représente 1-2C-alkyle, ou
R712 et R713 ensemble et en incluant l'atome d'azote auquel ils sont liés forment un radical Het3, dans lequel
Het3 représente morpholin-4-yle, et
R8 représente -C(O)-OR9, où
R9 représente 1-2C-alkyle ;
et les sels, stéréo-isomères, hydrates et hydrates des sels de ces composés.

5. Composés de formule I selon la revendication 1, où :
soit, dans une première forme de réalisation indépendante,
R1 est lié en 8ème position du cycle pyrrolo[2,1-a]isoquinoléine et représente méthoxy,
R2 est lié en 7ème position du cycle pyrrolo[2,1-a]isoquinoléine, et représente hydrogène ou fluor, R3 est lié en 9ème position du cycle pyrrolo[2,1-a]isoquinoléine et représente méthoxy,
R4 représente hydrogène,
R41 représente hydrogène,
R5 représente méthyle ou cyano,
R51 représente hydrogène,
R6 représente méthyle, éthyle ou 2-méthoxycarbonyléthyle,
R7 représente 4-hydroxy-3,5-diméthylphényle, 4-méthoxy-3,5-diméthylphényle, 4-carboxyphényle, 2-méthyl-4-hydroxyphényle, 4-aminophényle, 4-(2H-tétrazol-5-yl)-phényle, 4-morpholinosulfonylaminophényle, 4-méthylsulfonylaminophényle,
pyridyle, quinoléinyle,
2-méthylpyridin-4-yle, 3-méthylpyridin-4-yle, 1-tolylsulfonylpyrrol-3-yle, 1-tolylsulfonylindol-3-yle, 1-phénylsulfonylindol-3-yle, 1-méthylsulfonylindol-3-yle, 1-diméthylaminosulfonylindol-3-yle, ou 1-morpholinosulfonylindol-3-yle, et
R8 représente cyano ;
soit, dans une deuxième forme de réalisation indépendante,
R1 est lié en 8ème position du cycle pyrrolo[2,1-a]isoquinoléine et représente méthoxy,
R2 est lié en 7ème position du cycle pyrrolo[2,1-a]isoquinoléine, et représente hydrogène ou fluor,
R3 est lié en 9ème position du cycle pyrrolo[2,1-a]isoquinoléine et représente méthoxy,
R4 représente hydrogène,
R41 représente hydrogène,
R5 représente méthyle ou cyano,
R51 représente hydrogène,
R6 représente méthyle, éthyle ou 2-méthoxycarbonyléthyle,
R7 représente 4-hydroxy-3,5-diméthylphényle, 4-méthoxy-3,5-diméthylphényle, 4-carboxyphényle, 2-méthyl-4-hydroxyphényle, 4-aminophényle, 4-(2H-tétrazol-5-yl)-phényle, 4-morpholinosulfonylaminophényle, 4-méthylsulfonylaminophényle,
pyridyle, quinoléinyle,
2-méthylpyridin-4-yle, 3-méthylpyridin-4-yle,
1-tolylsulfonylpyrrol-3-yle, 1-tolylsulfonylindol-3-yle, 1-phénylsulfonylindol-3-yle, 1-méthylsulfonylindol-3-yle, 1-diméthylaminosulfonylindol-3-yle, ou 1-morpholinosulfonylindol-3-yle, et
R8 représente -C(O)-OR9, où
R9 représente méthyle ou éthyle ;
et les sels, stéréo-isomères, hydrates et hydrates des sels de ces composés.

6. Composés de formule I selon la revendication 1,
où
R1 est lié en 8ème position du cycle pyrrolo[2,1-a]isoquinoléine et représente 1-2C-alcoxy, tel que par exemple méthoxy,
R2 est lié en 7ème position du cycle pyrrolo[2,1-a]isoquinoléine, et représente fluor,
R3 est lié en 9ème position du cycle pyrrolo[2,1-a]isoquinoléine et représente 1-2C-alcoxy, tel que par exemple méthoxy,
R4 représente hydrogène,
R41 représente hydrogène,
R5 représente méthyle ou cyano,
R51 représente hydrogène,
R6 représente méthyle, éthyle ou 2-méthoxycarbonyléthyle,
R7 représente 4-hydroxy-3,5-diméthylphényle, 4-méthoxy-3,5-diméthylphényle, 4-carboxyphényle, 2-méthyl-4-hydroxyphényle, 4-aminophényle, 4-(2H-tétrazol-5-yl)-phényle, 4-morpholinosulfonylaminophényle, 4-méthylsulfonylaminophényle, pyridyle, quinoléinyle, 2-méthylpyridin-4-yle, 3-méthylpyridin-4-yle, 1-tolylsulfonylpyrrol-3-yle, 1-tolylsulfonylindol-3-yle, 1-phénylsulfonylindol-3-yle, 1-méthylsulfonylindol-3-yle, 1-diméthylaminosulfonylindol-3-yle, ou 1-morpholinosulfonylindol-3-yle,
R8 représente cyano ;
et les sels, stéréo-isomères, hydrates et hydrates des sels de ces composés.

7. Composés de formule I selon la revendication 1,
où
R1 est lié en 8ème position du cycle pyrrolo[2,1-a]isoquinoléine et représente méthoxy,
R2 est lié en 7ème position du cycle pyrrolo[2,1-a]isoquinoléine, et représente fluor,
R3 est lié en 9ème position du cycle pyrrolo[2,1-a]isoquinoléine et représente méthoxy,
R4 représente hydrogène,
R41 représente hydrogène,
R5 représente méthyle,
R51 représente hydrogène,
R6 représente méthyle,
R7 représente 4-hydroxy-3,5-diméthylphényle, 4-méthoxy-3,5-diméthylphényle, 4-carboxyphényle, 2-méthyl-4-hydroxyphényle, 4-aminophényle, 4-(2H-tétrazol-5-yl)-phényle, 4-morpholinosulfonylaminophényle, 4-méthylsulfonylaminophényle,
pyridyle, quinoléinyle,
2-méthylpyridin-4-yle, 3-méthylpyridin-4-yle, 1-tolylsulfonylpyrrol-3-yle, 1-tolylsulfonylindol-3-yle, 1-phénylsulfonylindol-3-yle, 1-méthylsulfonylindol-3-yle, 1-diméthylaminosulfonylindol-3-yle, ou 1-morpholinosulfonylindol-3-yle,
R8 représente cyano ;
et les sels, stéréo-isomères, hydrates et hydrates des sels de ces composés.

8. Composés de formule I selon la revendication 1, où :
R1 représente halogène ou 1-2C-alcoxy,
R2 représente hydrogène ou 1-2C-alcoxy,
R3 représente 1-2C-alcoxy,
R4 représente hydrogène,
R41 représente hydrogène,
R5 représente 1-2C-alkyle,
R51 représente hydrogène,
R6 représente méthyle, éthyle ou méthoxycarbonyléthyle,
R7 représente phényle, Het2, phényle substitué par R71 et/ou R72 et/ou R73, ou naphtyle, où
Het2 représente un radical hétéroaryle choisi dans le groupe constitué par furannyle, thiophényle, pyrrolyle, pyridinyle, quinoléyle, indolyle, benzothiophényle et benzofurannyle,
R71 représente hydroxyle, chlore, méthoxy, diméthylamino, ou aryloxy, où
aryle représente phényle substitué par R711, où
R711 représente chlore,
R72 représente méthyle, tert-butyle ou méthoxy,
R73 représente méthyle, tert-butyle ou méthoxy,
R8 représente cyano,
et les sels, stéréo-isomères, hydrates et hydrates des sels de ces composés.

9. Composés selon la revendication 1, qui sont de formule Ia ou Ib 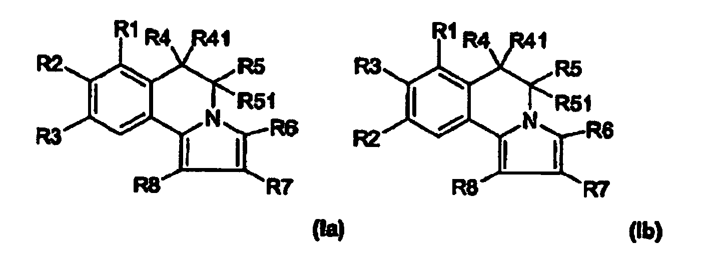 où
comme première alternative,
R1 représente hydrogène,
R2 représente chlore ou fluor,
R3 représente méthoxy ou éthoxy,
ou, comme deuxième alternative,
R1 représente hydrogène,
R2 représente méthoxy ou éthoxy,
R3 représente méthoxy ou éthoxy,
ou, comme troisième alternative,
R1 représente méthoxy ou éthoxy,
R2 représente chlore ou fluor,
R3 représente méthoxy ou éthoxy,
ou, comme quatrième alternative,
R1 représente chlore ou fluor,
R2 représente méthoxy ou éthoxy,
R3 représente méthoxy ou éthoxy,
ou, comme cinquième alternative,
R1 représente méthoxy ou éthoxy,
R2 représente méthoxy ou éthoxy,
R3 représente méthoxy ou éthoxy,
R4 représente hydrogène,
R41 représente hydrogène,
R5 représente méthyle,
R51 représente hydrogène,
R6 représente méthyle, éthyle ou méthoxycarbonyléthyle,
R7 représente Het2, Het2 substitué par R75, ou 4-hydroxy-3,5-diméthylphényle, où
Het2 représente pyridinyle ou quinoléinyle,
R75 représente 1-4C-alkyle,
R8 représente cyano,
et les sels, stéréo-isomères, hydrates et hydrates des sels de ces composés.

10. Composés selon l'une quelconque des revendications précédentes,
où
R1 est lié en 8ème position du cycle pyrrolo[2,1-a]isoquinoléine et représente 1-2C-alcoxy, tel que par exemple méthoxy,
R2 est lié en 7ème position du cycle pyrrolo[2,1-a]isoquinoléine, et représente hydrogène, chlore ou fluor,
R3 est lié en 9ème position du cycle pyrrolo[2,1-a]isoquinoléine et représente 1-2C-alcoxy, tel que par exemple méthoxy,
et
R4 représente hydrogène,
R41 représente hydrogène,
R5 représente 1-2C-alkyle ou cyano,
R51 représente hydrogène, et
R8 représente cyano,
et les sels, stéréo-isomères, hydrates et hydrates des sels de ces composés.

11. Composés selon l'une quelconque des revendications 1 à 9,
où
R1 est lié en 8ème position du cycle pyrrolo[2,1-a]isoquinoléine et représente 1-2C-alcoxy, tel que par exemple méthoxy,
R2 est lié en 7ème position du cycle pyrrolo[2,1-a]isoquinoléine, et représente chlore ou fluor,
R3 est lié en 9ème position du cycle pyrrolo[2,1-a]isoquinoléine et représente 1-2C-alcoxy, tel que par exemple méthoxy,
et
R4 représente hydrogène,
R41 représente hydrogène,
R5 représente 1-2C-alkyle ou cyano,
R51 représente hydrogène, et
R8 représente cyano,
et les sels, stéréo-isomères, hydrates et hydrates des sels de ces composés.

12. Composé selon l'une quelconque des revendications 1 à 9,
où ledit composé est de formule Ia telle que définie dans la revendication 9, où
R2 représente méthoxy,
R3 représente méthoxy,
R4 représente hydrogène,
R41 représente hydrogène,
R51 représente hydrogène,
et où R1, R5, R6 et R8 présentent l'une quelconque des significations 1.) à 75.) spécifiées dans le tableau suivant :
| | R1 | R5 | R6 | R8 |
|---|---|---|---|---|
| 1.) | hydrogène | méthyle | méthyle | cyano |
| 2.) | hydrogène | méthyle | méthyle | éthoxycarbonyle |
| 3.) | hydrogène | méthyle | 2-méthoxycarbonyléthyle | cyano |
| 4.) | hydrogène | méthyle | 2-méthoxycarbonyléthyle | éthoxycarbonyle |
| 7.) | fluor | méthyle | méthyle | cyano |
| 8.) | fluor | méthyle | méthyle | éthoxycarbonyle |
| 9.) | fluor | méthyle | 2-méthoxycarbonyléthyle | cyano |
| 10.) | fluor | méthyle | 2-méthoxycarbonyléthyle | éthoxycarbonyle |
| 15.) | hydrogène | cyano | méthyle | cyano |
| 16.) | hydrogène | cyano | méthyle | éthoxycarbonyle |
| 17.) | hydrogène | cyano | 2-méthoxycarbonyléthyle | cyano |
| 18.) | hydrogène | cyano | 2-méthoxycarbonyléthyle | éthoxycarbonyle |
| 19.) | fluor | cyano | méthyle | cyano |
| 20.) | fluor | cyano | méthyle | éthoxycarbonyle |
| 21.) | fluor | cyano | 2-méthoxycarbonyléthyle | cyano |
| 22.) | fluor | cyano | 2-méthoxycarbonyléthyle | éthoxycarbonyle |
| 23.) | chlore | méthyle | méthyle | cyano |
| 24.) | chlore | méthyle | méthyle | éthoxycarbonyle |
| 25.) | chlore | méthyle | 2-méthoxycarbonyléthyle | cyano |
| 26.) | chlore | méthyle | 2-méthoxycarbonyléthyle | éthoxycarbonyle |
| 31.) | chlore | cyano | méthyle | cyano |
| 32.) | chlore | cyano | méthyle | éthoxycarbonyle |
| 33.) | chlore | cyano | 2-méthoxycarbonyléthyle | cyano |
| 34.) | chlore | cyano | 2-méthoxycarbonyléthyle | éthoxycarbonyle |
| 35.) | hydrogène | méthyle | méthyle | méthoxycarbonyle |
| 36.) | hydrogène | méthyle | 2-méthoxycarbonyléthyle | méthoxycarbonyle |
| 37.) | fluor | méthyle | méthyle | méthoxycarbonyle |
| 38.) | fluor | méthyle | 2-méthoxycarbonyléthyle | méthoxycarbonyle |
| 41.) | hydrogène | cyano | méthyle | méthoxycarbonyle |
| 42.) | hydrogène | cyano | 2-méthoxycarbonyléthyle | méthoxycarbonyle |
| 43.) | fluor | cyano | méthyle | méthoxycarbonyle |
| 44.) | fluor | cyano | 2-méthoxycarbonyléthyle | méthoxycarbonyle |
| 45.) | chlore | méthyle | méthyle | méthoxycarbonyle |
| 46.) | chlore | méthyle | 2-méthoxycarbonyléthyle | méthoxycarbonyle |
| 49.) | chlore | cyano | méthyle | méthoxycarbonyle |
| 50.) | chlore | cyano | 2-méthoxycarbonyléthyle | méthoxycarbonyle |
| 51.) | hydrogène | méthyle | éthyle | cyano |
| 52.) | hydrogène | méthyle | éthyle | éthoxycarbonyle |
| 54.) | fluor | méthyle | éthyle | cyano |
| 55.) | fluor | méthyle | éthyle | éthoxycarbonyle |
| 58.) | hydrogène | cyano | éthyle | cyano |
| 59.) | hydrogène | cyano | éthyle | éthoxycarbonyle |
| 60.) | fluor | cyano | éthyle | cyano |
| 61.) | fluor | cyano | éthyle | éthoxycarbonyle |
| 62.) | chlore | méthyle | éthyle | cyano |
| 63.) | chlore | méthyle | éthyle | éthoxycarbonyle |
| 66.) | chlore | cyano | éthyle | cyano |
| 67.) | chlore | cyano | éthyle | éthoxycarbonyle |
| 68.) | hydrogène | méthyle | éthyle | méthoxycarbonyle |
| 69.) | fluor | méthyle | éthyle | méthoxycarbonyle |
| 71.) | hydrogène | cyano | éthyle | méthoxycarbonyle |
| 72.) | fluor | cyano | éthyle | méthoxycarbonyle |
| 73.) | chlore | méthyle | éthyle | méthoxycarbonyle |
| 75.) | chlore | cyano | éthyle | méthoxycarbonyle |
ou un sel, stéréo-isomère, hydrate ou hydrate d'un sel de ce composé.

13. Composé selon la revendication 1, qui est choisi dans le groupe constitué par :
1. ester éthylique de l'acide 2-(4-hydroxy-3,5-diméthylphényl)-8,9-diméthoxy-3,5,5-triméthyl-5,6-dihydropyrrolo[2,1-a]isoquinoléine-1-carboxylique
2. ester éthylique de l'acide 8,9-diméthoxy-3,5,5-triméthyl-2-(3,4,5-triméthoxyphényl)-5,6-dihydropyrrolo[2,1-a]isoquinoléine-1-carboxylique
3. ester éthylique de l'acide 2-[3-(4-chlorophénoxy)-phényl]-8,9-diméthoxy-3,5,5-triméthyl-5,6-dihydropyrrolo[2,1-a]isoquinoléine-1-carboxylique
4. ester éthylique de l'acide 2-(3-diméthylaminophényl)-8,9-diméthoxy-3,5,5-triméthyl-5,6-dihydropyrrolo[2,1-a]isoquinoléine-1-carboxylique
5. ester éthylique de l'acide (5RS)-(4-hydroxy-3,5-diméthylphényl)-8,9-diméthoxy-3,5-diméthyl-5,6-dihydropyrrolo[2,1-a]isoquinoléine-1-carboxylique
6. ester éthylique de l'acide (5RS)-5-éthyl-2-(4-hydroxy-3,5-diméthylphényl)-8,9-diméthoxy-3-méthyl-5,6-dihydropyrrolo[2,1-a]isoquinoléine-1-carboxylique
7. ester éthylique de l'acide (5RS)-2-chloro-5-éthyl-8,9-diméthoxy-3-méthyl-5,6-dihydropyrrolo[2,1-a]isoquinoléine-1-carboxylique
8. ester éthylique de l'acide (4aRS,8aRS)-cis-2-(4-hydroxy-3,5-diméthylphényl)-10,11-diméthoxy-3-méthyl-4a,5,6,7,8,8a-hexahydropyrrolo[2,1-f]phénanthridine-1-carboxylique
9. ester éthylique de l'acide (5RS)-3-éthyl-2-(4-hydroxy-3,5-diméthylphényl)-8,9-diméthoxy-5-méthyl-5,6-dihydropyrrolo[2,1-a]isoquinoléine-1-carboxylique
10. ester éthylique de l'acide (5RS)-8,9-diméthoxy-3,5-diméthyl-2-(3,4,5-triméthoxyphényl)-5,6-dihydropyrrolo[2,1-a]isoquinoléine-1-carboxylique
11. ester éthylique de l'acide (5RS)-8,9-diméthoxy-3,5-diméthyl-2-naphtalén-1-yl-5,6-dihydropyrrolo[2,1-a]isoquinoléine-1-carboxylique
12. ester éthylique de l'acide (4aRS,8aRS)-cis-10,11-diméthoxy-3-méthyl-2-naphtalén-1-yl-4a,5,6,7,8,8a-hexahydropyrrolo[2,1-f]phénanthridine-1-carboxylique
13. ester éthylique de l'acide (4aRS,8aRS)-cis-10,11-diméthoxy-3-méthyl-2-quinoléin-4-yl-4a,5,6,7,8,8a-hexahydropyrrolo[2,1-f]phénanthridine-1-carboxylique
14. ester éthylique de l'acide (4aR,8aR)-10,11-diméthoxy-3-méthyl-2-quinoléin-4-yl-4a,5,6,7,8,8a-hexahydropyrrolo[2,1-f]phénanthridine-1-carboxylique
15. ester éthylique de l'acide (4aR,8aR)-10,11-diméthoxy-3-méthyl-2-naphtalén-1-yl-4a,5,6,7,8,8a-hexahydropyrrolo[2,1-f]phénanthridine-1-carboxylique
16. ester éthylique de l'acide (4aR,8aR)-2-(4-hydroxy-3,5-diméthylphényl)-10,11-diméthoxy-3-méthyl-4a,5,6,7,8,8a-hexahydropyrrolo[2,1-f]phénanthridine-1-carboxylique
17. ester éthylique de l'acide (5RS)-5-éthyl-8,9-diméthoxy-3-méthyl-2-naphtalén-1-yl-5,6-dihydropyrrolo[2,1-a]isoquinoléine-1-carboxylique
18. ester éthylique de l'acide (5RS)-2-(4-hydroxy-3,5-diméthylphényl)-7,8,9-triméthoxy-3,5-diméthyl-5,6-dihydropyrrolo[2,1-a]isoquinoléine-1-carboxylique
19. ester 1-éthylique 5-méthylique de l'acide 2-(4-hydroxy-3,5-diméthylphényl)-8,9-diméthoxy-5,6-dihydropyrrolo[2,1-a]isoquinoléine-1,5-dicarboxylique
20. ester éthylique de l'acide (5RS)-8,9-diméthoxy-3-(2-méthoxycarbonyléthyl)-5-méthyl-2-naphtalén-1-yl-5,6-dihydropyrrolo[2,1-a]isoquinoléine-1-carboxylique
26. 8,9-diméthoxy-3,5-diméthyl-2-pyridin-4-yl-5,6-dihydropyrrolo[2,1-a]isoquinoléine-1-carbonitrile
27. ester méthylique de l'acide 3-[1-cyano-2-(4-hydroxy-3,5-diméthyl)-8,9-diméthoxy-5-méthyl-5,6-dihydropyrrolo[2,1-a]isoquinoléin3-yl]-propionique
28. 2-(4-hydroxy-3,5-diméthylphényl)-8,9-diméthoxy-3,5-diméthyl-5,6-dihydropyrrolo[2,1-a]isoquinoléine-1-carbonitrile
ou un sel, stéréo-isomère, hydrate ou hydrate d'un sel de ceux-ci.

14. Composé selon la revendication 1, qui est choisi dans le groupe constitué par :
1. ester éthylique de l'acide 2-(4-hydroxy-3,5-diméthylphényl)-8,9-diméthoxy-3,5,5-triméthyl-5,6-dihydropyrrolo[2,1-a]isoquinoléine-1-carboxylique
2. ester éthylique de l'acide 8,9-diméthoxy-3,5,5-triméthyl-2-(3,4,5-triméthoxyphényl)-5,6-dihydropyrrolo[2,1-a]isoquinoléine-1-carboxylique
3. ester éthylique de l'acide 2-[3-(4-chlorophénoxy)-phényl]-8,9-diméthoxy-3,5,5-triméthyl-5,6-dihydropyrrolo[2,1-a]isoquinoléine-1-carboxylique
4. ester éthylique de l'acide 2-(3-diméthylaminophényl)-8,9-diméthoxy-3,5,5-triméthyl-5,6-dihydropyrrolo[2,1-a]isoquinoléine-1-carboxylique
5. ester éthylique de l'acide (5RS)-(4-hydroxy-3,5-diméthylphényl)-8,9-diméthoxy-3,5-diméthyl-5,6-dihydropyrrolo[2,1-a]isoquinoléine-1-carboxylique
6. ester éthylique de l'acide (5RS)-5-éthyl-2-(4-hydroxy-3,5-diméthylphényl)-8,9-diméthoxy-3-méthyl-5,6-dihydropyrrolo[2,1-a]isoquinoléine-1-carboxylique
7. ester éthylique de l'acide (5RS)-2-chloro-5-éthyl-8,9-diméthoxy-3-méthyl-5,6-dihydropyrrolo[2,1-a]isoquinoléine-1-carboxylique
8. ester éthylique de l'acide (4aRS,8aRS)-cis-2-(4-hydroxy-3,5-diméthylphényl)-10,11-diméthoxy-3-méthyl-4a,5,6,7,8,8a-hexahydropyrrolo[2,1-f]phénanthridine-1-carboxylique
9. ester éthylique de l'acide (5RS)-3-éthyl-2-(4-hydroxy-3,5-diméthylphényl)-8,9-diméthoxy-5-méthyl-5,6-dihydropyrrolo[2,1-a]isoquinoléine-1-carboxylique
10. ester éthylique de l'acide (5RS)-8,9-diméthoxy-3,5-diméthyl-2-(3,4,5-triméthoxyphényl)-5,6-dihydropyrrolo[2,1-a]isoquinoléine-1-carboxylique
11. ester éthylique de l'acide (5RS)-8,9-diméthoxy-3,5-diméthyl-2-naphtalén-1-yl-5,6-dihydropyrrolo[2,1-a]isoquinoléine-1-carboxylique
12. ester éthylique de l'acide (4aRS,8aRS)-cis-10,11-diméthoxy-3-méthyl-2-naphtalén-1-yl-4a,5,6,7,8,8a-hexahydropyrrolo[2,1-f]phénanthridine-1-carboxylique
13. ester éthylique de l'acide (4aRS,8aRS)-cis-10,11-diméthoxy-3-méthyl-2-quinoléin-4-yl-4a,5,6,7,8,8a-hexahydropyrrolo[2,1-f]phénanthridine-1-carboxylique
14. ester éthylique de l'acide (4aR,8aR)-10,11-diméthoxy-3-méthyl-2-quinoléin-4-yl-4a,5,6,7,8,8a-hexahydropyrrolo[2,1-f]phénanthridine-1-carboxylique
15. ester éthylique de l'acide (4aR,8aR)-10,11-diméthoxy-3-méthyl-2-naphtalén-1-yl-4a,5,6,7,8,8a-hexahydropyrrolo[2,1-f]phénanthridine-1-carboxylique
16. ester éthylique de l'acide (4aR,8aR)-2-(4-hydroxy-3,5-diméthylphényl)-10,11-diméthoxy-3-méthyl-4a,5,6,7,8,8a-hexahydropyrrolo[2,1-f]phénanthridine-1-carboxylique
17. ester éthylique de l'acide (5RS)-5-éthyl-8,9-diméthoxy-3-méthyl-2-naphtalén-1-yl-5,6-dihydropyrrolo[2,1-a]isoquinoléine-1-carboxylique
18. ester éthylique de l'acide (5RS)-2-(4-hydroxy-3,5-diméthylphényl)-7,8,9-triméthoxy-3,5-diméthyl-5,6-dihydropyrrolo[2,1-a]isoquinoléine-1-carboxylique
19. ester 1-éthylique 5-méthylique de l'acide 2-(4-hydroxy-3,5-diméthylphényl)-8,9-diméthoxy-5,6-dihydropyrrolo-[2,1-a]isoquinoléine-1,5-dicarboxylique
20. ester éthylique de l'acide (5RS)-8,9-diméthoxy-3-(2-méthoxycarbonyléthyl)-5-méthyl-2-naphtalén-1-yl-5,6-dihydropyrrolo[2,1-a]isoquinoléine-1-carboxylique
26. 8,9-diméthoxy-3,5-diméthyl-2-pyridin-4-yl-5,6-dihydropyrrolo[2,1-a]isoquinoléine-1-carbonitrile
27. ester méthylique de l'acide 3-[1-cyano-2-(4-hydroxy-3,5-diméthyl)-8,9-diméthoxy-5-méthyl-5,6-dihydropyrrolo[2,1-a]isoquinoléin3-yl]-propionique
28. 2-(4-hydroxy-3,5-diméthylphényl)-8,9-diméthoxy-3,5-diméthyl-5,6-dihydropyrrolo[2,1-a]isoquinoléine-1-carbonitrile
30. 7-fluoro-2-(4-hydroxy-3,5-diméthylphényl)-8,9-diméthoxy-3,5-diméthyl-5,6-dihydropyrrolo[2,1-a]isoquinoléine-1-carbonitrile
33. 8,9-diméthoxy-2-(4-méthoxy-3,5-diméthylphényl)-3,5-diméthyl-5,6-dihydropyrrolo[2,1-a]isoquinoléine-1-carbonitrile
37. 8,9-diméthoxy-3,5-diméthyl-2-[1-(toluène-4-sulfonyl)-1H-pyrrol-3-yl]-5,6-dihydropyrrolo[2,1-a]isoquinoléine-1-carbonitrile
38. 8,9-diméthoxy-3,5-diméthyl-2-[1-(toluène-4-sulfonyl)-1H-indol-3-yl]-5,6-dihydropyrrolo[2,1-a]isoquinoléine-1-carbonitrile
39. 2-(1-benzènesulfonyl-1H-indol-3-yl)-8,9-diméthoxy-3,5-diméthyl-5,6-dihydropyrrolo[2,1-a]isoquinoléine-1-carbonitrile
40. 2-(1-méthanesulfonyl-1H-indol-3-yl)-8,9-diméthoxy-3,5-diméthyl-5,6-dihydropyrrolo[2,1-a]isoquinoléine-1-carbonitrile
41. 8,9-diméthoxy-3,5-diméthyl-2-(1-oxypyridin-4-yl)-5,6-dihydropyrrolo[2,1-a]isoquinoléine-1-carbonitrile
42. 7-fluoro-8,9-diméthoxy-3,5-diméthyl-2-[1-(toluène-4-sulfonyl)-1H-indol-3-yl]-5,6-dihydropyrrolo[2,1-a]isoquinoléine-1-carbonitrile
44. 2-(4-hydroxy-3,5-diméthylphényl)-8,9-diméthoxy-5-méthyl-3-morpholin-4-ylméthyl-5,6-dihydropyrrolo[2,1-a]isoquinoléine-1-carbonitrile
45. 8,9-diméthoxy-3,5-diméthyl-2-(2-méthylpyridin-4-yl)-5,6-dihydropyrrolo[2,1-a]isoquinoléine-1-carbonitrile
46. 8,9-diméthoxy-3,5-diméthyl-2-(4-nitrophényl)-5,6-dihydropyrrolo[2,1-a]isoquinoléine-1-carbonitrile
47. acide 4-(1-cyano-8,9-diméthoxy-3,5-diméthyl-5,6-dihydropyrrolo[2,1-a]isoquinoléin-2-yl)-benzoïque
48. 2-(4-aminophényl)-8,9-diméthoxy-3,5-diméthyl-5,6-dihydropyrrolo[2,1-a]isoquinoléine-1-carbonitrile
49. 8,9-diméthoxy-3,5-diméthyl-2-(3-méthylpyridin-4-yl)-5,6-dihydropyrrolo[2,1-a]isoquinoléine-1-carbonitrile
50. acide 4-(1-cyano-8-éthoxy-9-méthoxy-3,5-diméthyl-5,6-dihydropyrrolo[2,1-a]isoquinoléin-2-yl)-benzoïque
51. 2-(4-hydroxy-2-méthylphényl)-8,9-diméthoxy-3,5-diméthyl-5,6-dihydro-pyrrolo[2,1-a]isoquinoléine-1-carbonitrile
52. 4-(1-cyano-8,9-diméthoxy-3,5-diméthyl-5,6-dihydropyrrolo[2,1-a]isoquinoléin-2-yl)-benzamide
53. 8-éthoxy-2-(4-hydroxy-3,5-diméthylphényl)-9-méthoxy-3,5-diméthyl-5,6-dihydropyrrolo[2,1-a]isoquinoléine-1-carbonitrile
54. diméthylamide de l'acide 3-(1-cyano-8,9-diméthoxy-3,5-diméthyl-5,6-dihydropyrrolo[2,1-a]isoquinoléin-2-yl)-indole-1-sulfonique
55. 8,9-diméthoxy-3,5-diméthyl-2-(2-méthyl-1-oxypyridin-4-yl)-5,6-dihydropyrrolo[2,1-a]isoquinoléine-1-carbonitrile
56. 8,9-diméthoxy-3,5-diméthyl-2-[1-(morpholine-4-sulfonyl)-1H-indol-3-yl]-5,6-dihydropyrrolo[2,1-a]isoquinoléine-1-carbonitrile
57. 8,9-diméthoxy-3,5-diméthyl-2-[4-(2H-tétrazol-5-yl)-phényl]-5,6-dihydropyrrolo[2,1-a]isoquinoléine-1-carbonitrile
58. [4-(1-cyano-8,9-diméthoxy-3,5-diméthyl-5,6-dihydropyrrolo[2,1-a]isoquinoléin-2-yl)-phényl]-amide de l'acide morpholine-4-sulfonique
59. N-[4-(1-cyano-8,9-diméthoxy-3,5-diméthyl-5,6-dihydropyrrolo[2,1-a]isoquinoléin-2-yl)-phényl]-méthanesulfonamide
60. ester éthylique de l'acide 5-éthyl-2-(2-fluoro-3,4-diméthoxyphényl)-8,9-diméthoxy-3-méthyl-5,6-dihydropyrrolo[2,1-a]isoquinoléine-1-carboxylique
61. ester éthylique de l'acide 7-chloro-8,9-diméthoxy-3,5-diméthyl-2-pyridin-4-yl-5,6-dihydropyrrolo[2,1-a]isoquinoléine-1-carboxylique
62. ester éthylique de l'acide 7-chloro-2-(4-hydroxy-3,5-diméthylphényl)-8,9-diméthoxy-3,5-diméthyl-5,6-dihydropyrrolo[2,1-a]isoquinoléine-1-carboxylique
63. ester éthylique de l'acide 7,8,9-triméthoxy-3,5-diméthyl-2-pyridin-4-yl-5,6-dihydropyrrolo[2,1-a]isoquinoléine-1-carboxylique
65. ester méthylique de l'acide 2-(4-hydroxy-3,5-diméthylphényl)-8,9-diméthoxy-3,5-diméthyl-5,6-dihydropyrrolo[2,1-a]isoquinoléine-1-carboxylique
66. ester méthylique de l'acide 8,9-diméthoxy-3,5-diméthyl-2-[1-(toluène-4-sulfonyl)-1H-indol-3-yl]-5,6-dihydropyrrolo[2,1-a]isoquinoléine-1-carboxylique
67. ester éthylique de l'acide 5-cyano-2-(4-hydroxy-3,5-diméthylphényl)-8,9-diméthoxy-3-méthyl-5,6-dihydropyrrolo[2,1-a]isoquinoléine-1-carboxylique
ou un sel, stéréo-isomère, hydrate ou hydrate d'un sel de ceux-ci.

15. Composé selon l'une quelconque des revendications 1 à 14 destiné à une utilisation en thérapie.

16. Utilisation d'un composé selon l'une quelconque des revendications 1 à 14 dans la production de compositions pharmaceutiques pour le traitement de troubles neurologiques et/ou psychiatriques.

17. Composition pharmaceutique comprenant comme ingrédient actif une quantité efficace d'au moins un des composés selon la revendication 1 ensemble avec des auxiliaires et/ou des excipients pharmaceutiques appropriés.
